# EUROPÄISCHE PATENTSCHRIFT

(11) **EP 3 227 302 B1**
(45) Veröffentlichungstag und Bekanntmachung des Hinweises auf die Patenterteilung: **11.07.2018**
(21) Anmeldenummer: 15801469.6
(22) Anmeldetag: 30.11.2015
(51) Int. Cl.: C07D 513/04, A01N 43/90

(54) **BICYCLISCHE VERBINDUNGEN ALS SCHÄDLINGSBEKÄMPFUNGSMITTEL**
BICYCLIC COMPOUNDS AS PEST CONTROLLERS
COMPOSÉS BICYCLIQUES COMME PESTICIDES

(30) Priorität: 02.12.2014 EP 14195946
(43) Veröffentlichungstag der Anmeldung: 11.10.2017
(73) Patentinhaber: Bayer CropScience AG, 40789 Monheim am Rhein (DE)
(72) Erfinder: CEREZO-GALVEZ, Silvia, 40764 Langenfeld (DE); ARLT, Alexander, 50859 Köln (DE); BRETSCHNEIDER, Thomas, 53797 Lohmar (DE); FISCHER, Reiner, 40789 Monheim am Rhein (DE); FÜSSLEIN, Martin, 40225 Düsseldorf (DE); JESCHKE, Peter, 51467 Bergisch Gladbach (DE); VOERSTE, Arnd, 50674 Köln (DE); ILG, Kerstin, 50670 Köln (DE); MALSAM, Olga, 51503 Rösrath (DE); LÖSEL, Peter, 51371 Leverkusen (DE)
(74) Vertreter: BIP Patents
(86) Internationale Anmeldenummer: PCT/EP2015/078062
(87) Internationale Veröffentlichungsnummer: WO 2016/087373

(56) Entgegenhaltungen:
- WO-A1-2014/104407
- WO-A1-2014/125651
- WO-A1-2015/038503
- WO-A2-2012/000896
- DATABASE REGISTRY [Online] CHEMICAL ABSTRACTS SERVICE, COLUMBUS, OHIO, US; 24. November 2013 (2013-11-24), XP002737643, Database accession no. 1479655-59-8 (RN)

## Beschreibung

Die vorliegende Anmeldung betrifft neue bicyclische Verbindungen, Mittel enthaltend diese Verbindungen, ihre Verwendung zur Bekämpfung von tierischen Schädlingen sowie Verfahren und Zwischenprodukte zu Ihrer Herstellung.

Kürzlich sind bicyclische Verbindungen bekannt geworden, die insektizide Eigenschaften besitzen (WO 2015/038503 A1 und WO 2014/125651 A1). In WO 2012/102387 A1 sind heterocyclische Verbindungen beschrieben, die insbesondere als Insektizide und Akarizide verwendet werden können.

In Synthesis 2003, (13), 2033-2040 wird über einen neuen Zugang zu Oxazolopyridinen über Hydroxyamidin-Derivate berichtet.

In US 4,038,396 und DE 2 330 109 A1 sind Synthese und Anwendung von Oxazolopyridinen und Thiazolopyridinen als antiinflammatorische, analgetische und antipyretische Substanzen beschrieben.

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch Verbindungen der Formel (I) 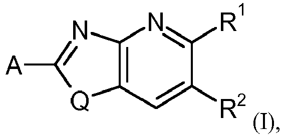 in welcher
- A: für den Rest (A-a) 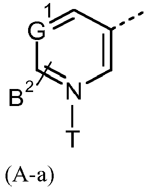 steht, worin die gestrichelte Linie die Bindung zum Kohlenstoffatom des Bicyclus der Formel (I) bedeutet,
G¹ für C-B¹ steht,
B¹ für Wasserstoff oder Fluor steht,
B² für Wasserstoff steht,
T für ein Elektronenpaar oder für Sauerstoff steht,
Q für Schwefel steht,
R¹ für Wasserstoff steht,
R² a) für einen der folgenden B-Reste 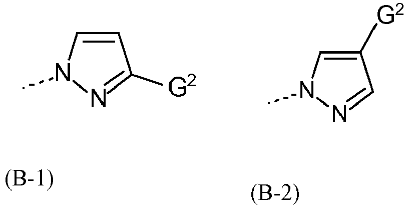 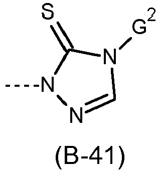 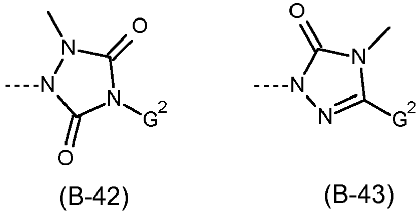 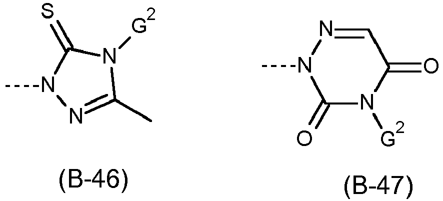 steht, worin die gestrichelte Linie die Bindung zum Kohlenstoffatom des Bicyclus der Formel (I) bedeutet, oder
- R²: c) für einen Rest der Formel 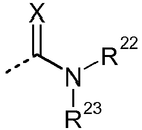 steht, worin X für Sauerstoff oder Schwefel steht und die gestrichelte Linie die Bindung zum Kohlenstoffatom des Bicyclus der Formel (I) bedeutet, oder
R² f) für einen Rest der Formel 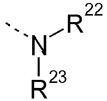 steht, worin die gestrichelte Linie die Bindung zum Kohlenstoffatom des Bicyclus der Formel (I) bedeutet, worin
G² für einen Rest aus der Reihe Wasserstoff, C₁-C₄-Alkyl, Halogen-C₁-C₄-alkyl C₁-C₄-Alkoxy, Halogen-C₁-C₁-alkoxy, C₁-C₄-Alkylthio und C₁-C₄-Halogenalkylthio steht, und, im Fall, dass R² für c) steht
R²² für den D-Rest 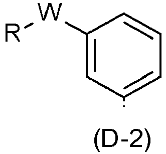 steht, worin
- R: für gegebenfalls einfach, zweifach, dreifach, vierfach oder fünffach durch Fluor, Chlor substituiertes C₁-C₄-Alkyl steht,
- W: für einen Rest aus der Reihe S, SO und SO₂ steht, und, im Fall, dass R² für f) steht,
- R²²: für gegebenenfalls durch Fluor, Chlor oder Brom substituiertes Phenyl steht und
- R²³: für Wasserstoff oder C₁-C₆-Alkyl steht.

In den Definitionen, steht Halogen für Fluor, Chlor, Brom und Iod, bevorzugt wiederum für Fluor, Chlor und Brom.

Durch Halogen substituierte Reste, z.B. Halogenalkyl (= Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein. Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Wenn T im Rest A der Formel (A-a) 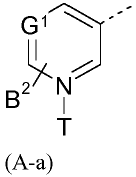 für ein Elektronenpaar steht, liegt der Rest als Pyridinderivat der Formel 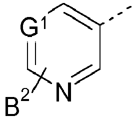 vor.

Wenn T im Rest A der Formel (A-a) 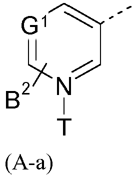 für Sauerstoff steht, liegt der Rest als Pyridin-*N*-Oxid-derivat der Formel 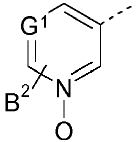 vor. Auf die Darstellung der Formalladungen (+ am Stickstoff und - am Sauerstoff) wurde hier verzichtet.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Eine bevorzugte Ausführungsform der Erfindung betrifft Verbindungen der Formel (I), in welchen A für Pyridin-3-yl steht.

Eine weitere bevorzugte Ausführungsform der Erfindung betrifft Verbindungen der Formel (I), in welchen A für 5-Fluor-pyridin-3-yl steht.

Eine weitere bevorzugte Ausführungsform der Erfindung betrifft Verbindungen der Formel (I), in welchen R² die unter a) aufgeführten Bedeutungen hat.

Eine weitere bevorzugte Ausführungsform der Erfindung betrifft Verbindungen der Formel (I), in welchen R² die unter c) aufgeführten Bedeutungen hat.

Eine weitere bevorzugte Ausführungsform der Erfindung betrifft Verbindungen der Formel (I), in welchen R² die unter f) aufgeführten Bedeutungen hat.

Die oben aufgeführten allgemeinen oder in Vorzugsbereichen aufgeführten Restedefinitionen bzw. Erläuterungen gelten für die Endprodukte (auch für die Verbindungen der später aufgeführten Formeln (I-A) bis (I-P) und für die Ausgangsprodukte und Zwischenprodukte entsprechend. Diese Restedefinitionen können untereinander, also auch zwischen den jeweiligen Vorzugsbereichen, beliebig kombiniert werden.

In einer bevorzugten Ausführungsform betrifft die Erfindung Verbindungen der Formel (I-A)

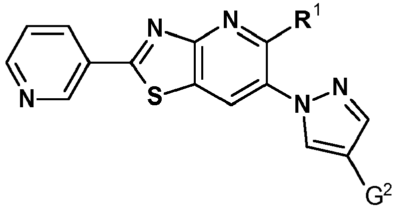

In einer weiteren bevorzugten Ausführungsform betrifft die Erfindung Verbindungen der Formel (I-E)

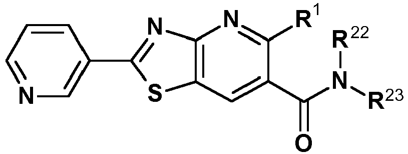

In einer weiteren bevorzugten Ausführungsform betrifft die Erfindung Verbindungen der Formel (I-H)

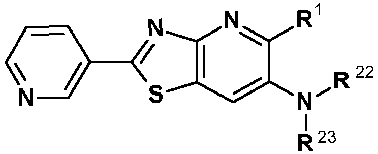

In einer weiteren bevorzugten Ausführungsform betrifft die Erfindung Verbindungen der Formel (I-I)

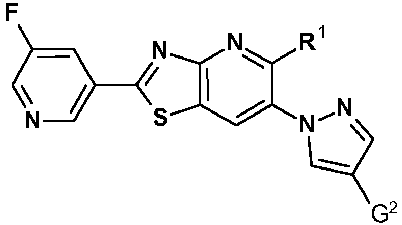

In einer weiteren bevorzugten Ausführungsform betrifft die Erfindung Verbindungen der Formel (I-M)

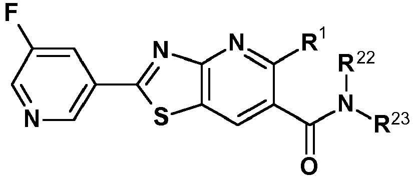

In einer weiteren bevorzugten Ausführungsform betrifft die Erfindung Verbindungen der Formel (I-P)

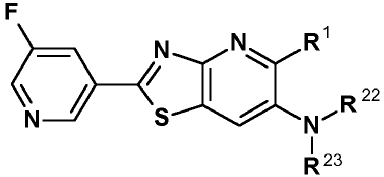

In den Formeln (I-A) bis (I-P) haben die Variablen die weiter oben genannten Bedeutungen.

Die Verbindungen der Formel (I) und deren Säureadditionssalze und Metallsalzkomplexe besitzen gute Wirksamkeit, insbesondere zur Bekämpfung von tierischen Schädlingen, zu denen Arthropoden und insbesondere Insekten zählen.

Als geeignete Salze der Verbindungen der allgemeinen Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

Die Verbindungen der Formel (I) können gegebenenfalls auch in Abhängigkeit von der Art der Substituenten als Stereoisomere, d.h. als geometrische und/oder als optische Isomere oder Isomerengemische vorliegen in unterschiedlichen Zusammensetzungen vorliegen. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Die Erfindung betrifft daher sowohl die reinen Enantiomeren und Diastereomeren, als auch deren Gemische zur Bekämpfung von tierischen Schädlingen, zu denen Arthropoden und insbesondere Insekten zählen.

Vorzugsweise werden jedoch die optisch aktiven, stereoisomeren Formen der Verbindungen der Formel (I) und deren Salze erfindungsgemäß verwendet.

Im Folgenden werden die Begriffe "Synthesebeispiel" und "Anwendungsbeispiel" gleichbedeutend verwendet, sofern nichts anderes angegeben ist.

Weiter wurde gefunden, dass sich die Verbindungen der Formel (I) und auch diejenigen in der Tabelle 1 aufgeführten Verbindungen, die nicht unter die Formel (I) fallen, nach den im Folgenden beschriebenen Verfahren herstellen lassen.

**Methode** A: Verbindungen der Formel (I), in denen der Heterocyclus A für gegebenenfalls mit einem Rest B² substituiertes Pyrimidin-5-yl (A-a; G¹ = N), Pyridin-3-yl (A-a; G¹ = C-B¹), Pyrazin-2-yl (A-b), Pyridazin-3-yl (A-c), Thiazol-5-yl (A-d), Isothiazol-4-yl (A-e) und Pyrazol-4-yl (A-f) steht, können beispielsweise gemäß Methode A (vgl. Reaktionsschema I) in drei Schritten hergestellt werden.

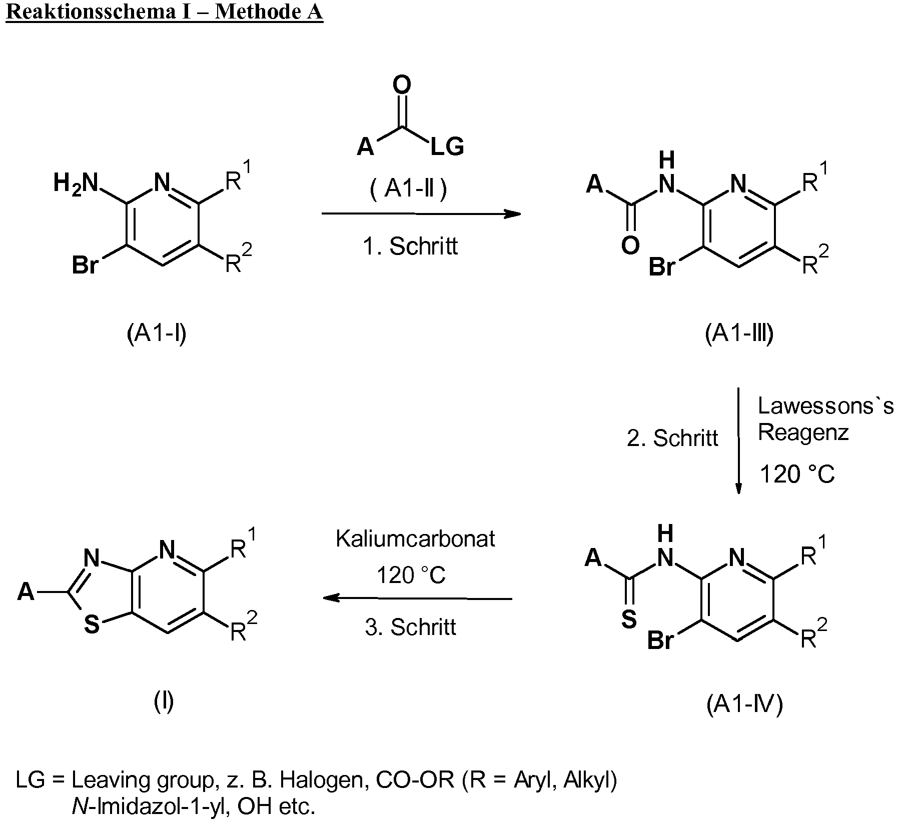

Im Reaktionsschema I haben A, R¹ und R², sofern nichts anderes angegeben ist, die oben genannten Bedeutungen.

Beispielsweise können die substituierten Aniline der Formel (A1-I) mit den entsprechenden aktivierten Carbonsäuren (z. B. als Carbonsäurechlorid oder dessen Hydrochlorid) der Formel (A1-II) in Gegenwart von basischen Reaktionshilfsmitteln in einem ersten Reaktionsschritt zu Verbindungen der Formel (A1-III) umgesetzt werden. Diese werden dann durch entsprechende Schwefeldonatoren wie z. B. Lawesson's Reagenz zu Verbindungen der Formel (A1-IV) thioniert. In einem dritten Reaktionsschritt können diese dann in Gegenwart einer geeigneten Base, beispielsweise Kaliumcarbonat, unter Bildung der Verbindungen (I) cyclisiert werden.

**Methode** A - **Schritt 1:** Die Verbindungen der Formel (A1-I) sind teilweise bekannt und kommerziell erhältlich bzw. können nach im Prinzip bekannten Herstellungsverfahren (z. B. für R¹ = H, R² = Br; 3,5-Dibrompyridin-2-amin (Tetrahedron Letters (2014), 55(36), 5058-5061) erhalten werden.

Die Verbindungen der Formel (A1-II) sind teilweise bekannt und kommerziell erhältlich bzw. können nach im Prinzip bekannten Herstellungsverfahren (z. B. für A = Pyridin-3-yl, LG = Cl; Nicotinsäurechlorid (Journal of the American Chemical Society (1953), 75, 4364) oder für A = 5-Fluor-pyridin-3-yl, LG = Cl; 5-Fluornicotinoylchlorid (US 2,516,830) erhalten werden.

Für den Amidierungsschritt sind zahlreiche Reaktionsbedingungen beschrieben worden, z.B. G. Benz in Comprehensive Organic Synthesis, 1st Ed., Pergamon Press, Oxford, 1991, Vol. 6, S. 381-417; P.D. Bailey et al. in Comprehensive Organic Functional Group Transformation, 1st Ed., Elsevier Science Ltd., Oxford, 1995, Vol. 5, S. 257-308 und R.C. Larock in Comprehensive Organic Transformations, 2nd Ed., Wiley-VCH, New York, Weinheim, 1999, S. 1929-1994. Einige dieser Reaktionen verlaufen über intermediäre Carbonsäurechloride, die isoliert oder in-situ aus A1-II (LG = OH) erzeugt eingesetzt werden können.

Die Amidierungsreaktionen erfolgen gegebenenfalls in Gegenwart eines Kondensationsmittels, gegebenenfalls in Gegenwart eines Säureakzeptors und gegebenenfalls in Gegenwart eines Lösungsmittels.

Als Kondensationsmittel kommen alle üblicherweise für derartige Amidierungsreaktionen verwendbaren Kondensationsmittel infrage. Beispielhaft genannt seien Säurehalogenidbildner wie Phosgen, Phosphortrichlorid, Oxalylchlorid oder Thionylchlorid; Carbodiimide, wie *N,N'-*Dicyclohexylcarbodiimid (DCC) und 1-(3-Dimethylaminopropyl)-3-ethyl-carbodiimid (EDCI), oder andere übliche Kondensationsmittel, wie Phosphorpentoxid, Polyphosphorsäure, *N,N'-*Carbonyldiimidazol, 2-Chlorpyridin 1-Methoiodid (Mukaiyamas Reagenz), 2-Ethoxy-N-ethoxycarbonyl-1,2-dihydrochinolin (EEDQ), Triphenylphosphin/Tetrachlorkohlenstoff, Bromtripyrrolidino-phosphonium-hexafluorphosphat (BROP), O-(*1H*-Benzotriazol-1-yloxy)tris(dimethyl-amino)phosphonium-hexafluorphosphat (BOP), *N*,*N*,*N*',*N*'-Bis(tetramethylen)cloruronium-tetrafluorborat, O-(*1H*-Benzotriazol-1-yl)-*N,N,N'*,*N*'-tetramethyluronium-hexafluorphosphat (HBTU), O-(*1H*-Benzotriazol-1-yl)-*N,N,N',N'*-bis(tetramethylen)uronium-hexafluorphosphat, O-(*1H-*Benzotriazol-1-yl)-*N,N,N',N'*-tetramethyluronium-tetrafluorborat (TBTU), O-(*1H*-Benzotriazol-1-yl)-*N,N,N',N'*-bis(tetramethylen)uronium-tetrafluorborat, O-(7-Azabenzotriazol-1-yl)-*N,N,N',N'-*tetramethyluronium-hexafluorphosphat (HATU), 1-Hydroxybenzotriazol (HOBt) und 4-(4,6-Dimethoxy-1,3,5-triazin-2-yl)-4-methylmorpholiniumsalz (DMT.MM), meistens als Chlorid verfügbar. Diese Reagenzien können separat oder gegebenenfalls in Kombination eingesetzt werden.

Als Säureakzeptor kommen alle üblichen anorganischen oder organischen Basen infrage, beispielsweise Triethylamin, Diisopropylethylamin, *N*-Methylmorpholin oder *N,N*-Dimethylaminopyridin. Das erfindungsgemäße Verfahren A wird gegebenenfalls in Gegenwart eines geeigneten Reaktionshilfstoffes wie beispielsweise *N,N*-Dimethylformamid oder *N*,*N-*Dimethylaminopyridin durchgeführt.

Als Lösungs- oder Verdünnungsmittel kommen alle inerten organischen Lösungsmittel in Betracht, beispielsweise aliphatische oder aromatische Kohlenwasserstoffe (wie Petrolether, Toluol), halogenierte Kohlenwasserstoffe (wie Chlortoluol, Dichlormethan, Chloroform, 1,2-Dichlorethan), Ether (wie Diethylether, Dioxan, Tetrahydrofuran, 1,2-Dimethoxyethan), Ester (wie Essigsäureethylester- oder - methylester), Nitrokohlenwasserstoffe (wie Nitromethan, Nitroethan, Nitrobenzol), Nitrile (wie Acetonitril, Benzonitril), Amide (wie *N,N*-Dimethylformamid, *N,N*-Dimethylacetamide, *N-*Methylformanilid, *N*-Methylpyrrolidon, Hexamethylphosphorsäuretriamid) sowie Dimethylsulfoxid oder Wasser oder Gemische der genannten Lösungsmittel.

Zur Synthese der Verbindungen mit der Formel (A1-III) können aber auch gemischte Anhydride (LG = COOR) verwendet werden (vgl. G. W. Anderson et al. J. Am. Chem. Soc. 1967, 89, 5012-5017). Bei diesem Verfahren, das über Verbindungen der Formel (A1-II, LG = CO-OR, R = Alkyl, Aryl) führt, können Chlorameisensäureester zum Einsatz kommen, z.B. Chlorameisensäureisobutylester (LG = COOR mit R = iso-Butyl) und Chlorameisensäure-isopropylester (LG = COOR mit R = iso-Propyl). Ebenfalls können dafür Diethylacetylchlorid, Trimethylacetylchlorid und ähnliche Verbindungenverwendet werden.

**Methode A** - **Schritt 2:** Die Amidfunktion der Carbonsäureamide des Typs (A1-III) kann durch geeignete Schwefelungsreagenzien, z. B. Lawesson's Reagenz oder Phosphor(V)-sulfid, unter Erhitzung in einem geeigneten Lösungsmittel, z. B. Toluol oder Anisol, in eine Thioamidfunktion überführt werden, wodurch Verbindungen des Typs (A1-IV) entstehen (vgl. z. B. WO 2013/33901 für 5-Brom-N-(2,6-difluorphenyl)pyridin-3-carbothioamid). Bei diesem Reaktionstyp kann bereits teilweise eine Cyclisierung zu Verbindungen der Formel (I) erfolgen.

**Methode A** - **Schritt 3**: Schließlich lassen sich die Verbindungen der Formel (I) nach literaturbekannten Methoden (vgl. z. B. WO 2013/33901 für 2-(5-Brompyridin-3-yl)-4-fluor-1,3-benzothiazol) durch Erhitzen in geeignetem Lösungsmittel, z. B. Toluol oder DMF, in Anwesenheit einer Base wie z. B. Natriumhydrid oder Kaliumcarbonat, in die bicyclischen Thiazole des Typs (I) überführen.

Wird bei dem erfindungsgemäßen Verfahren zur Herstellung der Verbindungen der Formel (I) als Verbindung der Formel (A1-I) 3,5-Dibrompyridin-2-amin (R¹ = H, R² = Br) und als Verbindung der Formel (A1-II) 3-(Chlorcarbonyl)pyridiniumchlorid (A = 3-Pyridin-3-yl) eingesetzt, so entsteht zunächst das N-(3,5-Dibrompyridin-2-yl)nicotinamid (A = 3-Pyridin-3-yl, R¹ = H, R² = Br). Nachfolgende Thionierung und Cyclisierung führt dann zum 6-Brom-2-(pyridin-3-yl)[1,3]thiazolo[4,5-b]pyridin (I-a-1)), A = Pyridin-3-yl, R¹ = H, R² = Br) [vgl. Synthesebeispiel I-a-1].

Verbindungen der Formel (I), in denen R² für Halogen, beispielsweise Brom oder Iod, steht, können gemäß Reaktionsschema I aus halogenierten 2-Brom-anilinderivaten (A1-I) erhalten werden. Hieraus können nach literaturbekannten Methoden (B bis F) weitere Verbindungen der Formel (I) generiert werden. Beispielhaft sind die Methoden B bis F und die daraus resultierenden Produkttypen in Reaktionsschema II dargestellt.

**Methode B/F:** Gemäß WO 2010/71819 bzw. Bioorganic and Medicinal Chemistry Letters (2009), 19(21), 6176 - 6180 lassen sich bromierte Thiazolopyridine mit gegebenenfalls in-situ erzeugten, substituierten Arylboronsäuren bzw. Arylboronsäurepinakolestern in Anwesenheit von geeigneten Kupplungskatalysatoren, wie z. B. Tetrakis(triphenylphosphin)-palladium(0) oder Bis-triphenylphosphin-palladium(II)chlorid in Anwesenheit einer Base, wie z. B. Natriumcarbonat, in einem inerten, organischen Lösungs- bzw. Verdünnungsmittel, z. B. Toluol in Kombination mit Ethanol, arylieren (Methode B), wodurch kohlenstoffverknüpfte Verbindungen des Typs (I-b) [vgl. auch Synthesebeispiel (19)] erhalten werden können. Auch gegebenenfalls substituierte heteroaromatische Boronsäuren bzw. deren Pinakolester können analog mit bromierten bicyclischen heteroaromatischen Systemen nach Methode B umgesetzt werden (vgl. z. B. EP 1 214 319 B1 für 3-[6-(3-Furyl)-3H-imidazo[4,5-b]pyridin-3-yl]phenol).

Alternativ können die Verbindungen der Formel (I-a) zunächst mittels literaturbekannter Methoden in Verbindungen des Typs (I-h) überführt werden, die dann anschließend mit halogen-aktivierten, gegebenenfalls weiter substituierten Heterocyclen gemäß Reaktionsschema II (Methode F) [vgl. T. Ishiyama et al., J. Org. Chem., 1995, 60, 7508 - 7510; WO 2010/151601] in einem inerten, organischen Lösungs- bzw. Verdünnungsmittel zu kohlenstoffverknüpften Verbindungen des Typs (I-i) weiterreagieren. 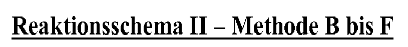 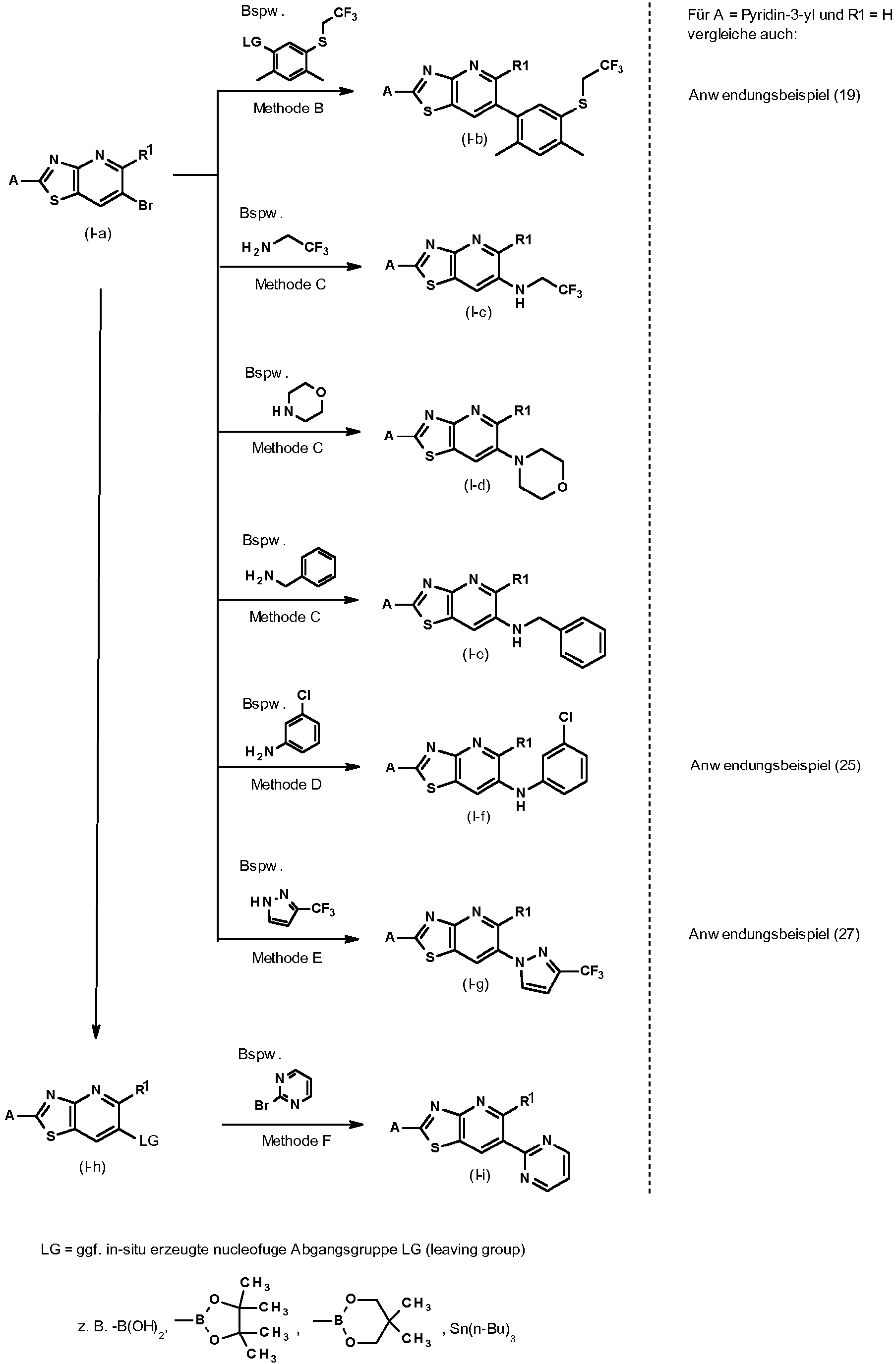 Als Kupplungskatalysatoren kommen Palladium-Katalysatoren wie [1,1'-Bis (diphenylphosphino)ferrocen]dichlorpalladium(II)] oder Tetrakis(triphenylphosphin) Palladium(0) in Betracht.

Als geeignete basische Reaktionshilfsmittel zur Durchführung der Verfahren gemäß Reaktionsschema II finden bevorzugt Carbonate des Natriums oder Kaliums Verwendung.

Als Lösungsmittel werden bevorzugt Nitrile wie Acetonitril, Benzonitril, insbesondere Acetonitril, oder Ether wie Diethylether, Dioxan, Tetrahydrofuran, 1,2-Dimethoxyethan, insbesondere 1,2-Dimethoxyethan in Kombination mit Wasser eingesetzt.

**Methode C:** Bevorzugt mittels Katalyse durch geeignete Kupplungskatalysatoren, wie z. B. Tris-(dibenzylidenaceton)-dipalladium(O) unter Verwendung geeigneter Liganden, z. B. 2,2'-Bis-(diphenylphosphino)-1,1'-binaphthyl (BINAP), und einer Base wie beispielsweise Natrium-tert-butanolat lassen sich bromierte heteroaromatische Bicyclen in einem inerten organischen Lösungs- bzw. Verdünnungsmittel auch mit gegebenenfalls substituierten aliphatischen primären bzw. sekundären Aminen oder Arylmethyl- bzw Hetarylmethylaminen umsetzen (Beispieltyp (I-e)) [vgl. z. B. WO 2013/123215 A2 zur Synthese von 3-(4-Ethoxybenzyl)-N-(4-ethylbenzyl)-3H-imidazo[4,5-b]pyridin-6-amin].

Als Lösungs- oder Verdünnungsmittel kommen alle inerten organischen Lösungsmittel in Betracht, beispielsweise aliphatische oder aromatische Kohlenwasserstoffe. Bevorzugt werden aromatische Kohlenwasserstoffe wie beispielsweise Toluol eingesetzt.

**Methode D:** Mit gegebenenfalls substituierten Anilinen reagieren bromierte heteroaromatische Bicyclen in einem inerten organischen Lösungs- bzw. Verdünnungsmittel zu den entsprechenden Arylamino-Verbindungen (vgl. Beispieltyp (I-f) und Anwendungsbeispiel (25)), bevorzugt unter Katalyse durch geeignete Kupplungskatalysatoren wie z. B. Tris-(dibenzylidenaceton)-dipalladium(0), in Anwesenheit eines geeigneten Liganden, wie z. B. Xantphos, und einer Base, z. B. Cäsiumcarbonat (vgl. z. B. EP 2 341 052 zur Herstellung von 7-Anilino-N-phenyl-4H-pyrido[3,2-b][1,4]oxazin-4-carboxamid). Auch ggf. substituierte Amino-heteroaromaten lassen sich in einem inerten, organischen Lösungs- bzw. Verdünnungsmittel auf analoge Weise umsetzen, z. B. unter Katalyse durch geeignete Kupplungskatalysatoren wie Tris-(dibenzylidenaceton)-dipalladium(0), mit geeigneten Liganden, z. B. 2,2'-Bis-(diphenylphosphino)-1,1'- binaphthyl (BINAP) und einer Base, bspw. Natrium-tert-butanolat.

Als Lösungs- oder Verdünnungsmittel kommen alle inerten organischen Lösungsmittel in Betracht, beispielsweise aliphatische oder aromatische Kohlenwasserstoffe. Bevorzugt werden aromatische Kohlenwasserstoffe (beispielsweise Toluol) eingesetzt.

**Methode E:** Heterocyclische Amine wie beispielsweise Imidazole, Pyrazole oder Triazole, ggf. substituiert, lassen sich in Anlehnung an die im Reaktionsschema II gezeigte Methode E [vgl. US 2012/122843 zur Herstellung von 6-Amino-3-(4-methyl-1H-imidazol-1-yl)pyridin-2-carbonitril] bevorzugt in Gegenwart geeigneter Katalysatoren wie Kupfer(I)iodid, in Anwesenheit basischer Liganden, z. B. L-Prolin oder *trans*-N,N'-Dimethylcyclohexan-1,2-diamin, und einer Base wie Cäsiumcarbonat oder Kaliumcarbonat, in einem inerten organischen Lösungs- bzw. Verdünnungsmittel in bromierte bicyclische Systeme einführen (vgl. Beispieltyp (I-g) und Anwendungsbeispiel (27)).

Als Lösungs- oder Verdünnungsmittel kommen alle inerten organischen Lösungsmittel in Betracht, beispielsweise aliphatische oder aromatische Kohlenwasserstoffe. Bevorzugt werden aromatische Kohlenwasserstoffe wie beispielsweise Toluol eingesetzt, aber auch polarere Lösungsmittel wie DMF. Verbindungen der Formel (I), in denen R² für Halogen, beispielsweise Brom oder Iod steht, können gemäß Reaktionsschema I aus halogenierten 2-Brom-anilinderivaten erhalten werden. Hieraus können nach literaturbekannten Methoden (G) weitere Verbindungen der Formel (I) generiert werden, wie in Reaktionsschema III dargestellt.

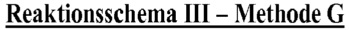

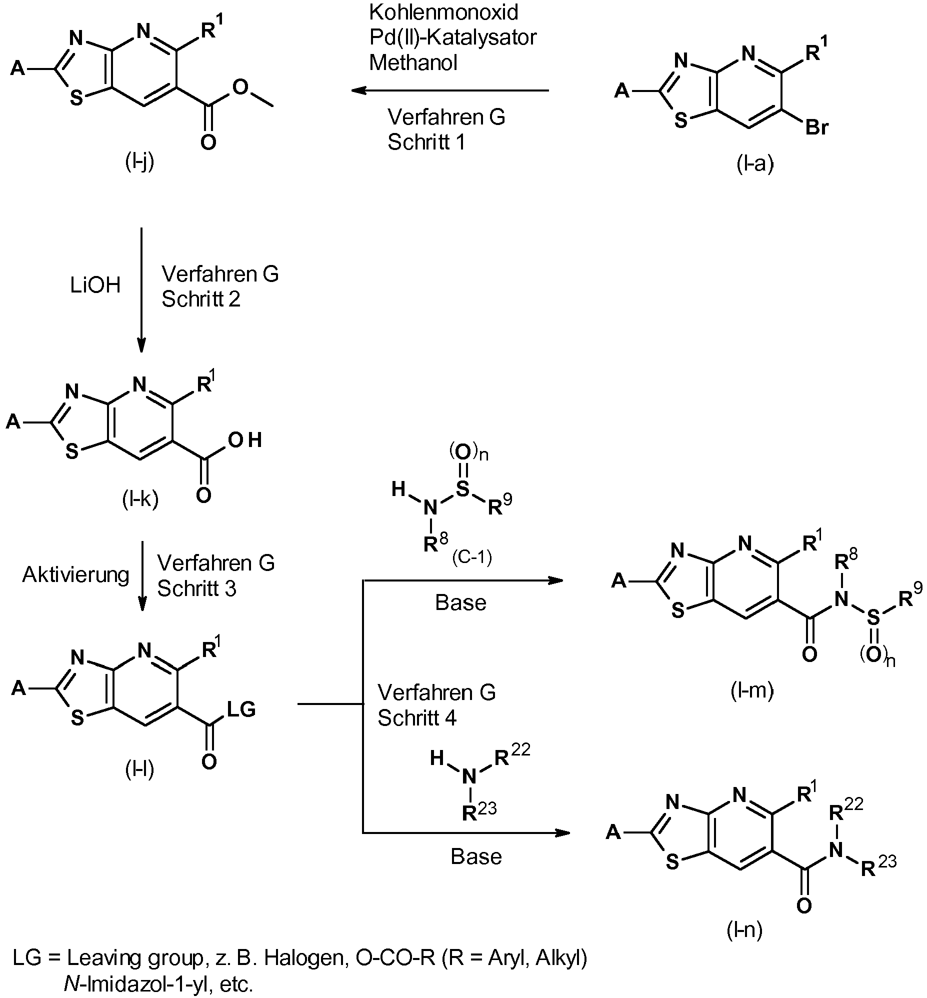

**Methode G** - **Schritt 1:** Verbindungen der Formel (I-a), in denen R² für Halogen, beispielsweise Brom oder Iod, steht, können nach literaturbekannten Methoden mit Kohlenmonoxid und einem Alkohol, z. B. Methanol, unter Katalyse durch geeignete Metallverbindungen wie z. B. (1,1'-Bis(diphenylphosphino)ferrocen)-palladium(II)dichlorid, und in Anwesenheit von Basen wie Triethylamin, in geeigneten Lösungsmitteln wie z. B. dem verwendeten Alkohol selbst, THF und/oder DMF, zu den entsprechenden Carbonsäureestern (I-j) umgesetzt werden [vgl. z. B. WO 2009/152072 für Methyl-2-{[(tert-butoxycarbonyl)amino]methyl}-3H-imidazo[4,5-b]pyridin-6-carboxylat].

**Methode G** - **Schritt 2**: Ester des Typs (I-j) können nach literaturbekannten Methoden mittels geeigneten Basen, wie z. B. wässriger Lithiumhydroxid- oder Natriumhydroxid-Lösung, in geeigneten Lösungs- bzw. Verdünnungsmitteln, wie z. B. Dioxan oder THF, in die Verbindungen (I-k) mit freier Säurefunktion überführt werden.

**Methode G** - **Schritt 3 und 4:** Verbindungen der Formel (I), in denen R² für einen Rest aus der Reihe (C-1) bis (C-9) (Typ I-m) oder eine Gruppe der Formel C(X)-NR²²R²³ (Typ I-n) steht, können beispielsweise aus Verbindungen der Formel (I), in denen R² für eine Carboxylgruppe steht (Typ I-k), nach geeigneter Aktivierung (d.h. LG steht für eine gegebenenfalls in-situ erzeugte nucleofuge Abgangsgruppe "Leaving Group") mittels allgemein bekannter Methoden hergestellt werden [vgl. Methode A, Schritt 1].

Die nachfolgenden Reaktionen der aktivierten Verbindungen der Formel (I-l) mit den jeweiligen Aminkomponenten nach Reaktionsschema III wird gegebenenfalls in Gegenwart eines geeigneten Reaktionshilfstoffes und in Gegenwart eines geeigneten Lösungs- oder Verdünnungsmittels durchgeführt.

Verbindungen der Formel (I), in denen R² für -NR²³-C(X)-R²² steht, können beispielsweise aus Verbindungen der Formel (I), in denen R² für -NHR²³ steht, mittels N-Acylierungsreaktion unter Verwendung von aktivierten Verbindungen der Formel LG-C(X)-R²², worin LG für eine gegebenenfalls in-situ erzeugte nucleofuge Abgangsgruppe steht, erhalten werden.

Diese Verbindungen der Formel (I), in denen R² für eine Gruppe der Formel -NHR²³ steht, lassen sich aus Verbindungen der Formel (I), in denen R² für eine Carboxylgruppe steht, gemäß Reaktionsschema IV nach bekannten Methoden darstellen.

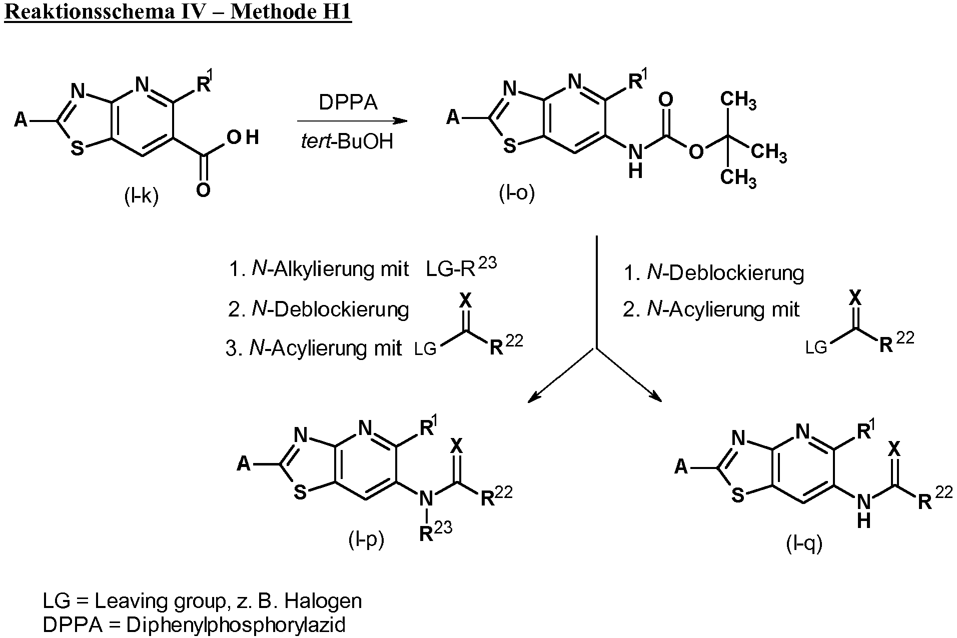

**Methode H1:** Beispielsweise können Verbindungen der Formel (I-o) mittels Curtius-Abbau, wie beispielsweise in Houben-Weyl, Methoden der Organischen Chemie, Band XI/1 (Georg Thieme Verlag Stuttgart), S. 865 beschrieben, erhalten werden.

Hierbei können die Verbindungen der Formel (I-k) beispielsweise mit Diphenylphosphorylazid (DPPA) in Gegenwart von tert-Butanol direkt zu Verbindungen der Formel (I-o) reagieren.

Aus den Verbindungen der Formel (I-o) lassen sich die Verbindungen der Formel (I-p) durch N-Alkylierung in einem ersten Reaktionsschritt, *N*-Deblockierung (d.h. Abspaltung der Boc-Gruppe) in einem zweiten Reaktionsschritt und nachfolgende *N*-Acylierung in einem dritten Reaktionsschritt erhalten.

Die Verbindungen der Formel (I-q) können mittels *N*-Deblockierung (d.h. Abspaltung der Boc-Gruppe) in einem ersten Reaktionsschritt und nachfolgender *N*-Acylierung in einem zweiten Reaktionsschritt hergestellt werden.

Im Allgemeinen können für die Entfernung der Schutzgruppe saure oder basische Reaktionshilfsmittel nach literaturbekannter Verfahrensweise verwendet werden. Bei Verwendung von Schutzgruppen des Carbamat-Typs werden bevorzugt saure Reaktionshilfsmittel verwendet. Bei Verwendung der *tert-*Butylcarbamat-Schutzgruppe (Boc-Gruppe) werden beispielsweise Mischungen von Mineralsäuren wie Salzsäure, Bromwasserstoffsäure, Salpetersäure, Schwefelsäure, Phosphorsäure oder von organischen Säuren wie Benzoesäure, Ameisensäure, Essigsäure, Trifluoressigsäure, Methansulfonsäure, Benzolsulfonsäure oder Toluolsulfonsäure in einem geeigneten Verdünnungsmittel wie Wasser und/oder einem organischen Lösungsmittel wie Tetrahydrofuran, Dioxan, Dichlormethan, Chloroform, Essigester, Ethanol oder Methanol verwendet. Bevorzugt sind Mischungen von Salzsäure oder Essigsäure mit Wasser und/oder einem organischen Lösungsmittel wie Essigester.

Alternativ zu Methode H1 können die Verbindungen der Typen I-p bzw. I-q auch aus nach Methode A synthetisierten Verbindungen durch die im Folgenden beschriebene Methode H2 hergestellt werden:

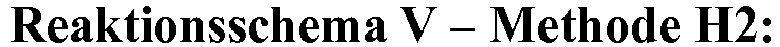

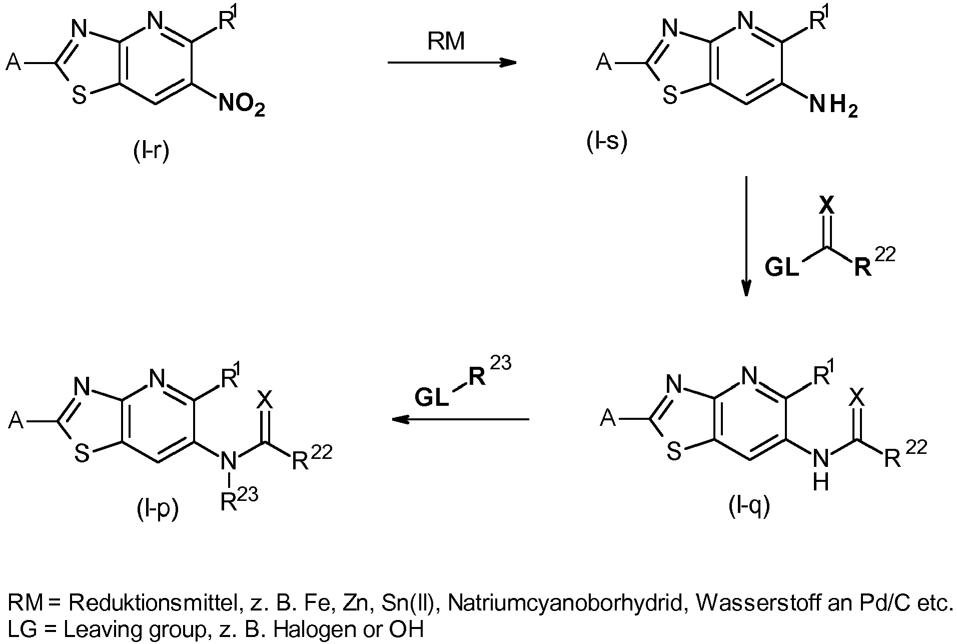

**Methode H2**: Nach Methode A hergestellte Verbindungen, in denen R² für Nitro steht (I-r), können durch Reduktion der Nitrogruppe nach literaturbekannten Methoden in die entsprechenden Aminoverbindungen (I-s) überführt werden (vgl. z. B. WO2009/14674 A1, 2009 für die Reduktion durch Fe(0); US2005/197331 A1, 2005 für die Reduktion durch Sn(II)Cl2; WO2007/86800 A1, 2007 für die Reduktion durch Natriumcyanoborhydrid). Diese lassen sich durch Acylierung und ggf. nachfolgende Alkylierung nach literaturbekannten Methoden zu den erfindungsgemäßen Verbindungen des Typs (I-q) bzw. (I-p) umsetzen.

### Methode I - Allgemeine Verfahren für die Oxidation von Thioethern zu Sulfoxiden und Sulfonen

Verbindungen der Formel (I), in welchen W für SO (Sulfoxide) oder für SO₂ (Sulfone) steht, lassen sich nach literaturbekannten Verfahren durch Oxidation aus Verbindungen der Formel (I), in welchen W für S (Thioethern) steht, herstellen, beispielsweise durch ein Oxidationsmittel in einem geeigneten Lösungs- bzw. Verdünnungsmittel. Als Oxidationsmittel eignen sich zum Beispiel verdünnte Salpetersäure, Wasserstoffperoxid, Oxone® und Peroxycarbonsäuren, wie etwa *meta*-Chlorperbenzoesäure. Als Lösungsmittel bzw. Verdünnungsmittel eignen sich inerte organische Lösungsmittel, typischerweise Acetonitril und halogenierte Lösungsmittel wie Dichlormethan, Chloroform oder Dichlorethan, sowie Wasser und Alkohole wie Methanol für die Reaktion mit Oxone®.

Möglich ist auch die Einführung geeigneter Aniline R¹-NH₂ oder Boronsäuren R¹-B(OH)₂, in welchen W für SO oder SO₂ steht, nach Verfahren □□bzw. D. Diese lassen sich aus den entsprechenden Vorstufen, in denen W für S steht, nach literaturbekannten Verfahren oxidieren, wie zum Beispiel in WO 2013/092350 beschrieben.

Zur Erzeugung enantiomeren angereicherter Sulfoxide eignen sich eine Vielfalt von Methoden, wie von G. E. O'Mahony et al., in ARKIVOC (Gainesville, FL, United states), 2011, 1, 1-110, beschrieben: Metall-katalysierte asymmetrische Oxidationen von Thioethern, zum Beispiel mit Titanium oder Vanadium als meistbenutzten Katalysatorquellen, in Form von Ti(OⁱPr₄) oderVO(acac)₂, zusammen mit einem chiralen Liganden und einem Oxidationsmittel wie tert-Butylwasserstoffperoxid (TBHP), 2-Phenylpropan-2-ylhydroperoxid (CHP) oder Wasserstoffperoxid; nicht-metall katalysierte asymmetrische Oxidationen durch Verwendung von chiralen Oxidationsmitteln oder chiralen Katalysatoren; elektrochemische oder biologische asymmetrische Oxidationen sowie kinetische Resolution von Sulfoxiden und nukleophilische Versetzung (nach Andersens Methode).

Die Enantiomere können auch aus dem Racemat gewonnen werden, beispielsweise durch präparative Trennung mittels einer chiralen HPLC.

Wenn im Folgenden von Verbindungen der Formel (I) die Rede ist, sind auch diejenigen Verbindungen in der Tabelle 1 eingeschlossen, die nicht unter die Formel (I) fallen (ausgenommen Zwischenprodukte).

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

### Verfahren und Verwendungen

Die Erfindung betrifft nicht-therapeutische Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Linepithema humile, Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor oder Flupyradifurone.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide.

Weitere Wirkstoffe mit unbekanntem oder nicht eindeutigem Wirkmechanismus, wie beispielsweise Afidopyropen, Afoxolaner, Azadirachtin, Benclothiaz, Benzoximate, Bifenazate, Bromopropylate, Chinomethionat, Cryolite, Cyclaniliprole, Cycloxaprid, Cyhalodiamide Dicloromezotiaz, Dicofol, Diflovidazin, Flometoquin, Fluensulfone, Flufenerim, Flufenoxystrobin, Flufiprole, Fluhexafon, Fluopyram, Fluralaner, Fufenozide, Guadipyr, Heptafluthrin, Imidaclothiz, Iprodione, Meperfluthrin, Paichongding, Pyflubumide, Pyridalyl, Pyrifluquinazon, Pyriminostrobin, Tetramethylfluthrin, Tetraniliprole, Tetrachlorantraniliprole, Tioxazafen, Triflumezopyrim und Iodmethan; desweiteren Präparate auf Basis von Bacillus firmus (1-1582, BioNeem, Votivo), sowie folgende bekannte wirksame Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), Methyl-2-[2-({[3-brom-1-(3-chlorpyridin-2-yl)-1H-pyrazol-5-yl]carbonyl} amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), N-[(2E)-1-[(6-Chlorpyridin-3-yl)methyl]pyridin-2(1H)-yliden]-2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969, 3-[Benzoyl(methyl)amino]-N-[2-brom-4-[1,2,2,2-tetrafluor-1-(trifluormethyl)ethyl]-6-(trifluormethyl)phenyl]-2-fluor-benzamid (bekannt aus WO 2010018714), Butyl-[2-(2,4-dichlorphenyl)-3-oxo-4-oxaspiro[4.5]dec-1-en-1-yl]-carbonat (bekannt aus CN 102060818), 4-[5-(3,5-Dichlorphenyl)-5-(trifluormethyl)-4H-isoxazol-3-yl]-N-[(Z)-methoxyimino-methyl]-2-methyl-benzamid (bekannt aus WO2007/026965), 3E)-3-[1-[(6-Chlor-3-pyridyl)methyl]-2-pyridyliden]-1,1,1-trifluor-propan-2-on (bekannt aus WO2013/144213, N-(Methylsulfonyl)-6-[2-(pyridin-3-yl)-1,3-thiazol-5-yl]pyridin-2-carboxamid (bekannt aus WO2012/000896), N-[3-(Benzylcarbamoyl)-4-chlorphenyl] -1 -methyl-3-(pentafluorethyl)-4-(trifluormethyl)-1H-pyrazol-5-carboxamid bekannt aus WO2010/051926).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).

Alle aufgeführten fungiziden Mischpartner der Klassen (1) bis (15) können optional Salze mit entsprechenden Basen oder Säuren bilden, sofern geeignete funktionelle Gruppen vorliegen. Außerdem sind für die aufgeführten fungiziden Mischpartner der Klassen (1) bis (15) auch tautomere Formen eingeschlossen, sofern Tautomerie möglich ist.
1) Inhibitoren der Ergosterolbiosynthese, zum Beispiel (1.01) Aldimorph, (1.02) Azaconazol, (1.03) Bitertanol, (1.04) Bromuconazol, (1.05) Cyproconazol, (1.06) Diclobutrazol, (1.07) Difenoconazol, (1.08) Diniconazol, (1.09) Diniconazol-M, (1.10) Dodemorph, (1.11) Dodemorphacetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-cis, (1.25) Hexaconazol, (1.26) Imazalil, (1.27) Imazalilsulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) 1 -(2,2-Dimethyl-2,3 -dihydro-1H-inden-1 -yl)-1H-imidazol-5-carbonsäuremethylester, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'- {2-methyl-5-(trifluormethyl)-4-[3-(trimethylsilyl)propoxy]phenyl}imidoformamid, (1.64) O-[1-(4-Methoxyphenoxy)-3,3-dimethylbutan-2-yl]-1H-imidazol-1-carbothioat, (1.65) Pyrisoxazol, (1.66) 2-{[3-(2-Chlorphenyl)-2-(2,4-difluorphenyl)oxiran-2-yl]methyl}-2,4-dihydro-3H-1,2,4-triazol-3-thion, (1.67) 1-{[3-(2-Chlorphenyl)-2-(2,4-difluorphenyl)oxiran-2-yl]methyl}-1H-1,2,4-triazol-5-ylthiocyanat, (1.68) 5-(Allylsulfanyl)-1-{[3-(2-chlorphenyl)-2-(2,4-difluorphenyl)oxiran-2-yl]methyl}-1H-1,2,4-triazol, (1.69) 2-[1-(2,4-Dichlorphenyl)-5-hydroxy-2,6,6-trimethylheptan-4-yl]-2,4-dihydro-3H-1,2,4-triazol-3-thion, (1.70) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.71) 2-{[rel(2R,3R)-3-(2-Chlorphenyl)-2-(2,4-difluorphenyl)¬oxiran-2-yl]methyl}-2,4-dihydro-3H-1,2,4-triazol-3-thion, (1.72) 1-{[rel(2R,3S)-3-(2-Chlorphenyl)-2-(2,4-difluorphenyl)oxiran-2-yl]methyl}-1H-1,2,4-triazol-5-ylthiocyanat, (1.73) 1-{[rel(2R,3R)-3-(2-Chlorphenyl)-2-(2,4-difluorphenyl)oxiran-2-yl]methyl}-1H-1,2,4-triazol-5-ylthiocyanat, (1.74) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.75) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.76) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.77) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.78) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.79) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.80) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.81) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (1.82) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.83) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.84) 2-[4-(4-Chlorphenoxy)-2-(trifluormethyl)phenyl]-1-(1H-1,2,4-triazol-1-yl)propan-2-ol, (1.85) 2-[4-(4-Chlorphenoxy)-2-(trifluormethyl)phenyl]-1-(1H-1,2,4-triazol-1-yl)butan-2-ol, (1.86) 2-[4-(4-Chlorphenoxy)-2-(trifluormethyl)phenyl]-1-(1H-1,2,4-triazol-1-yl)pentan-2-ol, (1.87) 2-[2-Chlor-4-(4-chlorphenoxy)phenyl]-1-(1H-1,2,4-triazol-1-yl)butan-2-ol, (1.88) 2-[2-Chlor-4-(2,4-dichlorphenoxy)phenyl]-1-(1H-1,2,4-triazol-1-yl)propan-2-ol, (1.89) (2R)-2-(1-Chlorcyclopropyl)-4-[(1R)-2,2-dichlorcyclopropyl]-1-(1H-1,2,4-triazol-1-yl)butan-2-ol, (1.90) (2R)-2-(1-Chlorcyclopropyl)-4-[(1S)-2,2-dichlorcyclopropyl]-1-(1H-1,2,4-triazol-1-yl)butan-2-ol, (1.91) (2S)-2-(1-Chlorcyclopropyl)-4-[(1S)-2,2-dichlorcyclopropyl]-1-(1H-1,2,4-triazol-1-yl)butan-2-ol, (1.92) (2S)-2-(1-Chlorcyclopropyl)-4-[(1R)-2,2-dichlorcyclopropyl]-1-(1H-1,2,4-triazol-1-yl)butan-2-ol, (1.93) (1S,2R,5R)-5-(4-Chlorbenzyl)-2-(chlormethyl)-2-methyl-1-(1H-1,2,4-triazol-1-ylmethyl)cyclopentanol, (1.94) (1R,2S,5S)-5-(4-Chlorbenzyl)-2-(chlormethyl)-2-methyl-1-(1H-1,2,4-triazol-1-ylmethyl)cyclopentanol, (1.95) 5-(4-Chlorbenzyl)-2-(chlormethyl)-2-methyl-1-(1H-1,2,4-triazol-1-ylmethyl)cyclopentanol.
2) Inhibitoren der Atmungskette am Komplex I oder II, zum Beispiel (2.01) Bixafen, (2.02) Boscalid, (2.03) Carboxin, (2.04) Diflumetorim, (2.05) Fenfuram, (2.06) Fluopyram, (2.07) Flutolanil, (2.08) Fluxapyroxad, (2.09) Furametpyr, (2.10) Furmecyclox, (2.11) Isopyrazam (Mischung von syn-epimerem Racemat 1RS,4SR,9RS und anti-epimerem Racemat 1RS,4SR,9SR), (2.12) Isopyrazam (anti-epimeres Racemat 1RS,4SR,9SR), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Racemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxan, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]chinazolin-4-amin, (2.29) Benzovindiflupyr, (2.30) N-[(1S,4R)-9-(Dichlormethylen)-1,2,3,4-tetrahydro-1,4-methanonaphthalin-5-yl]-3-(difluormethyl)-1-methyl-1H-pyrazol-4-carboxamid, (2.31) N-[(1R,4S)-9-(Dichlormethylen)-1,2,3,4-tetrahydro-1,4-methanonaphthalin-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridin-3-carboxamid, (2.43) Isofetamid, (2.44) 1-Methyl-3-(trifluormethyl)-N-[2'-(trifluormethyl)biphenyl-2-yl]-1H-pyrazol-4-carboxamid, (2.45) N-(4'-Chlorbiphenyl-2-yl)-3-(difluormethyl)-1 -methyl-1H-pyrazol-4-carboxamid, (2.46) N-(2',4'-Dichlorbiphenyl-2-yl)-3 - (difluormethyl)-1-methyl-1H-pyrazol-4-carboxamid, (2.47) 3-(Difluormethyl)-1-methyl-N-[4'-(trifluormethyl)biphenyl-2-yl] -1H-pyrazol-4-carboxamid, (2.48) N-(2',5'-Difluorbiphenyl-2-yl)-1 - methyl-3-(trifluormethyl)-1H-pyrazol-4-carboxamid, (2.49) 3-(Difluormethyl)-1-methyl-N-[4'-(prop-1-in-1-yl)biphenyl-2-yl]-1H-pyrazol-4-carboxamid, (2.50) 5-Fluor-1,3-dimethyl-N-[4'-(prop-1-in-1-yl)biphenyl-2-yl]-1H-pyrazol-4-carboxamid, (2.51) 2-Chlor-N-[4'-(prop-1-in-1-yl)biphenyl-2-yl]nicotinamid, (2.52) 3-(Difluormethyl)-N-[4'-(3,3-dimethylbut-1-in-1-yl)biphenyl-2-yl]-1-methyl-1H-pyrazol-4-carboxamid, (2.53) N-[4'-(3,3-Dimethylbut-1-in-1-yl)biphenyl-2-yl]-5-fluor-1,3-dimethyl-1H-pyrazol-4-carboxamid, (2.54) 3-(Difluormethyl)-N-(4'-ethinylbiphenyl-2-yl)-1-methyl-1H-pyrazol-4-carboxamid, (2.55) N-(4'-Ethinylbiphenyl-2-yl)-5-fluor-1,3-dimethyl-1H-pyrazol-4-carboxamid, (2.56) 2-Chlor-N-(4'-ethinylbiphenyl-2-yl)nicotinamid, (2.57) 2-Chlor-N-[4'-(3,3-dimethylbut-1-in-1-yl)biphenyl-2-yl]nicotinamid, (2.58) 4-(Difluormethyl)-2-methyl-N-[4'-(trifluormethyl)biphenyl-2-yl]-1,3-thiazol-5-carboxamid, (2.59) 5-Fluor-N-[4'-(3-hydroxy-3-methylbut-1-in-1-yl)biphenyl-2-yl]-1,3-dimethyl-1H-pyrazol-4-carboxamid, (2.60) 2-Chlor-N-[4'-(3-hydroxy-3-methylbut-1-in-1-yl)biphenyl-2-yl]nicotinamid, (2.61) 3-(Difluormethyl)-N-[4'-(3-methoxy-3-methylbut-1-in-1-yl)biphenyl-2-yl]-1-methyl-1H-pyrazol-4-carboxamid, (2.62) 5-Fluor-N-[4'-(3-methoxy-3-methylbut-1-in-1-yl)biphenyl-2-yl]-1,3-dimethyl-1H-pyrazol-4-carboxamid, (2.63) 2-Chlor-N-[4'-(3-methoxy-3-methylbut-1-in-1-yl)biphenyl-2-yl]nicotinamid, (2.64) 1,3-Dimethyl-N-(1,1,3-trimethyl-2,3-dihydro-1H-inden-4-yl)-1H-pyrazol-4-carboxamid, (2.65) 1,3-Dimethyl-N-[(3R)-1,1,3-trimethyl-2,3-dihydro-1H-inden-4-yl]-1H-pyrazol-4-carboxamid, (2.66) 1,3-Dimethyl-N-[(3S)-1,1,3-trimethyl-2,3-dihydro-1H-inden-4-yl]-1H-pyrazol-4-carboxamid, (2.67) 3-(Difluormethyl)-N-methoxy-1-methyl-N-[1-(2,4,6-trichlor-phenyl)propan-2-yl]-1H-pyrazol-4-carboxamid, (2.68) 3-(Difluormethyl)-N-(7-fluor-1,1,3-trimethyl-2,3-dihydro-1H-inden-4-yl)-1-methyl-1H-pyrazol-4-carboxamid, (2.69) 3-(Difluormethyl)-N-[(3R)-7-fluor-1,1,3-trimethyl-2,3-dihydro-1H-inden-4-yl]-1-methyl-1H-pyrazol-4-carboxamid, (2.70) 3-(Difluormethyl)-N-[(3S)-7-fluor-1,1,3-trimethyl-2,3-dihydro-1H-inden-4-yl]-1-methyl-1H-pyrazol-4-carboxamid.
3) Inhibitoren der Atmungskette am Komplex III, zum Beispiel (3.01) Ametoctradin, (3.02) Amisulbrom, (3.03) Azoxystrobin, (3.04) Cyazofamid, (3.05) Coumethoxystrobin, (3.06) Coumoxystrobin, (3.07) Dimoxystrobin, (3.08) Enoxastrobin, (3.09) Famoxadon, (3.10) Fenamidon, (3.11) Flufenoxystrobin, (3.12) Fluoxastrobin, (3.13) Kresoxim-methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methyl¬acetamid, (3.24) (2E)-2-(Methoxyimino)-N-methyl-2-(2-{[({(1E)-1-[3-(trifluormethyl)phenyl]¬ethyliden}¬amino)oxy]methyl}phenyl)acetamid, (3.25) (2E)-2-(Methoxyimino)-N-methyl-2-{2-[(E)-({1-[3-(trifluormethyl)phenyl]ethoxy}imino)methyl]phenyl}acetamid, (3.26) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methyl-acetamid, (3.27) Fenaminostrobin, (3.28) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.29) (2E)-2- {2-[({Cyclopropyl[(4-methoxyphenyl)imino]methyl}sulfanyl)methyl]phenyl}-3-methoxyacryl-säuremethylester, (3.30) N-(3-Ethyl-3,5,5-trimethylcyclohexyl)-3-formamido-2-hydroxybenzamid, (3.31) 2-{2-[(2,5-Dimethylphenoxy)methyl]phenyl}-2-methoxy-N-methylacetamid, (3.32) 2-{2-[(2,5-Dimethylphenoxy)methyl]phenyl}-2-methoxy-N-methylacetamid, (3.33) (2E,3Z)-5-{[1-(4-Chlorphenyl)-1H-pyrazol-3-yl]oxy}-2-(methoxyimino)-N,3-dimethylpent-3-enamid.
4) Inhibitoren der Mitose und Zellteilung, zum Beispiel (4.01) Benomyl, (4.02) Carbendazim, (4.03) Chlorfenazol, (4.04) Diethofencarb, (4.05) Ethaboxam, (4.06) Fluopicolid, (4.07) Fuberidazol, (4.08) Pencycuron, (4.09) Thiabendazol, (4.10) Thiophanat-methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin, (4.14) 3-Chlor-5-(6-chlorpyridin-3-yl)-6-methyl-4-(2,4,6-trifluorphenyl)pyridazin.
5) Verbindungen, die dazu fähig sind, an mehreren Stellen anzugreifen ("Multisite Action"), zum Beispiel (5.01) Bordeaux-Mischung, (5.02) Captafol, (5.03) Captan, (5.04) Chlorothalonil, (5.05) Kupferhydroxid, (5.06) Kupfernaphthenat, (5.07) Kupferoxid, (5.08) Kupferoxychlorid, (5.09) Kupfer(2+)-sulfat, (5.10) Dichlofluanid, (5.11) Dithianon, (5.12) Dodin, (5.13) Dodin freie Base, (5.14) Ferbam, (5.15) Fluorofolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mancopper, (5.23) Mancozeb, (5.24) Maneb, (5.25) Metiram, (5.26) Metiram-Zink, (5.27) Oxin-Kupfer, (5.28) Propamidin, (5.29) Propineb, (5.30) Schwefel und Schwefelzubereitungen einschließlich Calciumpolysulfid, (5.31) Thiram, (5.32) Tolylfluanid, (5.33) Zineb, (5.34) Ziram, (5.35) Anilazin.
6) Verbindungen, die dazu fähig sind, eine Abwehrreaktion des Wirtes zu induzieren, zum Beispiel (6.01) Acibenzolar-S-methyl, (6.02) Isotianil, (6.03) Probenazol, (6.04) Tiadinil, (6.05) Laminarin.
7) Inhibitoren der Aminosäure- und/oder Proteinbiosynthese, zum Beispiel (7.01) Andoprim, (7.02) Blasticidin-S, (7.03) Cyprodinil, (7.04) Kasugamycin, (7.05) Kasugamycinhydrochlorid-hydrat, (7.06) Mepanipyrim, (7.07) Pyrimethanil, (7.08) 3-(5-Fluor-3,3,4,4-tetramethyl-3,4-dihydroisochinolin-1-yl)chinolin, (7.09) Oxytetracyclin, (7.10) Streptomycin.
8) Inhibitoren der ATP-Produktion, zum Beispiel (8.01) Fentinacetat, (8.02) Fentinchlorid, (8.03) Fentinhydroxid, (8.04) Silthiofam.
9) Inhibitoren der Zellwandsynthese, zum Beispiel (9.01) Benthiavalicarb, (9.02) Dimethomorph, (9.03) Flumorph, (9.04) Iprovalicarb, (9.05) Mandipropamid, (9.06) Polyoxine, (9.07) Polyoxorim, (9.08) Validamycin A, (9.09) Valifenalat, (9.10) Polyoxin B, (9.11) (2E)-3-(4-tert.-Butylphenyl)-3-(2-chlorpyridin-4-yl)-1-(morpholin-4-yl)prop-2-en-1-on, (9.12) (2Z)-3-(4-tert.-Butylphenyl)-3-(2-chlorpyridin-4-yl)-1-(morpholin-4-yl)prop-2-en-1-on.
10) Inhibitoren der Lipid- und Membransynthese, zum Beispiel (10.01) Biphenyl, (10.02) Chloroneb, (10.03) Dicloran, (10.04) Edifenphos, (10.05) Etridiazol, (10.06) Iodocarb, (10.07) Iprobenfos, (10.08) Isoprothiolan, (10.09) Propamocarb, (10.10) Propamocarbhydrochlorid, (10.11) Prothiocarb, (10.12) Pyrazophos, (10.13) Quintozen, (10.14) Tecnazen, (10.15) Tolclofos-methyl.
11) Inhibitoren der Melaninbiosynthese, zum Beispiel (11.01) Carpropamid, (11.02) Diclocymet, (11.03) Fenoxanil, (11.04) Phthalid, (11.05) Pyroquilon, (11.06) Tricyclazol, (11.07) 2,2,2-Trifluorethyl {3-methyl-1-[(4-methylbenzoyl)amino]butan-2-yl}carbamat.
12) Inhibitoren der Nukleinsäuresynthese, zum Beispiel (12.01) Benalaxyl, (12.02) Benalaxyl-M (Kiralaxyl), (12.03) Bupirimat, (12.04) Clozylacon, (12.05) Dimethirimol, (12.06) Ethirimol, (12.07) Furalaxyl, (12.08) Hymexazol, (12.09) Metalaxyl, (12.10) Metalaxyl-M (Mefenoxam), (12.11) Ofurace, (12.12) Oxadixyl, (12.13) Oxolinsäure, (12.14) Octhilinon.
13) Inhibitoren der Signalvermittlung, zum Beispiel (13.01) Chlozolinat, (13.02) Fenpiclonil, (13.03) Fludioxonil, (13.04) Iprodion, (13.05) Procymidon, (13.06) Quinoxyfen, (13.07) Vinclozolin, (13.08) Proquinazid.
14) Verbindungen, die als Entkoppler wirken können, zum Beispiel (14.01) Binapacryl, (14.02) Dinocap, (14.03) Ferimzon, (14.04) Fluazinam, (14.05) Meptyldinocap.
15) Weitere Verbindungen, zum Beispiel (15.001) Benthiazol, (15.002) Bethoxazin, (15.003) Capsimycin, (15.004) Carvon, (15.005) Chinomethionat, (15.006) Pyriofenon (Chlazafenon), (15.007) Cufraneb, (15.008) Cyflufenamid, (15.009) Cymoxanil, (15.010) Cyprosulfamid, (15.011) Dazomet, (15.012) Debacarb, (15.013) Dichlorophen, (15.014) Diclomezin, (15.015) Difenzoquat, (15.016) Difenzoquatmetilsulfat, (15.017) Diphenylamin, (15.018) Ecomat, (15.019) Fenpyrazamin, (15.020) Flumetover, (15.021) Fluoroimid, (15.022) Flusulfamid, (15.023) Flutianil, (15.024) Fosetyl-Aluminium, (15.025) Fosetyl-Calcium, (15.026) Fosetyl-Natrium, (15.027) Hexachlorbenzol, (15.028) Irumamycin, (15.029) Methasulfocarb, (15.030) Methylisothiocyanat, (15.031) Metrafenon, (15.032) Mildiomycin, (15.033) Natamycin, (15.034) Nickeldimethyldithiocarbamat, (15.035) Nitrothalisopropyl, (15.036) Oxamocarb, (15.037) Oxyfenthiin, (15.038) Pentachlorphenol und Salze, (15.039) Phenothrin, (15.040) phosphorige Säure und deren Salze, (15.041) Propamocarb-fosetylat, (15.042) Propanosin-Natrium, (15.043) Pyrimorph, (15.044) Pyrrolnitrin, (15.045) Tebufloquin, (15.046) Tecloftalam, (15.047) Tolnifanid, (15.048) Triazoxid, (15.049) Trichlamid, (15.050) Zarilamid, (15.051) 2-Methylpropansäure-(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-ylester, (15.052) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.053) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.054) Oxathiapiprolin, (15.055) 1H-Imidazol-1-carbonsäure-1-(4-methoxyphenoxy)-3,3-dimethylbutan-2-ylester, (15.056) 2,3,5,6-Tetrachlor-4-(methylsulfonyl)pyridin, (15.057) 2,3-Dibutyl-6-chlorthieno[2,3-d]pyrimidin-4(3H)-on, (15.058) 2,6-Dimethyl-1H,5H-[1,4]dithiino[2,3-c:5,6-c']dipyrrol-1,3,5,7(2H,6H)-tetron, (15.059) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.060) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.061) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.062) 2-Butoxy-6-iod-3-propyl-4H-chromen-4-on, (15.063) 2-Chlor-5-[2-chlor-1-(2,6-difluor-4-methoxyphenyl)-4-methyl-1H-imidazol-5-yl]pyridin, (15.064) 2-Phenylphenol und Salze, (15.065) 3-(4,4,5-Trifluor-3,3-dimethyl-3,4-dihydroisochinolin-1-yl)chinolin, (15.066) 3,4,5-Trichlorpyridin-2,6-dicarbonsäurenitril, (15.067) 3-Chlor-5-(4-chlorphenyl)-4-(2,6-difluorphenyl)-6-methylpyridazin, (15.068) 4-(4-Chlorphenyl)-5-(2,6-difluorphenyl)-3,6-dimethylpyridazin, (15.069) 5-Amino-1,3,4-thiadiazol-2-thiol, (15.070) 5-Chlor-N'-phenyl-N'-(prop-2-in-1-yl)thiophen-2-sulfonohydrazid, (15.071) 5-Fluor-2-[(4-fluorbenzyl)oxy]pyrimidin-4-amin, (15.072) 5-Fluor-2-[(4-methylbenzyl)oxy]pyrimidin-4-amin, (15.073) 5-Methyl-6-octyl[1,2,4]triazolo[1,5-a]pyrimidin-7-amin, (15.074) (2Z)-3-Amino-2-cyano-3-phenylacrylsäureethylester, (15.075) N'-(4-{[3-(4-Chlorbenzyl)-l,2,4-thiadiazol-5-yl]oxy}-2,5-dimethylphenyl)-N-ethyl-N-methylimidoformamid, (15.076) N-(4-Chlorbenzyl)-3-[3-methoxy-4-(prop-2-in-1-yloxy)phenyl]propanamid, (15.077) N-[(4-Chlorphenyl)(cyano)methyl]-3-[3-methoxy-4-(prop-2-in-1-yloxy)phenyl]propanamid, (15.078) N-[(5-Brom-3-chlorpyridin-2-yl)methyl]-2,4-dichlor-nicotinamid, (15.079) N-[1-(5-Brom-3-chlorpyridin-2-yl)ethyl]-2,4-dichlornicotinamid, (15.080) N-[1-(5-Brom-3-chlorpyridin-2-yl)ethyl]-2-fluor-4-iodnicotinamid, (15.081) N-{(E)-[(Cyclopropyl-methoxy)imino][6-(difluormethoxy)-2,3-difluorphenyl]methyl}-2-phenylacetamid, (15.082) N-{(Z)-[(Cyclopropylmethoxy)imino][6-(difluormethoxy)-2,3-difluorphenyl]methyl}-2-phenylacetamid, (15.083) N'-{4-[(3-tert.-Butyl-4-cyano-1,2-thiazol-5-yl)oxy]-2-chlor-5-methylphenyl}-N-ethyl-N-methylimidoformamid, (15.084) N-Methyl-2-(1-{[5-methyl-3-(trifluormethyl)-1H-pyrazol-1-yl]acetyl}piperidin-4-yl)-N-(1,2,3,4-tetrahydronaphthalin-1-yl)-1,3-thiazol-4-carboxamid, (15.085) N-Methyl-2-(-{[5-methyl-3-(trifluormethyl)-1H-pyrazol-1-yl]acetyl}piperidin-4-yl)-N-[(1R)-1,2,3,4-tetrahydronaphthalin-1-yl]-1,3-thiazol-4-carboxamid, (15.086) N-Methyl-2-(1-{[5-methyl-3-(trifluormethyl)-1H-pyrazol-1-yl]acetyl}piperidin-4-yl)-N-[(1S)-1,2,3,4-tetrahydronaphthalin-1-yl]-1,3-thiazol-4-carboxamid, (15.087) {6-[({[(1-Methyl-1H-tetrazol-5-yl)(phenyl)methylen]amino}oxy)methyl]pyridin-2-yl}¬carbamidsäurepentylester, (15.088) Phenazin-1-carbonsäure, (15.089) Chinolin-8-ol, (15.090) Chinolin-8-olsulfat (2:1), (15.091) {6-[({[(1-Methyl-1H-tetrazol-5-yl)(phenyl)methylen]¬amino}¬oxy)methyl]pyridin-2-yl}carbamidsäure-tert.-butylester, (15.092) (5-Brom-2-methoxy-4-methyl¬pyridin-3-yl)(2,3,4-trimethoxy-6-methylphenyl)methanon, (15.093) N-[2-(4-{[3-(4-Chlorphenyl)prop-2-in-1-yl]oxy}-3-methoxyphenyl)ethyl]-N2-(methylsulfonyl)valinamid, (15.094) 4-Oxo-4-[(2-phenylethyl)amino]butansäure, (15.095) {6-[({[(Z)-(1-Methyl-1H-tetrazol-5-yl)(phenyl)methylen]¬amino}oxy)methyl]pyridin-2-yl}carbamidsäurebut-3-in-1-ylester, (15.096) 4-Amino-5-fluor¬pyrimidin-2-ol (tautomere Form: 4-Amino-5-fluorpyrimidin-2(1H)-on), (15.097) 3,4,5-Trihydroxybenzoesäurepropylester, (15.098) [3-(4-Chlor-2-fluorphenyl)-5-(2,4-difluorphenyl)-1,2-oxazol-4-yl](pyridin-3-yl)methanol, (15.099) (S)-[3-(4-Chlor-2-fluorphenyl)-5-(2,4-difluorphenyl)-1,2-oxazol-4-yl](pyridin-3-yl)methanol, (15.100) (R)-[3-(4-Chlor-2-fluorphenyl)-5-(2,4-difluorphenyl)-1,2-oxazol-4-yl](pyridin-3-yl)methanol, (15.101) 2-Fluor-6-(trifluormethyl)-N-(1,1,3-trimethyl-2,3-dihydro-1H-inden-4-yl)benzamid, (15.102) 2-(6-Benzylpyridin-2-yl)chinazolin, (15.103) 2-[6-(3-Fluor-4-methoxyphenyl)-5-methylpyridin-2-yl]chinazolin, (15.104) 3-(4,4-Difluor-3,3-dimethyl-3,4-dihydroisochinolin-1-yl)chinolin, (15.105) Abscisinsäure, (15.106) N'-[5-Brom-6-(2,3-dihydro-1H-inden-2-yloxy)-2-methylpyridin-3-yl]-N-ethyl-N-methylimidoformamid, (15.107) N'- {5-Brom-6-[1-(3,5-difluorphenyl)ethoxy]-2-methylpyridin-3-yl}-N-ethyl-N-methylimidoformamid, (15.108) N'-{5-Brom-6-[(1R)-1-(3,5-difluorphenyl)ethoxy]-2-methylpyridin-3-yl}-N-ethyl-N-methylimidoformamid, (15.109) N'-{5-Brom-6-[(1S)-1-(3,5-difluorphenyl)ethoxy]-2-methylpyridin-3-yl}-N-ethyl-N-methylimidoformamid, (15.110) N'-{5-Brom-6-[(cis-4-isopropylcyclohexyl)oxy]-2-methylpyridin-3-yl}-N-ethyl-N-methylimidoformamid, (15.111) N'-{5-Brom-6-[(trans-4-isopropylcyclohexyl)oxy]-2-methylpyridin-3-yl}-N-ethyl-N-methylimidoformamid, (15.112) N-Cyclopropyl-3-(difluormethyl)-5-fluor-N-(2-isopropylbenzyl)-1-methyl-1H-pyrazol-4-carboxamid, (15.113) N-Cyclopropyl-N-(2-cyclopropylbenzyl)-3-(difluormethyl)-5-fluor-1-methyl-1H-pyrazol-4-carboxamid, (15.114) N-(2-tert.-Butylbenzyl)-N-cyclopropyl-3-(difluormethyl)-5-fluor-1-methyl-1H-pyrazol-4-carboxamid, (15.115) N-(5-Chlor-2-ethylbenzyl)-N-cyclopropyl-3-(difluormethyl)-5-fluor-1-methyl-1H-pyrazol-4-carboxamid, (15.116) N-(5-Chlor-2-isopropylbenzyl)-N-cyclopropyl-3-(difluormethyl)-5-fluor-1-methyl-1H-pyrazol-4-carboxamid, (15.117) N-Cyclopropyl-3-(difluormethyl)-N-(2-ethyl-5-fluorbenzyl)-5-fluor-1-methyl-1H-pyrazol-4-carboxamid, (15.118) N-Cyclopropyl-3-(difluormethyl)-5-fluor-N-(5-fluor-2-isopropylbenzyl)-1-methyl-1H-pyrazol-4-carboxamid, (15.119) N-Cyclopropyl-N-(2-cyclopropyl-5-fluorbenzyl)-3-(difluormethyl)-5-fluor-1-methyl-1H-pyrazol-4-carboxamid, (15.120) N-(2-Cyclopentyl-5-fluorbenzyl)-N-cyclopropyl-3 -(difluormethyl)-5 -fluor-1 -methyl-1H-pyrazol-4-carboxamid, (15.121) N-Cyclopropyl-3-(difluormethyl)-5-fluor-N-(2-fluor-6-isopropylbenzyl)-1-methyl-1H-pyrazol-4-carboxamid, (15.122) N-Cyclopropyl-3-(difluormethyl)-N-(2-ethyl-5-methylbenzyl)-5-fluor-1-methyl-1H-pyrazol-4-carboxamid, (15.123) N-Cyclopropyl-3-(difluormethyl)-5-fluor-N-(2-isopropyl-5-methylbenzyl)-1-methyl-1H-pyrazol-4-carboxamid, (15.124) N-Cyclopropyl-N-(2-cyclopropyl-5-methylbenzyl)-3-(difluormethyl)-5-fluor-1-methyl-1H-pyrazol-4-carboxamid, (15.125) N-(2-tert.-Butyl-5-methylbenzyl)-N-cyclopropyl-3-(difluormethyl)-5-fluor-1-methyl-1H-pyrazol-4-carboxamid, (15.126) N-[5-Chlor-2-(trifluormethyl)benzyl]-N-cyclopropyl-3-(difluormethyl)-5-fluor-1-methyl-1H-pyrazol-4-carboxamid, (15.127) N-Cyclopropyl-3-(difluormethyl)-5-fluor-1-methyl-N-[5-methyl-2-(trifluormethyl)benzyl] -1H-pyrazol-4-carboxamid,(15.128)N-[2-Chlor-6-(trifluormethyl)benzyl] -N-cyclopropyl-3-(difluormethyl)-5-fluor-1-methyl-1H-pyrazol-4-carboxamid, (15.129) N-[3-Chlor-2-fluor-6-(trifluormethyl)benzyl]-N-cyclopropyl-3-(difluormethyl)-5-fluor-1-methyl-1H-pyrazol-4-carboxamid, (15.130) N-Cyclopropyl-3-(difluormethyl)-N-(2-ethyl-4,5-dimethylbenzyl)-5-fluor-1-methyl-1H-pyrazol-4-carboxamid, (15.131) N-Cyclopropyl-3-(difluormethyl)-5-fluor-N-(2-isopropylbenzyl)-1-methyl-1H-pyrazol-4-carbothioamid, (15.132) N'-(2,5-Dimethyl-4-phenoxyphenyl)-N-ethyl-N-methylimidoformamid, (15.133) N'-{4-[(4,5-Dichlor-1,3-thiazol-2-yl)oxy]-2,5-dimethylphenyl}-N-ethyl-N-methylimidoformamid, (15.134) N-(4-Chlor-2,6-difluorphenyl)-4-(2-chlor-4-fluorphenyl)-1,3-dimethyl-1H-pyrazol-5-amin, (15.135) 9-Fluor-2,2-dimethyl-5-(chinolin-3-yl)-2,3-dihydro-1,4-benzoxazepin, (15.136) 2-{2-Fluor-6-[(8-fluor-2-methylchinolin-3-yl)oxy]phenyl}propan-2-ol, (15.137)2-{2-[(7,8-Difluor-2-methylchmolm-3-yl)oxy]-6-fluorphenyl}propan-2-ol, (15.138) 4-(2-Chlor-4-fluorphenyl)-N-(2-fluorphenyl)-1,3-dimethyl-1H-pyrazol-5-amin, (15.139) 4-(2-Chlor-4-fluorphenyl)-N-(2,6-difluorphenyl)-1,3-dimethyl-1H-pyrazol-5-amin, (15.140) 4-(2-Chlor-4-fluorphenyl)-N-(2-chlor-6-fluorphenyl)-1,3-dimethyl-1H-pyrazol-5-amin, (15.141) 4-(2-Brom-4-fluorphenyl)-N-(2-chlor-6-fluorphenyl)-1,3-dimethyl-1H-pyrazol-5-amin, (15.142) N-(2-Brom-6-fluorphenyl)-4-(2-chlor-4-fluorphenyl)-1,3-dimethyl-1H-pyrazol-5-amin, (15.143) 4-(2-Brom-4-fluorphenyl)- N -(2-bromphenyl)-1,3-dimethyl-1H -pyrazol-5-amin, (15.144) 4-(2-Brom-4-fluorphenyl)-N-(2-brom-6-fluorphenyl)-1,3-dimethyl-1H-pyrazol-5-amin, (15.145) 4-(2-Brom-4-fluorphenyl)-N-(2-chlorphenyl)-1,3-dimethyl-1H-pyrazol-5-amin, (15.146) N-(2-Bromphenyl)-4-(2-chlor-4-fluorphenyl)-1,3-dimethyl-1H-pyrazol-5-amin, (15.147) 4-(2-Chlor-4-fluorphenyl)-N-(2-chlorphenyl)-1,3-dimethyl-1H-pyrazol-5-amin, (15.148) 4-(2-Brom-4-fluorphenyl)-N-(2,6-difluorphenyl)-1,3-dimethyl-1H-pyrazol-5-amin, (15.149) 4-(2-Brom-4-fluorphenyl)-N-(2-fluorphenyl)-1,3-dimethyl-1H-pyrazol-5-amin, (15.150) N'-(4-{3-[(Difluormethyl)sulfanyl]phenoxy}-2,5-dimethylphenyl)-N-ethyl-N-methylimidoformamid, (15.151) N'-(2,5-Dimethyl-4-{3-[(1,1,2,2-tetrafluorethyl)sul-fanyl]phenoxy}phenyl)-N-ethyl-N-methylimidoformamid, (15.152) N'-(2,5-Dimethyl-4-{3-[(2,2,2-trifluorethyl)sulfanyl]phenoxy}phenyl)-N-ethyl-N-methylimidoformamid, (15.153) N'-(2,5-Dimethyl-4-{3-[(2,2,3,3-tetrafluorpropyl)sulfanyl]phenoxy}phenyl)-N-ethyl-N-methylimidoformamid, (15.154) N'-(2,5-Dimethyl-4-{3-[(pentafluorethyl)sulfanyl]phenoxy}phenyl)-N-ethyl-N-methylimidoformamid, (15.155) N'-(4-{[3-(Difluormethoxy)phenyl]sulfanyl}-2,5-dimethylphenyl)-N-ethyl-N-methylimido-formamid, (15.156) N'-(2,5-Dimethyl-4-{[3-(1,1,2,2-tetrafluorethoxy)phenyl]sulfanyl}phenyl)-N-ethyl-N-methylimidoformamid, (15.157) N'-(2,5-Dimethyl-4- {[3-(2,2,2-trifluorethoxy)phenyl]sulfanyl}phenyl)-N-ethyl-N-methylimidoformamid, (15.158) N'-(2,5-Dimethyl-4- {[3-(2,2,3,3-tetrafluorpropoxy)phenyl]sulfanyl}phenyl)-N-ethyl-N-methylimidoformamid, (15.159) N'-(2,5-Dimethyl-4-{[3-(pentafluorethoxy)phenyl]sulfanyl}phenyl)-N-ethyl-N-methylimidoformamid, (15.160) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.161) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.162) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.163) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.164) 2-{3-[2-(1-{[3,5-Bis(difluormethyl)-1H-pyrazol-1-yl]¬acetyl}piperidin-4-yl)-1,3-thiazol-4-yl]-4,5-dihydro-1,2-oxazol-5-yl}-3-chlorphenyl-methansulfonat, (15.165) 2-[3,5-Bis(difluormethyl)-1H-pyrazol-1-yl]-1-[4-(4-{(5S)-5-[2-(prop-2-in-1-yloxy)phenyl]-4,5-dihydro-1,2-oxazol-3-yl}-1,3-thiazol-2-yl)piperidm-1-yl]ethanon, (15.166) 2-[3,5-Bis(difluormethyl)-1H-pyrazol-1-yl]-1-[4-(4-{(5R)-5-[2-(prop-2-in-1-yloxy)phenyl]-4,5-dihydro-1,2-oxazol-3-yl}-1,3-thiazol-2-yl)piperidin-1-yl]ethanon, (15.167) 2-[3,5-Bis(difluormethyl)-1H-pyrazol-1-yl]-1-[4-(4-{(5S)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.168) 2-[3,5-Bis(difluormethyl)-1H-pyrazol-1-yl]-1-[4-(4-{(5R)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.169) 2-[3,5-Bis(difluormethyl)-1H-pyrazol-1-yl]-1-[4-(4-{(5S)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.170) 2-[3,5-Bis(difluormethyl)-1H-pyrazol-1-yl]-1-[4-(4-{(5R)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.171) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.172) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.173) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.174) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
Bacillus amyloliquefaciens, Stamm FZB42 (DSM 231179), oder Bacillus cereus, insbesondere B. cereus Stamm CNCM I-1562 oder Bacillus firmus, Stamm I-1582 (Accession number CNCM I-1582) oder Bacillus pumilus, insbesondere Stamm GB34 (Accession No. ATCC 700814) und Stamm QST2808 (Accession No. NRRL B-30087), oder Bacillus subtilis, insbesondere Stamm GB03 (Accession No. ATCC SD-1397), oder Bacillus subtilis Stamm QST713 (Accession No. NRRL B-21661) oder Bacillus subtilis Stamm OST 30002 (Accession No. NRRL B-50421) Bacillus thuringiensis, insbesondere B. thuringiensis subspecies israelensis (serotype H-14), Stamm AM65-52 (Accession No. ATCC 1276), oder B. thuringiensis subsp. aizawai, insbesondere Stamm ABTS-1857 (SD-1372), oder B. thuringiensis subsp. kurstaki Stamm HD-1, oder B. thuringiensis subsp. tenebrionis Stamm NB 176 (SD-5428), Pasteuria penetrans, Pasteuria spp. (Rotylenchulus reniformis nematode)-PR3 (Accession Number ATCC SD-5834), Streptomyces microflavus Stamm AQ6121 (= QRD 31.013, NRRL B-50550), Streptomyces galbus Stamm AQ 6047 (Acession Number NRRL 30232).

Beispiele für Pilze und Hefen, die als biologische Schädlingsbekämpfungsmittel eingesetzt werden bzw. verwendet werden können, sind:
Beauveria bassiana, insbesondere Stamm ATCC 74040, Coniothyrium minitans, insbesondere Stamm CON/M/91-8 (Accession No. DSM-9660), Lecanicillium spp., insbesondere Stamm HRO LEC 12, Lecanicillium lecanii, (ehemals bekannt als Verticillium lecanii), insbesondere Stamm KV01, Metarhizium anisopliae, insbesondere Stamm F52 (DSM3884/ ATCC 90448), Metschnikowia fructicola, insbesondere Stamm NRRL Y-30752, Paecilomyces fumosoroseus (neu: Isaria fumosorosea), insbesondere Stamm IFPC 200613, oder Stamm Apopka 97 (Accesion No. ATCC 20874), Paecilomyces lilacinus, insbesondere P. lilacinus Stamm 251 (AGAL 89/030550), Talaromyces flavus, insbesondere Stamm V117b, Trichoderma atroviride, insbesondere Stamm SC1 (Accession Number CBS 122089), Trichoderma harzianum, insbesondere T. harzianum rifai T39. (Accession Number CNCM I-952).

Beispiele für Viren, die als biologische Schädlingsbekämpfungsmittel eingesetzt werden bzw. verwendet werden können, sind:
Adoxophyes orana (Apfelschalenwickler) Granulosevirus (GV), Cydia pomonella (Apfelwickler) Granulosevirus (GV), Helicoverpa armigera (Baumwollkapselwurm) Nuklear Polyhedrosis Virus (NPV), Spodoptera exigua (Zuckerrübeneule) mNPV, Spodoptera frugiperda (Heerwurm) mNPV, Spodoptera littoralis (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
Agrobacterium spp., Azorhizobium caulinodans, Azospirillum spp., Azotobacter spp., Bradyrhizobium spp., Burkholderia spp., insbesondere Burkholderia cepacia (ehemals bekannt als Pseudomonas cepacia), Gigaspora spp., oder Gigaspora monosporum, Glomus spp., Laccaria spp., Lactobacillus buchneri, Paraglomus spp., Pisolithus tinctorus, Pseudomonas spp., Rhizobium spp., insbesondere Rhizobium trifolii, Rhizopogon spp., Scleroderma spp., Suillus spp., Streptomyces spp..

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,"Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lager-raum nach den üblichen Behandlungsmethoden, z.B. durch Tauchen, Sprühen, Ver¬dampfen, Ver¬nebeln, Streuen, Auf¬streichen, In¬jizieren und bei Ver¬mehrungs¬material, ins¬besondere bei Saatgut, weiterhin durch ein- oder mehr¬schichtiges Um¬hüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gen¬techno¬logisch erhaltenen) Pflanzen bzw. Pflanzensorten gehören alle Pflanzen, die durch die gen¬techno¬logische Modifikation genetisches Material erhielten, welches diesen Pflanzen besondere vorteilhafte wertvolle Eigenschaften ("Traits") verleiht. Beispiele für solche Eigen¬schaften sind besseres Pflanzenwachstum, erhöhte Toleranz gegen¬über hohen oder niedrigen Temperaturen, erhöhte Toleranz gegen Trockenheit oder gegen Wasser- bzw. Bodensalzgehalt, erhöhte Blühleistung, erleichterte Ernte, Be¬schleunigung der Reife, höhere Ernteerträge, höhere Qualität und/oder höherer Er¬nährungs¬wert der Ernteprodukte, höhere Lagerfähigkeit und/oder Bearbeitbarkeit der Ernte¬produkte. Weitere und besonders hervorgehobene Beispiele für solche Eigen¬schaften sind eine erhöhte Abwehrfähigkeit der Pflanzen gegen tierische und mikrobielle Schäd¬linge, wie Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird. Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform dieser Beschreibung werden die Verbindungen der Formel (I) an Säugetiere verabreicht.

Gemäß einer weiteren bevorzugten Ausführungsform dieser Beschreibung werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist. Die therapeutische Verwendung der Verbindungen der Formel (I) ist aber nicht Gegenstand der Erfindung. In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..
Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Beschreibung auf die Verwendung einer Verbindung der Formel (I) als Arzneimittel.

Ein weiterer Aspekt der Beschreibung bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt der Beschreibung wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung, ohne sie zu beschränken. Die Produkte wurden mittels ¹H-NMR Spektroskopie und/oder LC/MS (Liquid Chromatography Mass Spectrometry) charakterisiert.

Die Bestimmung der logP Werte erfolgte gemäß OECD Guideline 117 (EC Directive 92/69/EEC) durch HPLC (High Performance Liquid Chromatography) an reversed-phase (RP) Säulen (C18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker Avance 400, ausgestattet mit einem Durchflussprobenkopf (60 µl Volumen), bestimmt. In Einzelfällen wurden die NMR Spektren mit einem Bruker Avance II 600 gemessen.

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

### Synthese von Benzothiazolen der Formel (I) nach Methode A

### 6-Brom-2-(pyridin-3-yl)-1,3-benzothiazol (I-a-1)

### Schritt 1: N-(3,5-Dibrompyridin-2-yl)nicotinamid (A-III-1)

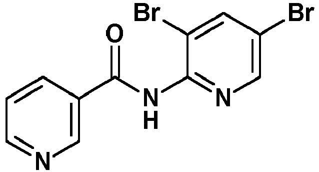

25,0 g (99,2 mmol) 3,5-Dibrompyridin-2-amin wurden in 200 ml Pyridin gelöst, im Eisbad abgekühlt und portionsweise mit 19,0 g (106,7 mmol) 3-(Chlorcarbonyl)pyridiniumchlorid versetzt. Der Ansatz wurde auf Raumtemperatur erwärmt, am Rotationsverdampfer im lauwarmen Wasserbad für 20 Minuten gedreht und anschließend im Vakuum eingedampft. Der erhaltene Rückstand wurde mit Wasser und wässriger Ammoniaklösung versetzt und der ausfallende Feststoff abgesaugt. Dieser wurde aus Fluorbenzol/Methyl-THF/Ethanol umkristallisiert, wobei kurz mit Aktivkohle aufgekocht und heiß filtriert wurde. Der entstehende Feststoff wurde mit wässriger Ammoniaklösung, Wasser und Petrolether gewaschen und getrocknet. Man erhielt so 11,4 g (100% Reinheit, 29,9% d. Th.) der Titelverbindung (A-III-1).
**1H-NMR (400,0 MHz, d₆-DMSO):** δ = 11,050 (9,9); 9,136 (8,3); 9,134 (9,2); 9,130 (8,9); 9,128 (8,6); 8,809 (6,9); 8,805 (7,4); 8,797 (7,3); 8,793 (7,2); 8,674 (13,9); 8,669 (15,7); 8,593 (16,0); 8,588 (14,1); 8,333 (3,9); 8,327 (5,3); 8,323 (3,9); 8,313 (4,3); 8,307 (5,7); 8,303 (4,0); 7,610 (4,7); 7,609 (4,9); 7,598 (4,6); 7,597 (4,8); 7,591 (4,6); 7,589 (4,7); 7,578 (4,4); 7,577 (4,5); 3,327 (53,4); 2,676 (0,3); 2,672 (0,5); 2,667 (0,3); 2,525 (1,4); 2,512 (26,9); 2,507 (54,1); 2,503 (71,3); 2,498 (51,3); 2,494 (24,4); 2,334 (0,3); 2,330 (0,5); 2,325 (0,3); 0,146 (0,5); 0,008 (4,4); 0,000 (110,7); -0,009 (3,9); -0,150 (0,5)

### Schritt 2&3: 6-Brom-2-(pyridin-3-yl)[1,3]thiazolo[4,5-b]pyridin (I-a-1)

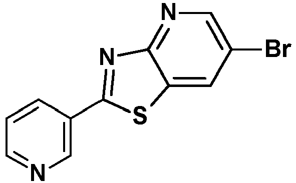

14,7 g (41,2 mmol) N-(3,5-Dibrompyridin-2-yl)nicotinamid (A-III-1) und 10,0 g (61,8 mmol) 2,4-Bis(4-methoxyphenyl)-1,3,2,4-dithiadiphosphetan-2,4-disulfid (Lawesson's Reagenz) wurden in möglichst wenig Anisol gelöst und für 2 Stunden bei 120 °C gerührt. Anschließend wurde der Ansatz eingeengt und gekühlt. Der Rückstand wurde in Ethylacetat und wässriger Kaliumcarbonatlösung aufgenommen. Die wässrige Phase wurde noch mehrfach mit Ethylacetat extrahiert. Die vereinigten organischen Phasen wurden mit Magnesiumsulfat getrocknet, filtriert und eingedampft.

Der Rückstand wurde direkt in 400 ml DMF aufgenommen, mit 24,0 g (173,7 mmol) Kaliumcarbonat versetzt und für 2 Stunden bei 120 °C gerührt. Anschließend wurde der Ansatz eingedampft und mit Wasser und Petrolether versetzt. Der ausfallende Feststoff wurde abfiltriert, mit wässriger Ammoniaklösung gewaschen und getrockent. Man erhielt so 9,6 g (100% Reinheit, 79,8% d. Th.) der Titelverbindung (I-a-1).
**1H-NMR(400,0 MHz, d₆-DMSO):** δ = 9,339 (8,2); 9,337 (8,7); 9,333 (8,6); 9,332 (8,2); 9,046 (15,3); 9,040 (16,0); 8,842 (15,8); 8,836 (15,1); 8,830 (7,6); 8,827 (7,8); 8,818 (7,7); 8,814 (7,5); 8,548 (4,4); 8,544 (5,4); 8,543 (5,3); 8,538 (4,3); 8,528 (4,7); 8,524 (5,4); 8,523 (5,8); 8,518 (4,4); 8,318 (0,4); 7,679 (5,3); 7,677 (5,4); 7,667 (5,2); 7,665 (5,3); 7,659 (5,2); 7,657 (5,1); 7,647 (5,0); 7,645 (5,0); 3,757 (1,1); 3,329 (112,8); 2,678 (0,5); 2,674 (0,7); 2,669 (0,5); 2,527 (1,9); 2,514 (39,9); 2,509 (80,5); 2,505 (105,7); 2,500 (75,9); 2,496 (35,9); 2,336 (0,5); 2,331 (0,7); 2,327 (0,5); 0,146 (0,7); 0,008 (5,9); 0,000 (157,7); -0,009 (5,5); -0,150 (0,7)

### Synthese von Verbindungen der Formel (I) nach Methode B und I

### 6-(2-Methylphenyl)-2-(pyridin-3-yl)[1,3]thiazolo[4,5-b]pyridin (2)

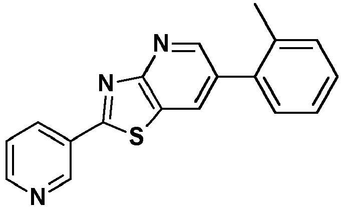

Unter Argon wurden 150,0 mg (0,51 mmol) 6-Brom-2-(pyridin-3-yl)[1,3]thiazolo[4,5-b]pyridin (I-a-1), 76,8 mg (0,56 mmol) (2-Methylphenyl)borsäure, 17,8 mg (0,01 mmol) Tetrakis(triphenylphosphin)palladium(0) und 108,8 mg (1,03 mmol) Natriumcarbonat zusammen in ein Mikrowellen-Reaktionsgefäß eingewogen und mit 3,8 ml einer 4:1-Mischung von entgastem Dioxan und Wasser versetzt. Das Gefäß wurde erneut mit Argon geflutet, verschlossen und für 30 Minuten bei 130 °C in einer Biotage Initiator Mikrowelle erhitzt. Der abgekühlte Ansatz wurde mit Wasser verdünnt und mehrfach mit Dichlormethan extrahiert. Die vereinigten organischen Phasen wurden über eine Kieselgel-Kartusche filtriert, mit Dichlormethan nachgewaschen und das Filtrat im Vakuum eingeengt. Der Rückstand wurde mit Acetonitril verrührt, filtriert und getrocknet. Man erhielt 53,0 mg (97,5% Reinheit, 33,2% d. Th.) der Titelverbindung (2).
**1H-NMR(400,0 MHz, d₆-DMSO):** δ = 9,366 (2,3); 9,361 (2,3); 8,833 (1,7); 8,829 (1,9); 8,821 (1,8); 8,817 (1,9); 8,753 (2,7); 8,748 (4,7); 8,735 (4,4); 8,730 (2,6); 8,573 (1,0); 8,569 (1,5); 8,564 (1,0); 8,553 (1,1); 8,549 (1,5); 8,544 (1,0); 7,693 (1,4); 7,681 (1,4); 7,673 (1,3); 7,661 (1,3); 7,404 (0,5); 7,393 (2,4); 7,390 (2,7); 7,383 (3,6); 7,376 (3,2); 7,369 (3,3); 7,362 (4,8); 7,355 (1,5); 7,349 (1,1); 7,340 (0,4); 3,329 (15,8); 2,526 (0,8); 2,508 (33,3); 2,504 (44,1); 2,500 (33,0); 2,383 (0,4); 2,331 (0,4); 2,315 (16,0); 0,008 (1,8); 0,000 (50,9); -0,009 (2,0)

### 6-(3-Chlorphenyl)-2-(pyridin-3-yl)[1,3]thiazolo[4,5-b]pyridin (7)

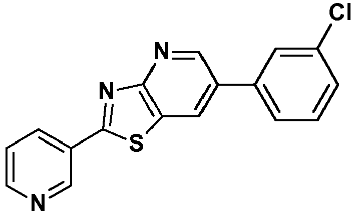

Unter Argon wurden 150 mg (0,51 mmol) 6-Brom-2-(pyridin-3-yl)[1,3]thiazolo[4,5-b]pyridin (I-a-1), 88,3 mg (0,57 mmol) (3-Chlorphenyl)borsäure, 17,8 mg (0,01 mmol) Tetrakis(triphenylphosphin)palladium(0) und 108,8 mg (1,03 mmol) Natriumcarbonat zusammen in ein Mikrowellen-Reaktionsgefäß eingewogen und mit 3,8 ml einer 4:1-Mischung von entgastem Dioxan und Wasser versetzt. Das Gefäß wurde erneut mit Argon geflutet, verschlossen und für 30 Minuten bei 130 °C in einer Biotage Initiator Mikrowelle erhitzt. Der abgekühlte Ansatz wurde mit Wasser verdünnt und mehrfach mit Dichlormethan extrahiert. Die vereinigten organischen Phasen wurden über eine Kieselgel-Kartusche filtriert, mit Dichlormethan nachgewaschen und das Filtrat im Vakuum eingeengt. Der Rückstand wurde mit Acetonitril verrührt, filtriert und getrocknet. Die Reinigung erfolgte chromatographisch per MPLC (Fließmittel Wasser/Acetonitril). Man erhielt 14,0 mg (100,0% Reinheit, 8,4% d. Th.) der Titelverbindung (7).
**1H-NMR(400,0 MHz, d₆-DMSO):** δ = 9,374 (8,4); 9,368 (8,3); 9,117 (11,3); 9,111 (15,3); 9,084 (16,0); 9,078 (11,2); 8,834 (6,6); 8,830 (6,7); 8,822 (6,8); 8,818 (6,5); 8,582 (3,8); 8,577 (5,3); 8,572 (3,6); 8,562 (4,1); 8,557 (5,5); 8,552 (3,5); 8,317 (1,9); 7,969 (6,9); 7,964 (12,0); 7,960 (6,7); 7,857 (5,8); 7,838 (6,5); 7,811 (0,4); 7,693 (5,3); 7,681 (5,2); 7,673 (5,1); 7,661 (4,9); 7,611 (4,5); 7,591 (10,7); 7,572 (7,8); 7,547 (7,4); 7,530 (2,8); 7,527 (3,2); 7,289 (0,4); 6,937 (0,5); 6,925 (0,4); 6,914 (0,4); 5,757 (0,7); 3,744 (3,2); 3,327 (467,3); 2,676 (4,1); 2,671 (5,6); 2,667 (4,1); 2,602 (0,5); 2,524 (15,1); 2,507 (606,6); 2,502 (788,2); 2,498 (575,0); 2,333 (3,8); 2,329 (5,2); 2,325 (3,7); 2,170 (0,5); 1,355 (0,5); 1,277 (0,4); 1,259 (0,5); 1,232 (5,8); 1,154 (0,4); 1,136 (0,6); 1,118 (0,4); 0,853 (0,7); 0,836 (0,4); 0,146 (2,7); 0,082 (0,4); 0,073 (6,2); 0,008 (20,0); 0,000 (579,1); -0,009 (21,1); -0,150 (2,8)

### 6-[2-(Ethylsulfanyl)phenyl]-2-(pyridin-3-yl)[1,3]thiazolo[4,5-b]pyridin (14)

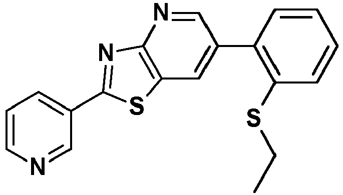

Unter Argon wurden 200,0 mg (0,68 mmol) 6-Brom-2-(pyridin-3-yl)[1,3]thiazolo[4,5-b]pyridin (I-a-1), 137,1 mg (0,75 mmol) [4-(Ethylsulfanyl)phenyl]borsäure, 23,7 mg (0,02 mmol) Tetrakis(triphenylphosphin)-palladium(0) und 145,1 mg (1,4 mmol) Natriumcarbonat zusammen in ein Reaktionsgefäß eingewogen und mit 2 ml einer 1:1-Mischung von entgastem Dioxan und Wasser versetzt.. Das Gefäß wurde erneut mit Argon geflutet, verschlossen und für 30 Minuten bei 130 °C in einer Biotage Initiator Mikrowelle erhitzt. Der abgekühlte Ansatz wurde mit Wasser verdünnt und mehrfach mit Dichlormethan extrahiert. Die vereinigten organischen Phasen wurden über eine Kieselgel-Kartusche filtriert, mit Dichlormethan nachgewaschen und das Filtrat im Vakuum eingeengt. Die Reinigung erfolgte chromatographisch per MPLC (Fließmittel Cyclohexan/Ethylacetat). Man erhielt 240,0 mg (81,3% Reinheit, 81,6% d. Th.) der Titelverbindung (14).
**1H-NMR(400,0 MHz, d₆-DMSO):** δ = 9,364 (3,7); 9,359 (3,7); 8,834 (2,8); 8,831 (3,0); 8,823 (3,0); 8,819 (2,9); 8,742 (4,8); 8,736 (6,6); 8,713 (6,5); 8,707 (4,7); 8,572 (1,6); 8,568 (2,3); 8,563 (1,6); 8,553 (1,8); 8,548 (2,4); 8,543 (1,6); 8,317 (0,4); 8,070 (6,5); 7,695 (2,2); 7,683 (2,2); 7,675 (2,1); 7,663 (2,0); 7,540 (2,1); 7,522 (3,9); 7,520 (3,9); 7,493 (1,8); 7,488 (1,9); 7,475 (2,3); 7,471 (2,7); 7,455 (1,1); 7,451 (1,4); 7,414 (1,8); 7,410 (2,1); 7,395 (4,0); 7,391 (3,4); 7,365 (3,2); 7,362 (3,2); 7,345 (4,2); 7,328 (2,3); 7,325 (2,4); 7,315 (0,8); 7,311 (0,7); 7,297 (0,9); 7,294 (0,9); 7,278 (0,4); 7,274 (0,4); 7,177 (0,6); 7,174 (0,7); 7,159 (1,0); 7,156 (1,0); 7,141 (0,4); 7,138 (0,4); 4,038 (0,5); 4,020 (0,6); 3,568 (0,4); 3,328 (165,3); 2,936 (2,2); 2,918 (7,2); 2,904 (3,7); 2,900 (7,5); 2,886 (3,1); 2,882 (2,7); 2,868 (0,9); 2,676 (1,1); 2,671 (1,5); 2,667 (1,1); 2,524 (4,1); 2,507 (166,9); 2,502 (217,8); 2,498 (164,7); 2,334 (1,1); 2,329 (1,5); 2,325 (1,1); 1,989 (2,2); 1,242 (0,4); 1,234 (0,3); 1,212 (2,8); 1,194 (6,2); 1,186 (7,9); 1,175 (4,5); 1,167 (16,0); 1,157 (1,3); 1,149 (7,4); 0,000 (0,5)

### 6-[2-(Ethylsulfinyl)phenyl]-2-(pyridin-3-yl)[1,3]thiazolo[4,5-b]pyridin (15)

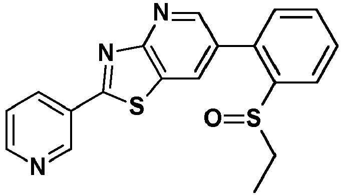

100,0 mg (0,28 mmol) 6-[2-(Ethylsulfanyl)phenyl]-2-(pyridin-3-yl)[1,3]thiazolo[4,5-b]pyridin (14) wurden in 10 ml Dichlormethan vorgelegt und mit 118,8 mg (0,68 mmol) m-Chlorperbenzoesäure versetzt. Der Ansatz wurde über Nacht bei Raumtemperatur gerührt. Anschließend wurde der Ansatz mit einem Tropfen wässriger Natriumhydrogensulfit versetzt, mit wässriger Natriumcarbonatlösung gewaschen, getrocknet und eingedampft. Die Reinigung erfolgte chromatographisch per MPLC (Fließmittel Wasser/Acetonitril). Man erhielt 26,0 mg (100,0% Reinheit, 24,9% d. Th.) der Titelverbindung (15).
**1H-NMR(400,0 MHz, d₆-DMSO):** δ = 9,374 (4,2); 9,370 (4,3); 8,866 (6,3); 8,860 (7,5); 8,843 (3,2); 8,839 (3,5); 8,831 (3,4); 8,827 (3,5); 8,805 (7,3); 8,800 (6,2); 8,585 (1,8); 8,580 (2,5); 8,575 (1,9); 8,565 (2,0); 8,559 (2,7); 8,555 (1,9); 8,318 (0,3); 8,006 (2,9); 8,003 (3,1); 7,987 (3,4); 7,984 (3,5); 7,799 (1,4); 7,795 (1,5); 7,780 (3,2); 7,777 (3,2); 7,761 (2,2); 7,757 (2,2); 7,740 (2,0); 7,736 (2,1); 7,721 (3,3); 7,718 (3,4); 7,701 (3,5); 7,699 (3,7); 7,688 (2,4); 7,681 (2,3); 7,669 (2,2); 7,667 (2,2); 7,571 (3,6); 7,569 (3,6); 7,553 (3,0); 7,550 (2,9); 5,758 (1,5); 3,332 (197,0); 2,783 (0,5); 2,764 (1,8); 2,746 (2,1); 2,731 (2,4); 2,712 (2,2); 2,694 (0,7); 2,677 (0,8); 2,672 (1,0); 2,668 (0,8); 2,542 (0,6); 2,525 (2,5); 2,508 (116,7); 2,503 (153,0); 2,499 (113,2); 2,444 (0,7); 2,426 (2,1); 2,408 (2,4); 2,392 (2,1); 2,374 (1,8); 2,355 (0,6); 2,334 (0,7); 2,330 (1,0); 2,325 (0,8); 0,905 (7,4); 0,887 (16,0); 0,869 (7,1); 0,000 (6,3)

### 6-[2-(Ethylsulfinyl)phenyl]-2-(1-oxidopyridin-3-yl)[1,3]thiazolo[4,5-b]pyridin (16)

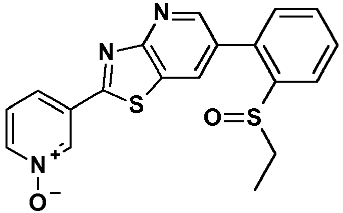

Bei der Herstellung der Verbindung (15) wurde die Verbindung (16) als Nebenprodukt isoliert. Man erhielt 16,7 mg (92,8% Reinheit, 14,2% d. Th.) der Titelverbindung (16).
**1H-NMR(400,0 MHz, d₆-DMSO):** δ = 9,371 (4,2); 9,365 (4,1); 8,840 (3,2); 8,836 (3,3); 8,828 (3,3); 8,824 (3,3); 8,764 (5,8); 8,759 (7,2); 8,719 (7,0); 8,713 (5,7); 8,580 (1,8); 8,575 (2,5); 8,571 (1,9); 8,561 (1,9); 8,555 (2,6); 8,551 (1,8); 8,318 (1,8); 8,132 (3,1); 8,115 (3,4); 8,112 (3,4); 7,889 (1,6); 7,873 (3,3); 7,870 (3,3); 7,854 (2,3); 7,851 (2,2); 7,815 (2,1); 7,812 (2,5); 7,795 (3,0); 7,792 (3,1); 7,776 (1,3); 7,773 (1,3); 7,699 (2,5); 7,686 (2,5); 7,679 (2,5); 7,666 (2,4); 7,627 (0,5); 7,601 (3,6); 7,598 (3,7); 7,583 (3,2); 7,580 (3,1); 5,757 (1,7); 4,040 (0,5); 4,022 (0,5); 3,376 (0,5); 3,327 (653,5); 3,012 (2,1); 2,993 (7,0); 2,975 (7,2); 2,957 (2,2); 2,675 (4,7); 2,671 (6,3); 2,667 (4,8); 2,623 (0,3); 2,524 (17,2); 2,510 (380,2); 2,506 (748,3); 2,502 (973,4); 2,497 (719,5); 2,372 (0,3); 2,333 (4,7); 2,328 (6,4); 2,324 (4,8); 2,299 (0,4); 2,206 (0,3); 1,355 (0,3); 1,234 (1,4); 1,165 (1,7); 1,026 (7,4); 1,008 (16,0); 0,989 (7,2); 0,146 (17,1); 0,096 (0,3); 0,086 (0,7); 0,070 (0,7); 0,058 (1,2); 0,047 (1,5); 0,007 (153,7); 0,000 (3211,2); - 0,009 (157,6); -0,068 (0,9); -0,075 (0,9); -0,095 (0,6); -0,121 (0,4); -0,150 (16,9)

### 6-{2,4-Dimethyl-5-[(2,2,2-trifluorethyl)sulfanyl]phenyl}-2-(pyridin-3-yl)[1,3]thiazolo[4,5-b]pyridin (19)

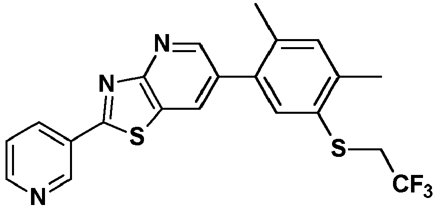

200,0 mg (0,68 mmol) 6-Brom-2-(pyridin-3-yl)[1,3]thiazolo[4,5-b]pyridin (I-a-1), 198,9 mg (0,75 mmol) {2,4-Dimethyl-5-[(2,2,2-trifluorethyl)sulfanyl]phenyl}borsäure und 23,7 mg (0,02 mmol) Tetrakis(triphenylphosphin)-palladium(0) wurden unter Argon vorgelegt und anschließend mit 9,4 ml entgastem Acetonitril und 1,4 ml zweimolarer Natriumcarbonatlösung versetzt. Der Ansatz wurde unter Argon über Nacht bei 80 °C gerührt. Anschließend wurde der abgekühlte Ansatz eingeengt, mit Dichlormethan versetzt und mit wenig Wasser gewaschen. Die organische Phase wurde getrocknet, filtriert und eingedampft. Die Reinigung erfolgte chromatographisch per MPLC (Fließmittel Wasser/Acetonitril). Man erhielt 92,0 mg (97,8% Reinheit, 30,5% d. Th.) der Titelverbindung (19).
**1H-NMR(400,0 MHz, d₆-DMSO):** δ = 9,370 (2,7); 9,366 (2,7); 9,365 (2,7); 8,834 (2,3); 8,830 (2,5); 8,822 (2,4); 8,818 (2,5); 8,759 (2,5); 8,753 (8,0); 8,749 (7,8); 8,744 (2,3); 8,578 (1,3); 8,574 (1,7); 8,568 (1,3); 8,558 (1,4); 8,553 (1,8); 8,548 (1,3); 8,318 (0,3); 7,694 (1,6); 7,693 (1,6); 7,682 (1,6); 7,681 (1,6); 7,674 (1,6); 7,673 (1,6); 7,662 (1,5); 7,661 (1,5); 7,536 (5,9); 7,297 (4,6); 4,035 (1,2); 4,009 (3,8); 3,982 (4,0); 3,956 (1,4); 3,328 (100,9); 2,676 (0,7); 2,672 (0,9); 2,667 (0,7); 2,525 (2,7); 2,520 (3,9); 2,512 (49,4); 2,507 (100,6); 2,503 (133,6); 2,498 (97,4); 2,494 (47,0); 2,418 (14,4); 2,334 (0,6); 2,329 (0,9); 2,325 (0,6); 2,275 (16,0); 1,233 (0,4); 0,146 (0,8); 0,019 (0,4); 0,008 (6,1); 0,000 (176,8); -0,009 (6,0); - 0,150 (0,8)

### 6-{2,4-Dimethyl-5-[(2,2,2-trifluorethyl)sulfinyl]phenyl}-2-(pyridin-3-yl)[1,3]thiazolo[4,5-b]pyridin (20)

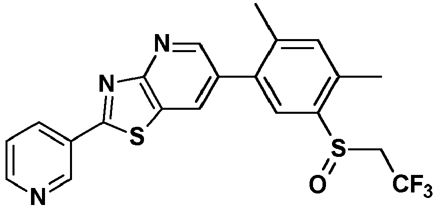

60,0 mg (0,14 mmol) 6-{2,4-Dimethyl-5-[(2,2,2-trifluorethyl)sulfanyl]phenyl}-2-(pyridin-3-yl)[1,3]thiazolo[4,5-b]pyridin (19) wurden in 10 ml Dichlormethan vorgelegt und mit 34,3 mg (0,15 mmol) m-Chlorperbenzoesäure versetzt. Der Ansatz wurde über Nacht bei Raumtemperatur gerührt. Anschließend wurde der Ansatz mit einem Tropfen wässriger Natriumhydrogensulfit versetzt, mit wässriger Natriumcarbonatlösung gewaschen, getrocknet und eingedampft. Die Reinigung erfolgte chromatographisch per MPLC (Fließmittel Wasser/Acetonitril). Man erhielt 28 mg (90,0% Reinheit, 40,5% d. Th.) der Titelverbindung (20).
**1H-NMR(400,0 MHz, d₆-DMSO):** δ = 9,379 (3,2); 9,374 (3,0); 8,830 (7,8); 8,825 (9,1); 8,586 (1,4); 8,581 (1,9); 8,577 (1,3); 8,566 (1,5); 8,561 (2,0); 8,557 (1,3); 8,317 (0,5); 8,145 (0,6); 7,820 (6,5); 7,697 (1,8); 7,685 (1,8); 7,676 (1,8); 7,664 (1,7); 7,400 (4,8); 5,758 (2,3); 4,197 (1,1); 4,170 (3,3); 4,142 (3,5); 4,115 (1,2); 3,366 (0,8); 3,329 (136,7); 2,891 (0,7); 2,731 (0,6); 2,695 (0,4); 2,676 (1,3); 2,671 (1,7); 2,667 (1,3); 2,539 (1,9); 2,507 (193,1); 2,502 (244,6); 2,498 (184,8); 2,431 (15,1); 2,398 (0,8); 2,378 (16,0); 2,334 (1,3); 2,329 (1,7); 2,325 (1,3); 1,234 (1,0); 1,165 (1,0); 1,150 (0,6); 0,000 (6,2)

### 6-{2,4-Dimethyl-5-[(2,2,2-trifluorethyl)sulfinyl]phenyl}-2-(pyridin-3-yl)[1,3]thiazolo[4,5-b]pyridin (21)

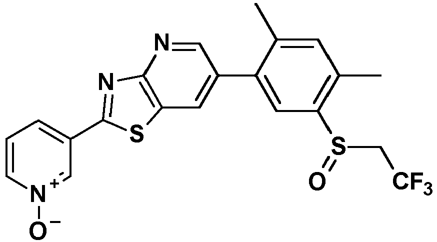

Bei der Herstellung der Verbindung (20) wurde die Verbindung (21) als Nebenprodukt isoliert. Man erhielt 21 mg (90,0% Reinheit, 29,3% d. Th.) der Titelverbindung (21).
**1H-NMR(400,0 MHz, d₆-DMSO):** δ = 8,927 (4,0); 8,861 (2,0); 8,854 (9,9); 8,848 (2,3); 8,841 (0,8); 8,830 (0,4); 8,814 (0,3); 8,690 (0,3); 8,466 (2,0); 8,448 (2,1); 8,317 (1,3); 8,148 (0,5); 8,104 (2,1); 8,084 (2,3); 7,846 (0,5); 7,818 (7,0); 7,680 (2,2); 7,663 (2,3); 7,660 (2,3); 7,643 (1,9); 7,555 (0,4); 7,401 (4,8); 7,383 (0,3); 5,757 (2,2); 4,194 (1,1); 4,167 (3,4); 4,140 (3,6); 4,113 (1,2); 4,037 (0,4); 4,020 (0,4); 3,428 (0,3); 3,416 (0,4); 3,402 (0,5); 3,368 (1,5); 3,330 (615,1); 3,294 (2,0); 2,891 (1,5); 2,731 (1,3); 2,695 (1,2); 2,676 (2,8); 2,671 (3,9); 2,667 (3,0); 2,545 (2,0); 2,540 (2,2); 2,524 (10,1); 2,507 (425,8); 2,502 (566,6); 2,498 (425,9); 2,430 (14,9); 2,392 (2,1); 2,372 (16,0); 2,333 (2,6); 2,329 (3,6); 2,324 (2,8); 1,234 (2,2); 1,165 (1,4); 1,149 (0,8); 0,853 (0,4); 0,008 (0,5); 0,000 (15,0); -0,008 (0,7)

### 6-{2-Fluor-4-methyl-5-[(2,2,2-trifluorethyl)sulfanyl]phenyl}-2-(pyridin-3-yl)[1,3]thiazolo[4,5-b]pyridin (22)

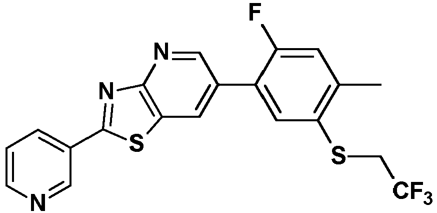

150,0 mg (0,51 mmol) 6-Brom-2-(pyridin-3-yl)[1,3]thiazolo[4,5-b]pyridin (I-a-1), 151,4 mg (0,56 mmol) {2-Fluor-4-methyl-5-[(2,2,2-trifluorethyl)sulfanyl]phenyl}borsäure, 17,8 mg (0,01 mmol) Tetrakis(triphenylphosphin)palladium(0) und 108,8 mg (1,0 mmol) Natriumcarbonat wurden unter Argon in ein Reaktionsgefäß eingewogen und mit 4,8 ml einer 5:1-Mischung von entgastem Dioxan und Wasser versetzt. Das Gefäß wurde erneut mit Argon geflutet, verschlossen und für 30 Minuten bei 130 °C in einer Biotage Initiator Mikrowelle erhitzt. Der abgekühlte Ansatz wurde mit Wasser verdünnt und mehrfach mit Dichlormethan extrahiert. Die vereinigten organischen Phasen wurden über eine Kieselgel-Kartusche filtriert, mit Dichlormethan gewaschen, anschließend wurde das Filtrat im Vakuum eingeengt. Der Rückstand wurde mit Acetonitril verrührt, filtriert und getrocknet. Man erhielt 75,0 mg (96,2% Reinheit, 32,3% d. Th.) der Titelverbindung (22).
**1H-NMR(400,0 MHz, d₆-DMSO):** δ = 9,375 (3,0); 9,370 (3,0); 8,953 (9,2); 8,950 (9,1); 8,836 (2,4); 8,833 (2,6); 8,824 (2,6); 8,821 (2,7); 8,583 (1,3); 8,578 (1,8); 8,573 (1,4); 8,563 (1,5); 8,558 (2,0); 8,553 (1,4); 7,902 (3,1); 7,882 (3,1); 7,693 (1,8); 7,681 (1,8); 7,673 (1,8); 7,663 (1,7); 7,661 (1,8); 7,422 (2,7); 7,393 (2,7); 5,758 (0,6); 4,114 (1,3); 4,088 (4,0); 4,062 (4,2); 4,036 (1,5); 3,328 (42,3); 2,677 (0,5); 2,673 (0,6); 2,668 (0,5); 2,526 (1,6); 2,521 (2,5); 2,512 (35,8); 2,508 (72,9); 2,503 (97,3); 2,499 (72,8); 2,495 (36,8); 2,468 (16,0); 2,403 (0,5); 2,335 (0,5); 2,330 (0,7); 2,326 (0,5); 2,076 (1,5); 0,146 (0,5); 0,008 (3,9); 0,000 (116,1); -0,009 (4,6); -0,150 (0,5)

### 6-{2-Fluor-4-methyl-5-[(2,2,2-trifluorethyl)sulfinyl]phenyl}-2-(pyridin-3-yl)[1,3]thiazolo[4,5-b]pyridin (23)

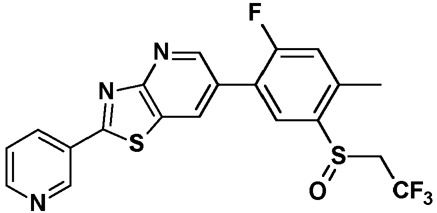

155,0 mg (0,35 mmol) 6-{2-Fluor-4-methyl-5-[(2,2,2-trifluorethyl)sulfanyl]phenyl}-2-(pyridin-3-yl)[1,3]thiazolo[4,5-b]pyridin (22) wurden in 25 ml Dichlormethan vorgelegt und mit 87,7 mg (0,39 mmol) m-Chlorperbenzoesäure versetzt. Der Ansatz wurde über Nacht bei Raumtemperatur gerührt.

Anschließend wurde der Ansatz mit einem Tropfen wässriger Natriumhydrogensulfit versetzt, mit wässriger Natriumcarbonatlösung gewaschen, getrocknet und eingedampft. Die Reinigung erfolgte chromatographisch per MPLC. Man erhielt 43,2 mg (98,3% Reinheit, 26,4% d. Th.) der Titelverbindung (23).
**1H-NMR(400,0 MHz, d₆-DMSO):** δ = 9,383 (12,6); 9,378 (12,3); 9,036 (15,7); 9,033 (14,8); 9,022 (12,3); 9,018 (16,0); 9,013 (7,6); 8,841 (9,7); 8,837 (10,6); 8,829 (10,1); 8,825 (10,6); 8,590 (5,6); 8,586 (7,4); 8,581 (5,7); 8,570 (6,1); 8,565 (8,1); 8,560 (5,8); 8,317 (1,4); 8,159 (13,2); 8,139 (13,3); 7,697 (7,3); 7,685 (7,1); 7,679 (6,9); 7,677 (7,0); 7,667 (6,7); 7,665 (6,9); 7,644 (0,6); 7,627 (0,8); 7,624 (0,8); 7,615 (0,7); 7,597 (0,8); 7,575 (0,6); 7,567 (0,7); 7,538 (10,5); 7,509 (10,2); 5,758 (3,5); 4,329 (0,4); 4,302 (1,4); 4,293 (2,2); 4,276 (2,8); 4,266 (7,1); 4,253 (6,9); 4,239 (7,4); 4,225 (7,4); 4,212 (2,8); 4,198 (2,7); 4,189 (1,6); 4,161 (0,5); 4,056 (1,0); 4,038 (2,9); 4,020 (3,0); 4,002 (1,0); 3,827 (0,4); 3,812 (0,5); 3,640 (0,6); 3,407 (0,3); 3,392 (0,7); 3,334 (1093,5); 3,268 (0,5); 3,175 (0,4); 3,162 (0,4); 2,677 (2,9); 2,672 (4,1); 2,668 (2,9); 2,645 (0,3); 2,525 (11,2); 2,512 (231,5); 2,508 (469,6); 2,503 (621,0); 2,499 (458,4); 2,494 (229,3); 2,485 (73,3); 2,434 (0,9); 2,410 (0,6); 2,388 (0,5); 2,372 (0,6); 2,334 (3,1); 2,330 (4,2); 2,325 (3,3); 1,990 (12,4); 1,909 (1,8); 1,298 (1,4); 1,259 (2,1); 1,233 (5,3); 1,193 (3,5); 1,175 (6,8); 1,157 (3,4); 0,853 (0,8); 0,836 (0,4); 0,146 (0,7); 0,008 (6,1); 0,000 (167,9); -0,008 (6,2); -0,150 (0,8)

### Synthese von Verbindungen der Formel (I) nach Methode D

### N-(3-Chlorphenyl)-2-(pyridin-3-yl)[1,3]thiazolo[4,5-b]pyridin-6-amin (25)

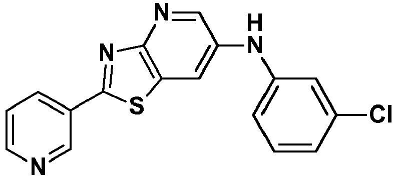

150 mg (0,51 mmol) 6-Brom-2-(pyridin-3-yl)[1,3]thiazolo[4,5-b]pyridin (I-a-1), 78,6 mg (0,61 mmol) 3-Chloranilin, 18,4 mg (0,08 mmol) Palladium(II)acetat, 57,5 mg (0,09) rac-BINAP und 301,1 mg (0,92 mmol) Cäsiumcarbonat wurden unter Argon vorgelegt und anschließend mit 5 ml entgastem Toluol versetzt. Das Gefäß wurde erneut mit Argon geflutet, verschlossen und für 60 Minuten bei 140 °C in einer Biotage Initiator Mikrowelle erhitzt. Der abgekühlte Ansatz wurde mit Wasser verdünnt und mehrfach mit Dichlormethan und Ethylacetat extrahiert. Die vereinigten organischen Phasen wurden filtriert und im Vakuum eingeengt. Die Reinigung erfolgte chromatographisch per MPLC (Fließmittel Wasser/Acetonitril). Man erhielt 44,0 mg (96,9% Reinheit, 24,5% d. Th.) der Titelverbindung (25).
**1H-NMR(400,0 MHz, d₆-DMSO):** δ = 9,269 (6,7); 9,264 (6,8); 8,962 (11,3); 8,772 (5,4); 8,768 (5,9); 8,760 (5,8); 8,756 (5,9); 8,483 (9,8); 8,476 (13,1); 8,465 (3,5); 8,461 (4,5); 8,456 (3,4); 8,445 (3,7); 8,440 (5,6); 8,436 (16,0); 8,429 (10,0); 8,317 (1,2); 7,647 (4,3); 7,646 (4,3); 7,635 (4,1); 7,627 (4,1); 7,626 (4,1); 7,615 (4,0); 7,614 (3,9); 7,356 (0,3); 7,339 (4,5); 7,319 (9,7); 7,299 (6,0); 7,189 (4,3); 7,184 (10,1); 7,179 (7,2); 7,170 (5,9); 7,150 (4,0); 7,146 (3,3); 6,967 (4,5); 6,964 (4,4); 6,947 (3,9); 6,944 (4,1); 5,757 (0,5); 3,326 (236,5); 2,891 (1,4); 2,732 (1,2); 2,680 (1,3); 2,676 (2,6); 2,671 (3,6); 2,667 (2,7); 2,662 (1,3); 2,525 (8,9); 2,520 (13,4); 2,511 (186,4); 2,507 (384,3); 2,502 (519,2); 2,498 (389,2); 2,493 (195,6); 2,338 (1,2); 2,333 (2,5); 2,329 (3,5); 2,325 (2,6); 2,086 (4,0); 2,075 (1,1); 0,146 (2,0); 0,008 (14,5); 0,000 (450,0); -0,009 (17,3); -0,150 (2,0)

### Synthese von Verbindungen der Formel (I) nach Methode E

### 6-(1H-Pyrazol-1-yl)-2-(pyridin-3-yl)[1,3]thiazolo[4,5-b]pyridin (26)

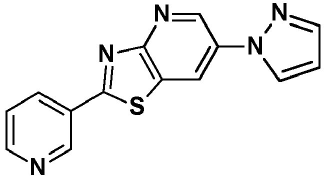

Unter Argon wurden 400,0 mg (1,4 mmol) 6-Brom-2-(pyridin-3-yl)[1,3]thiazolo[4,5-b]pyridin (I-a-1), 1,0 g (14,7 mmol) 1H-Pyrazol, 21 mg (0,1 mmol) Kupfer(I)iodid, 568,0 mg (4,1 mmol) Kaliumcarbonat und 40 mg (0,14 mmol) trans-1,2-Bis(2'-pyridylideneamino)cyclohexan (racemisch) [herstellbar gemäß Chem. Eur. J. 2005, 2483] in ein Reaktionsgefäß eingewogen und 19,0 g DMF zugegeben. Der Ansatz wurde unter Argon bei 120 °C über Nacht erhitzt. Der abgekühlte Ansatz wurde im Vakuum eingedampft, der Rückstand mit Wasser versetzt und mehrfach mit Ethylacetat extrahiert. Die vereinigten organischen Phasen wurden getrocknet, filtriert und eingedampft. Die Reinigung erfolgte chromatographisch per MPLC. Man erhielt 20,0 mg (100% Reinheit, 5,2% d. Th.) der Titelverbindung (26).
**1H-NMR(400,0 MHz, d₆-DMSO):** δ = 11,160 (0,7); 10,721 (0,7); 9,345 (14,2); 9,294 (15,7); 9,162 (14,9); 8,822 (10,6); 8,814 (10,8); 8,761 (0,5); 8,697 (15,8); 8,551 (8,4); 8,531 (8,8); 8,475 (0,5); 8,429 (0,4); 7,894 (16,0); 7,799 (0,4); 7,684 (7,1); 7,667 (8,7); 7,654 (6,8); 7,235 (0,5); 7,220 (0,4); 7,216 (0,4); 6,673 (14,6); 5,935 (0,7); 4,134 (0,5); 3,391 (0,6); 3,326 (63,2); 2,671 (2,1); 2,503 (265,8); 2,329 (2,3); 2,283 (0,7); 2,265 (0,6); 2,244 (0,5); 2,234 (0,5); 2,209 (0,4); 2,205 (0,4); 2,178 (0,4); 2,160 (0,4); 2,155 (0,4); 2,141 (0,4); 2,087 (4,1); 2,058 (0,6); 2,044 (0,4); 1,983 (0,7); 1,926 (0,4); 1,907 (0,5); 1,886 (0,4); 1,870 (0,4); 1,852 (0,4); 1,821 (0,4); 1,743 (1,2); 1,678 (0,5); 1,671 (0,5); 1,625 (1,4); 1,532 (0,7); 1,511 (0,7); 1,463 (0,6); 1,453 (0,6); 1,433 (0,6); 1,416 (0,6); 1,367 (0,9); 1,349 (1,1); 1,297 (3,4); 1,257 (4,8); 1,230 (8,0); 1,171 (2,8); 1,146 (4,1); 1,136 (4,0); 1,088 (1,2); 1,065 (1,0); 1,042 (0,9); 1,036 (0,9); 0,981 (0,6); 0,943 (0,6); 0,928 (0,7); 0,863 (3,1); 0,755 (0,5); 0,724 (0,4); 0,701 (0,4); 0,145 (0,9); 0,123 (0,4); 0,105 (0,5); 0,095 (0,6); -0,001 (131,7); -0,150 (1,2); -0,190 (0,4); -0,201 (0,4)

### 6-(1H-Pyrazol-1-yl)-2-(pyridin-3-yl)[1,3]thiazolo[4,5-b]pyridin (27)

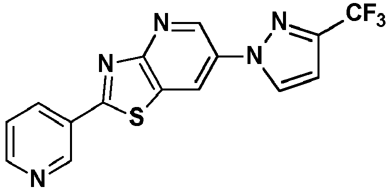

Unter Argon wurden 300,0 mg (1,0 mmol) 6-Brom-2-(pyridin-3-yl)[1,3]thiazolo[4,5-b]pyridin (I-a-1), 1,4 g (10,3 mmol) 3-(Trifluormethyl)-1H-pyrazol und 147 mg (1,0 mmol) Kupfer(I)oxid in ein Reaktionsgefäß eingewogen und 15,0 g Pyridin zugegeben. Der Ansatz wurde unter Argon bei 90 °C über Nacht erhitzt. Anschließend wurden weitere 300 mg (1,0 mmol) 6-Brom-2-(pyridin-3-yl)[1,3]thiazolo[4,5-b]pyridin (II-1) und 440 mg (3,0 mmol) Kupfer(I)oxid zugegeben und der Ansatz weitere 7 Stunden bei 115 °C erhitzt. Der abgekühlte Ansatz wurde anschließend im Vakuum eingedampft. Die Reinigung erfolgte chromatographisch per MPLC (zunächst Fließmittelgradient Wasser/Acetonitril; für angereicherte Fraktion dann Trennung per Fließmittelgradient Petrolether/Aceton). Man erhielt 20,0 mg (82% Reinheit, 4,6% d. Th.) der Titelverbindung (27).
**1H-NMR(601,6 MHz, CD3CN):** δ = 9,373 (4,7); 9,355 (0,8); 9,156 (8,3); 9,151 (8,5); 8,877 (8,9); 8,873 (8,7); 8,789 (3,3); 8,782 (3,4); 8,734 (0,6); 8,729 (0,6); 8,511 (2,6); 8,508 (3,8); 8,505 (2,6); 8,498 (3,3); 8,495 (3,9); 8,492 (3,0); 8,488 (1,3); 8,485 (1,1); 8,476 (0,6); 8,385 (5,8); 8,383 (5,8); 7,585 (3,2); 7,577 (3,3); 7,572 (3,3); 7,564 (3,3); 7,545 (0,5); 7,466 (0,6); 7,452 (1,5); 7,446 (0,9); 7,441 (0,7); 7,433 (0,7); 7,238 (0,5); 7,234 (0,4); 7,224 (0,4); 7,220 (0,4); 6,953 (6,6); 6,949 (6,5); 5,448 (0,7); 2,828 (1,2); 2,748 (0,6); 2,575 (2,2); 2,563 (2,3); 2,501 (0,5); 2,172 (9,1); 2,107 (5,6); 2,087 (3,1); 2,076 (2,7); 2,059 (3,2); 2,055 (4,1); 2,051 (5,1); 2,047 (3,9); 2,043 (2,7); 1,965 (67,2); 1,957 (37,4); 1,952 (51,3); 1,949 (253,4); 1,945 (433,6); 1,940 (624,6); 1,936 (441,3); 1,932 (228,3); 1,916 (4,5); 1,883 (1,7); 1,850 (1,1); 1,842 (1,1); 1,834 (2,0); 1,830 (3,1); 1,826 (4,2); 1,822 (3,0); 1,818 (1,8); 1,567 (0,5); 1,434 (0,5); 1,340 (13,1); 1,319 (2,0); 1,308 (2,5); 1,298 (2,3); 1,285 (16,0); 1,269 (9,4); 1,248 (1,2); 1,227 (1,0); 1,217 (1,0); 1,206 (1,0); 1,184 (1,3); 1,173 (1,2); 1,153 (0,8); 1,134 (0,8); 1,112 (0,7); 1,083 (0,8); 1,071 (0,7); 1,048 (0,6); 1,038 (0,6); 1,022 (4,4); 1,010 (8,3); 0,999 (4,3); 0,914 (0,7); 0,893 (1,2); 0,882 (2,1); 0,870 (1,5); 0,844 (1,3); 0,000 (2,8)

### 4,5-Dimethyl-2-[2-(pyridin-3-yl)[1,3]thiazolo[4,5-b]pyridin-6-yl]-2,4-dihydro-3H-1,2,4-triazol-3-on (30)

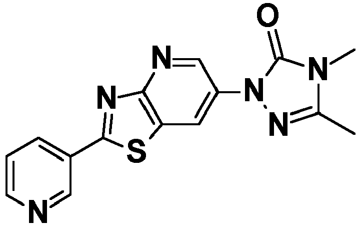

Unter Argon wurden 250 mg (0,86 mmol) 6-Brom-2-(pyridin-3-yl)[1,3]thiazolo[4,5-b]pyridin (I-a-1), 136 mg (1,20 mmol) 4,5-Dimethyl-2,4-dihydro-3H-1,2,4-triazol-3-on, 16 mg (0,09 mmol) Kupfer(I)iodid, 355 mg (2,57 mmol) Kaliumcarbonat und 43 mg (0,26 mmol) Kaliumiodid in 5 ml Dioxan vorgelegt und das Gefäß mit Argon geflutet. Anschließend wurden 24 mg (0,17 mmol) trans-N,N'-Dimethylcyclohexan-1,2-diamin (racemisch) hinzugegeben, das Reaktionsgefäß verschlossen und über Nacht bei 130 °C gerührt. Der abgekühlte Ansatz wurde dann mit 5 ml Ethylacetat verdünnt, über Celite filtriert und das Filtrat im Vakuum eingeengt. Die Reinigung erfolgte chromatographisch mittels MPLC (Fließmittel Wasser/Acetonitril). Man erhielt 6,0 mg (90,0% Reinheit, 1,95% d. Th.) der Titelverbindung (30).
**1H-NMR (400,0 MHz, d₆-DMSO):** δ = 9,336(0,6); 9,317(2,7); 9,310(3,0); 9,307(3,9); 9,300(3,8); 9,119(3,3); 9,113(3,3); 8,808(2,1); 8,800(2,2); 8,796(2,0); 8,712(0,4); 8,543(0,4); 8,522(1,4); 8,518(1,8); 8,512(1,4); 8,502(1,4); 8,497(1,6); 8,493(1,2); 7,673(1,3); 7,661(1,6); 7,655(1,5); 7,643(1,3); 3,556(0,4); 3,322(46,2); 3,255(17,4); 3,177(1,4); 3,141(0,4); 3,127(0,6); 3,122(0,5); 3,076(0,3); 3,060(2,0); 2,755(0,3); 2,736(0,4); 2,728(0,5); 2,670(2,4); 2,666(2,1); 2,505(269,8); 2,501(349,1); 2,497(274,9); 2,356(0,9); 2,338(16,0); 2,250(1,0); 2,227(0,7); 2,119(1,8); 1,234(0,5); 0,883(0,4); 0,000(50,2)

### 4,5-Dimethyl-2-[2-(pyridin-3-yl)[1,3]thiazolo[4,5-b]pyridin-6-yl]-2,4-dihydro-3H-1,2,4-triazol-3-thion (31)

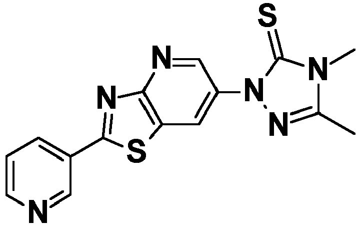

Unter Argon wurden 250 mg (0,86 mmol) 6-Brom-2-(pyridin-3-yl)[1,3]thiazolo[4,5-b]pyridin (I-a-1), 155 mg (1,20 mmol) 4,5-Dimethyl-2,4-dihydro-3H-1,2,4-triazol-3-thion, 16 mg (0,09 mmol) Kupfer(I)iodid, 355 mg (2,57 mmol) Kaliumcarbonat und 43 mg (0,26 mmol) Kaliumiodid in 5 ml Dioxan vorgelegt und das Gefäß mit Argon geflutet. Anschließend wurden 24 mg (0,17 mmol) trans-N,N'-Dimethylcyclohexan-1,2-diamin (racemisch) hinzugegeben, das Reaktionsgefäß verschlossen und über Nacht bei 130 °C gerührt. Der abgekühlte Ansatz wurde dann mit 5 ml Ethylacetat verdünnt, über Celite filtriert und das Filtrat im Vakuum eingeengt. Die Reinigung erfolgte chromatographisch mittels MPLC (Fließmittel Wasser/Acetonitril). Man erhielt 4,0 mg (90,0% Reinheit, 1,24% d. Th.) der Titelverbindung (31).
**1H-NMR (400,0 MHz, d₆-DMSO):** δ = 9,318(0,6); 8,816(0,4); 8,806(0,4); 8,701(0,7); 8,695(1,0); 8,668(0,9); 8,662(0,8); 8,524(0,4); 8,504(0,4); 7,669(0,4); 7,657(0,4); 7,649(0,4); 3,557(4,4); 3,387(0,7); 3,319(4,7); 2,506(37,0); 2,501(51,1); 2,497(42,5); 2,406(4,4); 2,328(0,3); 2,291(0,5); 1,158(2,3); 1,069(16,0); 0,000(7,1); -0,002(4,9)

### 4-Methyl-2-[2-(pyridin-3-yl)[1,3]thiazolo[4,5-b]pyridin-6-yl]-1,2,4-triazin-3,5(2H,4H)-dion (39)

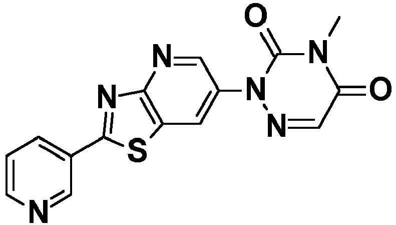

Unter Argon wurden 200 mg (0,69 mmol) 6-Brom-2-(pyridin-3-yl)[1,3]thiazolo[4,5-b]pyridin (I-a-1), 122 mg (0,96 mmol) 4-Methyl-1,2,4-triazin-3,5(2H,4H)-dion, 13 mg (0,07 mmol) Kupfer(I)iodid, 284 mg (2,05 mmol) Kaliumcarbonat und 34 mg (0,21 mmol) Kaliumiodid in 5 ml Dioxan vorgelegt und das Gefäß mit Argon geflutet. Anschließend wurden 19 mg (0,14 mmol) trans-N,N'-Dimethylcyclohexan-1,2-diamin (racemisch) hinzugegeben, das Reaktionsgefäß verschlossen und über Nacht bei 130 °C gerührt. Der abgekühlte Ansatz wurde mit 5 ml Ethylacetat verdünnt, über Celite filtriert und das Filtrat im Vakuum eingeengt. Die Reinigung erfolgte chromatographisch mittels MPLC (Fließmittel Wasser/Acetonitril). Man erhielt 8,0 mg (88,0% Reinheit, 3,04% d. Th.) der Titelverbindung (39).
**1H-NMR (400,0 MHz, d₆-DMSO):** δ = 9,356(8,6); 8,902(8,8); 8,897(11,6); 8,877(11,6); 8,871(8,2); 8,834(6,1); 8,823(6,3); 8,763(1,1); 8,755(1,0); 8,731(0,9); 8,711(0,9); 8,561(5,1); 8,542(5,7); 8,313(0,5); 8,177(0,4); 7,953(1,0); 7,879(16,0); 7,692(4,3); 7,681(5,2); 7,673(4,7); 7,661(4,5); 7,647(0,9); 7,550(0,8); 7,539(0,7); 7,529(0,7); 7,517(0,6); 5,308(0,4); 3,432(0,6); 3,320(260,1); 3,256(48,0); 3,190(0,5); 3,153(0,4); 3,076(0,3); 2,995(3,2); 2,891(4,8); 2,732(4,6); 2,711(1,2); 2,671(2,4); 2,645(0,4); 2,630(0,5); 2,541(215,4); 2,502(389,5); 2,368(2,0); 2,329(3,0); 2,295(0,8); 2,277(0,7); 2,243(0,6); 2,220(0,7); 2,200(0,6); 2,189(1,2); 2,173(0,6); 2,131(0,6); 2,117(0,9); 2,105(0,7); 2,086(0,6); 2,073(0,6); 2,036(0,6); 2,005(0,5); 1,997(0,5); 1,990(0,5); 1,980(0,5); 1,942(0,5); 1,909(5,1); 1,840(0,9); 1,816(0,4); 1,809(0,4); 1,762(0,4); 1,754(0,4); 1,722(0,3); 1,704(0,4); 1,673(0,3); 1,621(0,3); 1,610(0,4); 1,605(0,4); 1,603(0,4); 1,585(0,4); 1,574(0,4); 1,536(0,5); 1,514(0,6); 1,507(0,6); 1,500(0,6); 1,490(0,6); 1,466(0,7); 1,458(0,6); 1,386(1,4); 1,380(1,4); 1,347(1,8); 1,335(1,8); 1,298(1,7); 1,259(1,7); 1,234(2,6); 1,201(1,3); 1,141(1,4); 1,085(0,5); 1,070(0,6); 1,054(0,5); 1,045(0,5); 1,035(0,5); 0,988(0,5); 0,968(0,4); 0,922(0,3); 0,892(0,4); 0,864(0,6); 0,852(0,9); 0,830(1,3); 0,756(0,5); 0,726(0,3); 0,000(43,1)

### Synthese von Verbindungen der Formel (I) nach Methode G

### Methyl-2-(pyridin-3-yl)[1,3]thiazolo[4,5-b]pyridin-6-carboxylat (I-j-1)

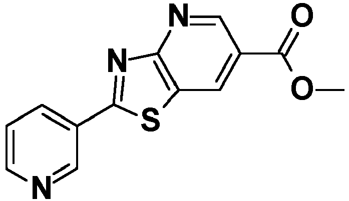

Zu einer Mischung aus 10,8 g (36,97 mmol) 6-Brom-2-(pyridin-3-yl)[1,3]thiazolo[4,5-b]pyridin (I-a-1) in 200 ml THF, 100 ml DMF und 100 ml Methanol wurden 11,22 g (110,91 mmol) Triethylamin und 5,41 g (7,39 mmol) [1,1'-Bis(diphenylphosphino)ferrocen]dichlor-palladium(II) gegeben. Kohlenmonoxid wurde in die Lösung geleitet und der Ansatz bei 70 °C und 45 psi CO-Druck für 16 Stunden gerührt. Anschließend wurde die Mischung filtriert und im Vakuum eingedampft. Die Reinigung erfolgte chromatographisch mittels MPLC (Fließmittelgradient Petrolether/Ethylacetat). Man erhielt 7,50 g der Zielverbindung (I-j-1), die ohne weitere Aufreinigung weiter zu Amiden umgesetzt wurde. Hierfür wurde eine Reinheit von 100% angenommen.
**LC-MS:** *m*/*z* 272 [M+H⁺]

### Methyl-2-(5-fluorpyridin-3-yl)[1,3]thiazolo[4,5-b]pyridin-6-carboxylat (I-j-2)

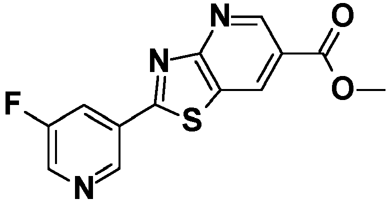

Die Herstellung erfolgte in Analogie zur Synthese von (I-j-1), im Unterschied ausgehend von 3-(Chlorcarbonyl)-5-fluorpyridiniumchlorid in Methode A, Schritt 1.
**LC-MS:** *m*/*z* 290 [M+H⁺]

### N-[2-(Methylsulfanyl)ethyl]-2-(pyridin-3-yl)[1,3]thiazolo[4,5-b]pyridin-6-carboxamid (52)

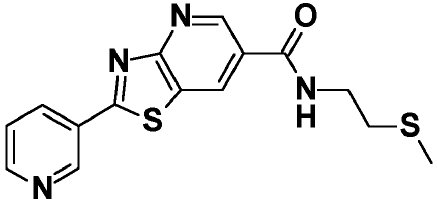

Zu einer Lösung von 300 mg (1,11 mmol, angenommene Reinheit 100%) Methyl-2-(pyridin-3-yl)[1,3]thiazolo[4,5-b]pyridin-6-carboxylat (I-j-1) in 8 ml THF wurden 1,2 Moläquivalente (eq.) Kaliumtrimethylsilanolat gegeben. Die Mischung wurde noch für 16 Stunden bei 30 °C gerührt und dann im Vakuum eingedampft. Der Rückstand wurde in 5 ml DMF aufgenommen und 1,2 Moläquivalente (eq.) 2-(7-Aza-1H-benzotriazol-1-yl)-1,1,3,3-tetramethyluronium-hexafluorophosphat (HATU) und 3 Moläquivalente (eq.) Diisopropylethylamin hinzugegeben. Die Mischung wurde 30 Minuten bei 30 °C gerührt. Anschließend wurden 121,4 mg (1,33 mmol, 1,2 eq.) 2-(Methylsulfanyl)ethanamin zugesetzt und der Ansatz noch für 16 Stunden bei 30 °C gerührt. Die Reaktionsmischung wurde danach im Vakuum eingedampft. Die Aufreinigung erfolgte chromatographisch mittels MPLC. Man erhielt 49,2 mg (95,4% Reinheit, 12,8% d. Th.) der Titelverbindung (52).
**1H-NMR (400,0 MHz, d₆-DMSO):** δ = 9,365(1,2); 9,153(2,3); 9,148(2,8); 9,101(2,7); 9,096(2,4); 8,981(0,5); 8,967(1,0); 8,953(0,5); 8,843(0,9); 8,831(0,8); 8,574(0,7); 8,568(1,0); 8,564(0,7); 8,554(0,7); 8,548(1,0); 8,544(0,8); 7,695(0,8); 7,683(0,8); 7,676(0,8); 7,664(0,8); 3,902(4,0); 3,556(0,8); 3,540(1,6); 3,522(1,6); 3,507(0,9); 3,356(130,8); 3,349(200,4); 2,724(1,7); 2,706(2,5); 2,689(1,6); 2,678(0,4); 2,673(0,5); 2,669(0,3); 2,526(1,2); 2,513(24,8); 2,509(53,4); 2,504(76,2); 2,500(58,4); 2,495(28,7); 2,331(0,4); 2,326(0,3); 2,128(16,0); 0,000(2,5)

### N-[2-(Methylsulfonyl)ethyl]-2-(pyridin-3-yl)[1,3]thiazolo[4,5-b]pyridin-6-carboxamid (54)

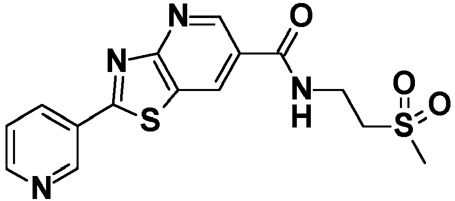

Zu einer Lösung von 300 mg (1,11 mmol, angenommene Reinheit 100%) Methyl-2-(pyridin-3-yl)[1,3]thiazolo[4,5-b]pyridin-6-carboxylat (I-j-1) in 8 ml THF wurden 1,2 Moläquivalente (eq.) Kaliumtrimethylsilanolat gegeben. Die Mischung wurde noch für 16 Stunden bei 30 °C gerührt und dann im Vakuum eingedampft. Der Rückstand wurde in 5 ml DMF aufgenommen und 1,2 Moläquivalente (eq.) 2-(7-Aza-1H-benzotriazol-1-yl)-1,1,3,3-tetramethyluronium-hexafluorophosphat (HATU) und 3 Moläquivalente (eq.) Diisopropylethylamin hinzugegeben. Die Mischung wurde 30 Minuten bei 30 °C gerührt. Anschließend wurden 163,8 mg (1,33 mmol, 1,2 eq.) 2-(Methylsulfonyl)ethanamin zugesetzt und der Ansatz noch für 16 Stunden bei 30 °C gerührt. Die Reaktionsmischung wurde danach im Vakuum eingedampft. Die Aufreinigung erfolgte chromatographisch mittels MPLC. Man erhielt 74,3 mg (97,6% Reinheit, 18,0% d. Th.) der Titelverbindung (54).
**1H-NMR (400,0 MHz, d₆-DMSO):** δ = 9,372(1,9); 9,152(3,4); 9,147(4,5); 9,125(1,7); 9,115(4,8); 9,109(4,2); 8,849(1,3); 8,838(1,3); 8,576(1,0); 8,571(1,4); 8,567(1,1); 8,556(1,1); 8,551(1,5); 8,547(1,1); 7,698(1,2); 7,686(1,2); 7,678(1,2); 7,666(1,1); 3,955(0,6); 3,902(8,0); 3,777(0,9); 3,760(2,4); 3,745(2,5); 3,729(1,2); 3,455(2,2); 3,438(4,0); 3,421(1,9); 3,344(186,2); 3,336(242,0); 3,176(0,6); 3,162(0,6); 3,075(16,0); 2,677(0,6); 2,672(0,8); 2,668(0,6); 2,543(0,6); 2,526(2,1); 2,512(47,9); 2,508(101,9); 2,503(144,1); 2,499(111,8); 2,334(0,6); 2,330(0,8); 2,325(0,6); 0,000(3,8)

### 2-(5-Fluorpyridin-3-yl)-N-{3-[(trifluormethyl)sulfanyl]phenyl}[1,3]thiazolo[4,5-b]pyridin-6-carboxamid (57)

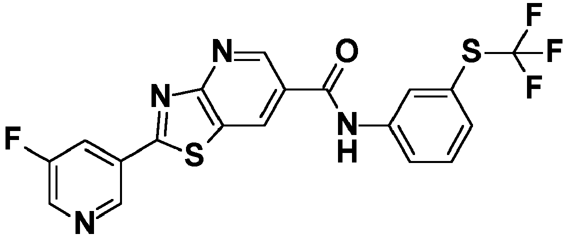

Zu einer Lösung von 300 mg (1,11 mmol, angenommene Reinheit 100%) Methyl-2-(5-fluorpyridin-3-yl)[1,3]thiazolo[4,5-b]pyridin-6-carboxylat (I-j-2) in 8 ml THF wurden 1,2 Moläquivalente (eq.) Kaliumtrimethylsilanolat gegeben. Die Mischung wurde noch für 16 Stunden bei 30 °C gerührt und dann im Vakuum eingedampft. Der Rückstand wurde in 5 ml DMF aufgenommen und 1,2 Moläquivalente (eq.) 2-(7-Aza-1H-benzotriazol-1-yl)-1,1,3,3-tetramethyluronium-hexafluorophosphat (HATU) und 3 Moläquivalente (eq.) Diisopropylethylamin hinzugegeben. Die Mischung wurde 30 Minuten bei 30 °C gerührt. Anschließend wurden 256,9 mg (1,33 mmol, 1,2 eq.) 3-[(Trifluormethyl)sulfanyl]anilin zugesetzt und der Ansatz noch für 16 Stunden bei 30 °C gerührt. Die Reaktionsmischung wurde danach im Vakuum eingedampft. Die Aufreinigung erfolgte chromatographisch mittels MPLC. Man erhielt 41,3 mg (99,5% Reinheit, 8,2% d. Th.) der Titelverbindung (57).
**1H-NMR(400,0 MHz, d₆-DMSO):** δ = 10,872(8,7); 9,298(6,5); 9,293(10,8); 9,277(16,0); 8,887(6,9); 8,880(7,1); 8,555(2,2); 8,549(3,3); 8,544(2,4); 8,532(2,3); 8,525(3,3); 8,521(2,3); 8,308(0,3); 8,273(7,1); 8,025(3,4); 8,004(3,7); 7,605(3,2); 7,585(7,0); 7,565(4,6); 7,509(5,1); 7,490(3,2); 3,902(15,3); 3,705(0,4); 3,657(0,4); 3,603(0,6); 3,508(1,4); 3,361(1440,1); 3,174(0,6); 2,678(1,6); 2,674(2,2); 2,670(1,6); 2,582(0,4); 2,544(1,6); 2,509(266,9); 2,505(367,0); 2,500(281,7); 2,336(1,5); 2,331(2,0); 1,233(0,6); 0,000(7,6)

### 2-(5-Fluorpyridin-3-yl)-N-{3-[(trifluormethyl)sulfinyl]phenyl}[1,3]thiazolo[4,5-b]pyridin-6-carboxamid (58)

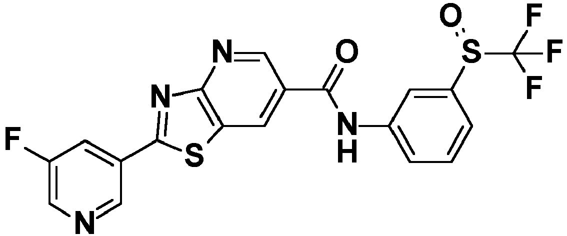

Zu einer Lösung von 300 mg (0,67 mmol) 2-(5-Fluorpyridin-3-yl)-N-{3-[(trifluormethyl)sulfanyl]phenyl}[1,3]thiazolo[4,5-b]pyridin-6-carboxamid (57) in 10 ml Dichlormethan wurden 2 Moläquivalente (222,5 mg, 1,3 mmol) meta-Chlorperbenzoesäure bei 0 °C zugesetzt. Die Reaktionsmischung wurde noch für 16 Stunden bei 20 °C gerührt und anschließend im Vakuum eingedampft. Die Aufreinigung erfolgte chromatographisch mittels MPLC. Man erhielt 17,4 mg (95,5% Reinheit, 5,3% d. Th.) der Titelverbindung (58).
**1H-NMR (601,6 MHz, d₆-DMSO):** δ = 11,037(1,3); 9,317(7,8); 9,313(14,4); 9,306(16,0); 9,302(8,6); 9,278(8,6); 8,887(8,8); 8,883(10,4); 8,833(0,9); 8,829(0,8); 8,825(0,9); 8,554(2,8); 8,551(3,7); 8,547(2,8); 8,539(3,0); 8,536(3,8); 8,532(2,6); 8,452(8,0); 8,270(1,6); 8,189(0,8); 8,176(0,9); 8,160(4,1); 8,147(4,3); 8,145(4,3); 8,022(0,8); 8,007(0,8); 7,760(4,0); 7,746(8,2); 7,733(4,8); 7,646(5,2); 7,634(4,1); 7,596(0,7); 7,583(1,5); 7,569(0,9); 7,505(1,0); 7,493(0,8); 3,322(411,9); 2,614(2,3); 2,611(1,7); 2,541(1,0); 2,523(4,5); 2,520(5,8); 2,517(6,8); 2,505(256,7); 2,502(334,0); 2,499(250,5); 2,386(2,0); 1,231(0,6); 0,000(7,3)

### Synthese von Verbindungen der Formel (I) nach Methode H

### 2-(Pyridin-3-yl)[1,3]thiazolo[4,5-b]pyridin-6-amin (I-s-1)

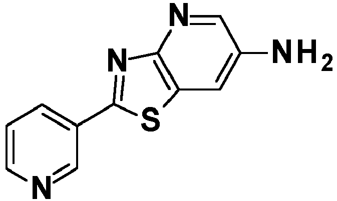

Zu einer gerührten Lösung von 8,0 g (31 mmol) 6-Nitro-2-(pyridin-3-yl)[1,3]thiazolo[4,5-b]pyridin (I-r-1) (hergestellt analog der Synthese zur Verbindung (I-a-1), ausgehend von 3-Brom-5-nitropyridin-2-amin in Methode A, Schritt 1) in 300 ml THF wurden bei 30 °C unter Stickstoff 1,6 g Pd/C (10 wt%) gegeben. Die Mischung wurde bei 50 °C unter Wasserstoff (50 psi) für 48 Stunden gerührt. Anschließend wurde die Reaktionsmischung filtriert und das Filtrat im Vakuum eingedampft. Man erhielt 5,0 g 2-(Pyridin-3-yl)[1,3]thiazolo[4,5-b]pyridin-6-amin (I-s-1) als Rohprodukt, das ohne weitere Aufreinigung weiter zu Amiden umgesetzt wurde. Hierfür wurde eine Reinheit von 100% angenommen.
**LC-MS:** *m*/*z* 229 [M+H⁺]

### 2-(5-Fluorpyridin-3-yl)[1,3]thiazolo[4,5-b]pyridin-6-amin (I-s-2)

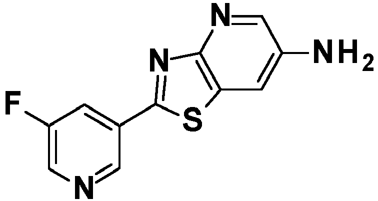

Die Herstellung erfolgte in Analogie zur Synthese von (I-s-1), im Unterschied ausgehend von 3-(Chlorcarbonyl)-5-fluorpyridiniumchlorid in Methode A, Schritt 1.
**LC-MS:** *m*/*z* 247 [M+H⁺]

### N-Methyl-N-[2-(pyridin-3-yl)[1,3]thiazolo[4,5-b]pyridin-6-yl]propanamid (59)

### Schritt 1: N-[2-(Pyridin-3-yl)[1,3]thiazolo[4,5-b]pyridin-6-yl]propanamid

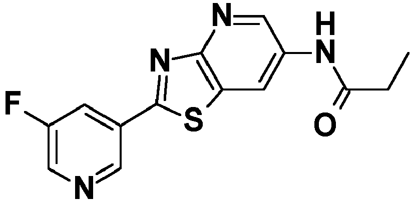

Zu einer Mischung von 300 mg (1,31 mmol) 2-(Pyridin-3-yl)[1,3]thiazolo[4,5-b]pyridin-6-amin (I-s-2) und 398 mg (3,93 mmol) Triethylamin in 5 ml Dichlormethan wurden 121,6 mg (1,31 mmol) Propanoylchlorid gegeben. Der Ansatz wurde für 16 Stunden bei 30 °C gerührt und anschließend im Vakuum eingedampft. Die Aufreinigung erfolgte chromatographisch mittels MPLC (Fließmittelgradient Petrolether/Ethylacetat). Man erhielt 300 mg (80,5% d. Th., angenommene Reinheit 100%) der Titelverbindung N-[2-(Pyridin-3-yl)[1,3]thiazolo[4,5-b]pyridin-6-yl]propanamid, die direkt weiter umgesetzt wurden.

### Schritt 2: N-Methyl-N-[2-(pyridin-3-yl)[1,3]thiazolo[4,5-b]pyridin-6-yl]propanamid (59)

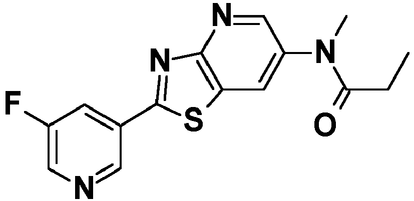

172 mg (0,605 mmol) N-[2-(Pyridin-3-yl)-1,3-benzothiazol-6-yl]propanamid (Beispiel 59, Schritt 1) wurden in 5 ml THF gelöst und auf 0 °C abgekühlt. Anschließend wurden 24,2 mg (0,605 mmol) Natriumhydrid (60%ige Suspension in Paraffin) bei 0 °C zugesetzt. Die Mischung wurde bei dieser Temperatur noch 30 Minuten gerührt und dann 85,9 mg (0,605 mmol) Methyliodid zugegeben. Der Ansatz wurde bei 30 °C noch 16 Stunden gerührt und dann bei 0 °C mit 1 ml wässriger Ammoniumchloridlösung versetzt. Nach Eindampfen der Mischung im Vakuum wurde der Rückstand chromatographisch mittels präparativer HPLC gereinigt. Man erhielt 34,8 mg (99,9% Reinheit, 19,3% d. Th.) der Titelverbindung (59).
**1H-NMR (400,0 MHz, d₆-DMSO):** δ = 9,231(6,6); 8,861(5,8); 8,854(6,1); 8,761(8,3); 8,508(1,9); 8,503(2,6); 8,485(1,9); 8,480(2,6); 8,475(1,8); 3,902(16,0); 3,508(0,4); 3,342(396,2); 3,338(410,6); 3,176(0,7); 3,164(0,7); 2,677(1,1); 2,672(1,5); 2,668(1,2); 2,548(0,6); 2,525(4,3); 2,512(90,4); 2,508(185,6); 2,503(257,8); 2,499(197,1); 2,335(1,2); 2,330(1,5); 2,325(1,2); 2,199(0,5); 2,143(0,6); 2,130(0,6); 2,112(0,6); 2,074(0,4); 1,249(0,3); 1,235(0,3); 0,969(4,6); 0,008(0,4); 0,000(10,5)

Verbindungen der Formel (I) und auch solche, die nicht unter die Formel (I) fallen, sind in der folgenden Tabelle aufgeführt. Auch die nicht unter die Formel (I) fallenden Verbindungen sind Gegenstand der Erfindung.

Auch die in der Tabelle aufgeführten Verbindungen I-a-1, I-j-1, I-j-2, I-s-1 und I-s-2 sind Gegenstand der Erfindung.

**Tabelle 1**

| Verbindungen der Formel | | | | | |
|---|---|---|---|---|---|
| 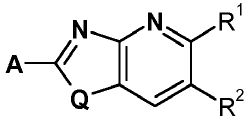 | | | | | |

| **Herstellmethode** | **Beispiel-Nr.** | **A** | **Q** | **R¹** | **R²** |
|---|---|---|---|---|---|
| B | 1 | 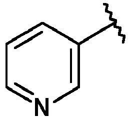 | S | H | 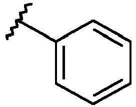 |
| B | 2 | 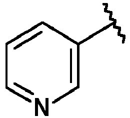 | S | H | 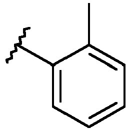 |
| B | 3 | 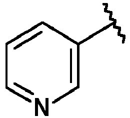 | S | H | 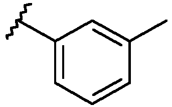 |
| B | 4 | 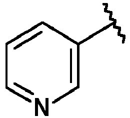 | S | H | 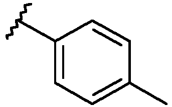 |
| B | 5 | 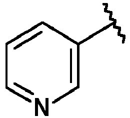 | S | H | 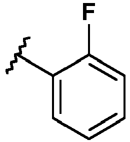 |
| B | 6 | 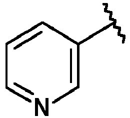 | S | H | 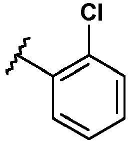 |
| B | 7 | 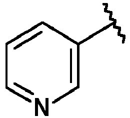 | S | H | 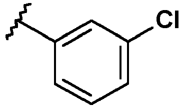 |
| B | 8 | 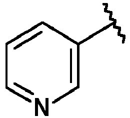 | S | H | 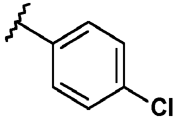 |
| B | 9 | 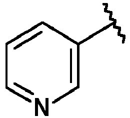 | S | H | 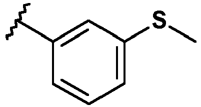 |
| B | 10 | 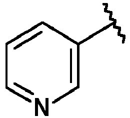 | S | H | 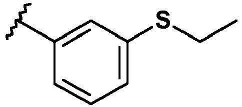 |
| B | 11 | 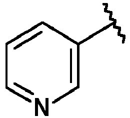 | S | H | 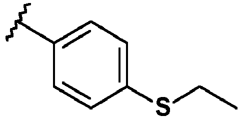 |
| I | 12 | 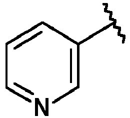 | S | H | 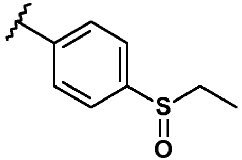 |
| I | 13 | 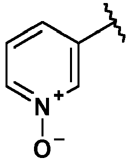 | S | H | 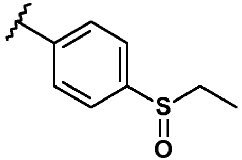 |
| B | 14 | 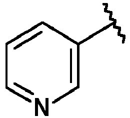 | S | H | 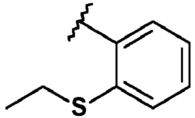 |
| I | 15 | 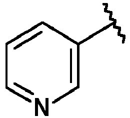 | S | H | 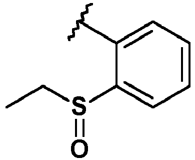 |
| I | 16 | 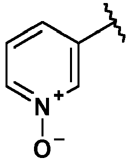 | S | H | 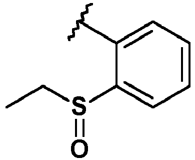 |
| B | 17 | 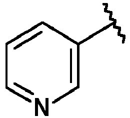 | S | H | 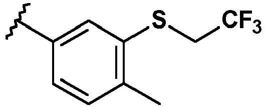 |
| I | 18 | 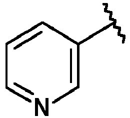 | S | H | 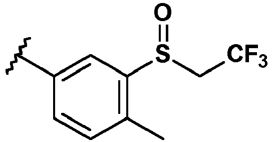 |
| B | 19 | 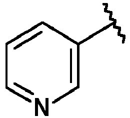 | S | H | 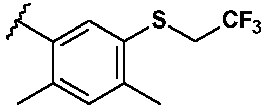 |
| I | 20 | 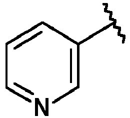 | S | H | 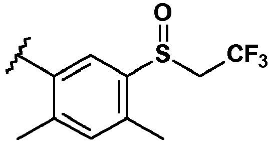 |
| I | 21 | 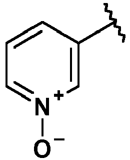 | S | H | 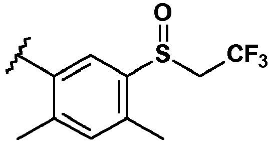 |
| B | 22 | 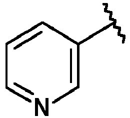 | S | H | 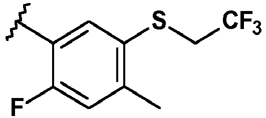 |
| I | 23 | 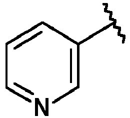 | S | H | 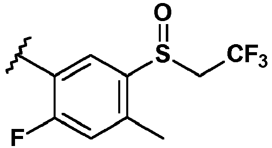 |
| D | 24 | 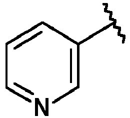 | S | H | 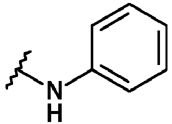 |
| D | 25 | 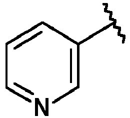 | S | H | 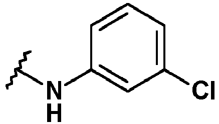 |
| E | 26 | 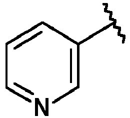 | S | H | 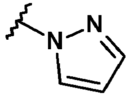 |
| E | 27 | 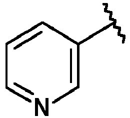 | S | H | 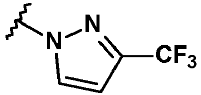 |
| E | 28 | 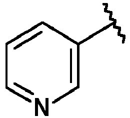 | S | H | 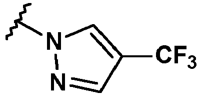 |
| E | 29 | 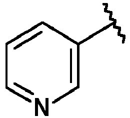 | S | H | 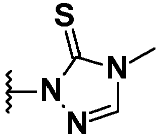 |
| E | 30 | 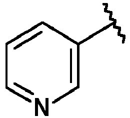 | S | H | 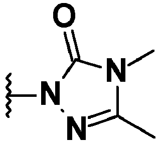 |
| E | 31 | 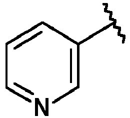 | S | H | 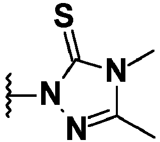 |
| E | 32 | 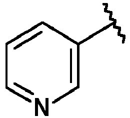 | S | H | 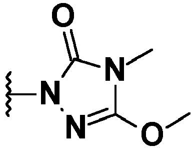 |
| E | 33 | 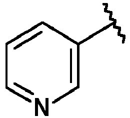 | S | H | 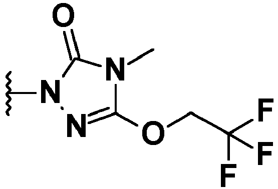 |
| E | 34 | 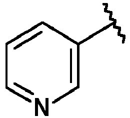 | S | H | 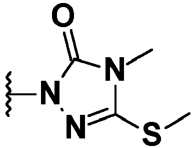 |
| E | 35 | 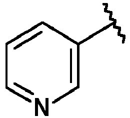 | S | H | 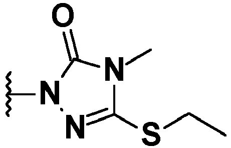 |
| E | 36 | 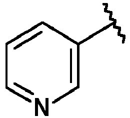 | S | H | 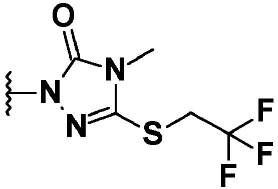 |
| E | 37 | 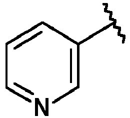 | S | H | 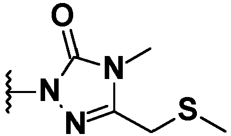 |
| E | 38 | 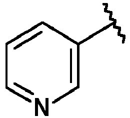 | S | H | 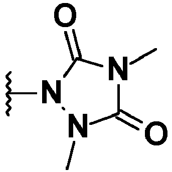 |
| E | 39 | 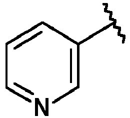 | S | H | 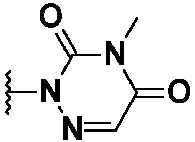 |
| G | 40 | 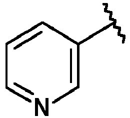 | S | H | 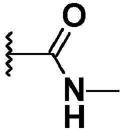 |
| G | 41 | 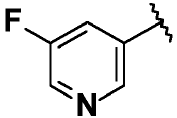 | S | H | 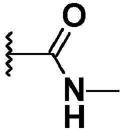 |
| G | 42 | 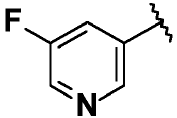 | S | H | 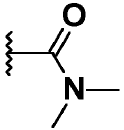 |
| G | 43 | 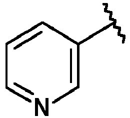 | S | H | 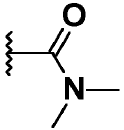 |
| G | 44 | 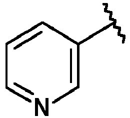 | S | H | 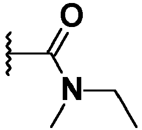 |
| G | 45 | 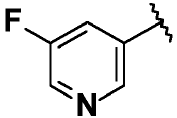 | S | H | 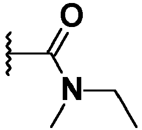 |
| G | 46 | 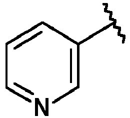 | S | H | 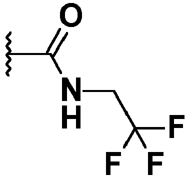 |
| G | 47 | 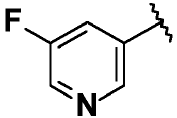 | S | H | 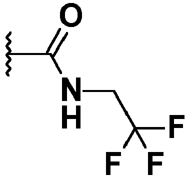 |
| G | 48 | 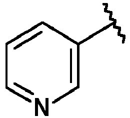 | S | H | 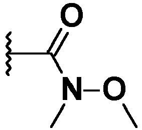 |
| G | 49 | 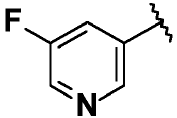 | S | H | 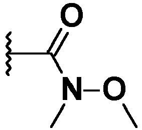 |
| G | 50 | 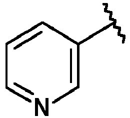 | S | H | 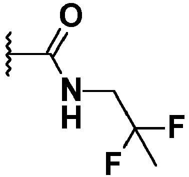 |
| G | 51 | 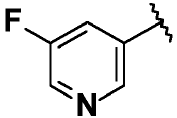 | S | H | 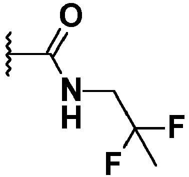 |
| G | 52 | 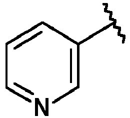 | S | H | 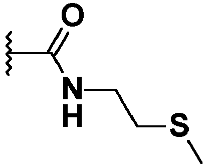 |
| G | 53 | 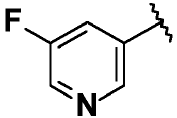 | S | H | 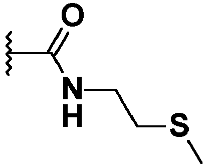 |
| G | 54 | 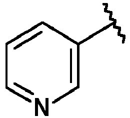 | S | H | 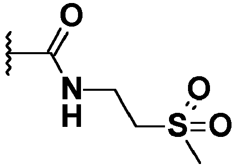 |
| G | 55 | 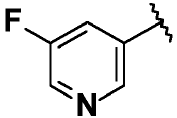 | S | H | 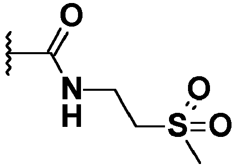 |
| G | 56 | 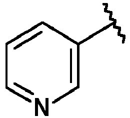 | S | H | 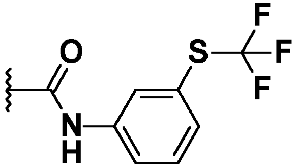 |
| G | 57 | 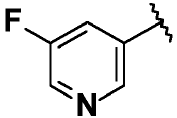 | S | H | 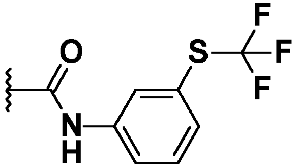 |
| I | 58 | 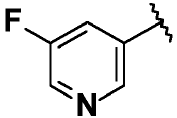 | S | H | 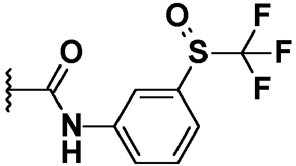 |
| H2 | 59 | 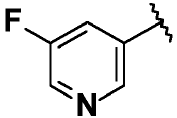 | S | H | 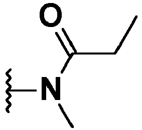 |
| A | I-a-1 | 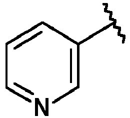 | S | H | Br |
| G | I-j-1 | 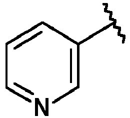 | S | H | 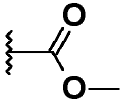 |
| G | I-j-2 | 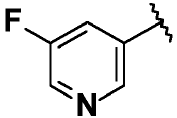 | S | H | 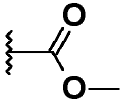 |
| H2 | I-s-1 | 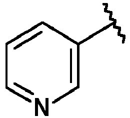 | S | H | 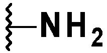 |
| H2 | I-s-2 | 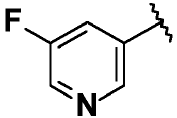 | S | H | 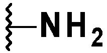 |

**Tabelle 2**

| Analytische Daten zu den angegebenen Verbindungen | | | |
|---|---|---|---|
| **Bsp.-Nr.** | **logP[a]** | **logP[b]** | **1H-NMR [σ (ppm)] bzw. LC-MS [m/z]** |
| 1 | 2,47 | 2,56 | ¹H-NMR (400,0 MHz, d₆-DMSO): |
| | | | δ = 9,366 (4,5); 9,361 (4,6); 9,084 (7,0); 9,079 (8,7); 9,029 (7,7); 9,023 (6,6); 8,830 (3,7); 8,826 (4,1); 8,818 (4,0); 8,814 (4,1); 8,574 (2,2); 8,570 (2,9); 8,564 (2,3); 8,554 (2,4); 8,549 (3,1); 8,544 (2,4); 8,316 (0,3); 7,875 (5,1); 7,871 (7,3); 7,853 (7,7); 7,692 (2,8); 7,690 (2,9); 7,678 (2,9); 7,672 (2,8); 7,670 (2,8); 7,660 (2,7); 7,658 (2,8); 7,585 (3,8); 7,567 (8,4); 7,547 (5,3); 7,495 (3,1); 7,492 (2,0); 7,481 (1,2); 7,476 (4,3); 7,471 (1,1); 7,458 (1,4); 3,762 (0,6); 3,569 (0,6); 3,351 (267,6); 3,349 (243,9); 3,340 (242,3); 3,335 (116,9); 2,682 (0,4); 2,677 (0,9); 2,673 (1,2); 2,668 (0,9); 2,526 (2,6); 2,521 (3,9); 2,513 (61,0); 2,508 (126,9); 2,504 (172,8); 2,499 (131,6); 2,495 (67,2); 2,335 (0,8); 2,331 (1,2); 2,326 (0,8); 2,075 (16,0); 0,146 (0,5); 0,008 (3,7); 0,000 (122,8); -0,008 (5,0); -0,150 (0,5) |
| 2 | 2,76 | 2,8 | ¹H-NMR (400,0 MHz, d₆-DMSO): |
| | | | δ = 9,366 (2,3); 9,361 (2,3); 8,833 (1,7); 8,829 (1,9); 8,821 (1,8); 8,817 (1,9); 8,753 (2,7); 8,748 (4,7); 8,735 (4,4); 8,730 (2,6); 8,573 (1,0); 8,569 (1,5); 8,564 (1,0); 8,553 (1,1); 8,549 (1,5); 8,544 (1,0); 7,693 (1,4); 7,681 (1,4); 7,673 (1,3); 7,661 (1,3); 7,404 (0,5); 7,393 (2,4); 7,390 (2,7); 7,383 (3,6); 7,376 (3,2); 7,369 (3,3); 7,362 (4,8); 7,355 (1,5); 7,349 (1,1); 7,340 (0,4); 3,329 (15,8); 2,526 (0,8); 2,508 (33,3); 2,504 (44,1); 2,500 (33,0); 2,383 (0,4); 2,331 (0,4); 2,315 (16,0); 0,008 (1,8); 0,000 (50,9); -0,009 (2,0) |
| 3 | 2,91 | 2,95 | ¹H-NMR (400,0 MHz, d₆-DMSO): |
| | | | δ = 9,362 (2,9); 9,357 (2,9); 9,066 (3,5); 9,060 (4,2); 9,008 (4,0); 9,003 (3,7); 8,824 (2,3); 8,815 (2,1); 8,812 (2,2); 8,563 (1,8); 8,559 (1,3); 8,548 (1,3); 8,543 (1,9); 8,539 (1,3); 7,688 (5,2); 7,676 (2,1); 7,668 (1,9); 7,656 (3,4); 7,636 (2,1); 7,463 (1,6); 7,444 (3,2); 7,425 (1,7); 7,297 (2,2); 7,279 (1,7); 3,346 (101,5); 3,339 (98,5); 2,673 (0,5); 2,508 (59,8); 2,504 (75,9); 2,500 (59,5); 2,424 (16,0); 2,330 (0,5); 2,076 (0,4); 0,000 (41,3) |
| 4 | 2,91 | 2,96 | ¹H-NMR (400,0 MHz, d₆-DMSO): |
| | | | δ = 9,358 (3,1); 9,353 (3,2); 9,060 (4,6); 9,054 (5,4); 8,992 (5,5); 8,987 (4,9); 8,824 (2,4); 8,820 (2,7); 8,812 (2,5); 8,808 (2,7); 8,564 (1,4); 8,559 (2,0); 8,554 (1,5); 8,544 (1,5); 8,539 (2,1); 8,534 (1,5); 7,914 (1,2); 7,766 (5,5); 7,746 (6,3); 7,685 (2,1); 7,672 (2,0); 7,665 (2,1); 7,652 (1,8); 7,378 (5,1); 7,358 (4,6); 3,328 (39,8); 2,677 (0,4); 2,672 (0,6); 2,668 (0,5); 2,544 (0,3); 2,525 (1,8); 2,512 (34,6); 2,508 (69,0); 2,503 (91,4); 2,499 (69,9); 2,388 (16,0); 2,330 (0,7); 2,326 (0,5); 2,298 (1,2); 0,146 (0,5); 0,008 (3,8); 0,000 (98,0); -0,008 (4,7); -0,150 (0,5) |
| 5 | 2,51 | 2,54 | ¹H-NMR (400,0 MHz, d₆-DMSO): |
| | | | δ = 9,373 (11,3); 9,369 (11,2); 9,367 (10,9); 8,952 (7,9); 8,947 (14,9); 8,943 (13,7); 8,933 (12,1); 8,928 |

| **Bsp.-Nr.** | **logP[a]** | **logP[b]** | **1H-NMR [σ (ppm)] bzw. LC-MS [m/z]** |
|---|---|---|---|
| | | | (15,9); 8,923 (7,2); 8,837 (9,0); 8,833 (10,0); 8,825 (9,6); 8,821 (9,8); 8,582 (5,5); 8,578 (7,1); 8,577 (6,9); 8,573 (5,6); 8,562 (5,9); 8,558 (7,1); 8,557 (7,6); 8,553 (5,7); 8,318 (2,6); 8,189 (1,8); 8,091 (0,4); 8,071 (0,5); 7,998 (1,3); 7,758 (3,8); 7,754 (4,3); 7,738 (7,0); 7,734 (8,3); 7,718 (4,2); 7,714 (4,9); 7,697 (7,1); 7,695 (7,1); 7,684 (6,6); 7,683 (6,8); 7,677 (6,6); 7,675 (6,8); 7,664 (6,4); 7,663 (6,6); 7,570 (1,7); 7,566 (2,0); 7,557 (2,0); 7,552 (4,2); 7,548 (4,0); 7,540 (2,9); 7,535 (4,5); 7,532 (5,5); 7,527 (3,4); 7,518 (3,1); 7,514 (2,9); 7,454 (6,4); 7,436 (4,4); 7,433 (4,7); 7,426 (14,0); 7,407 (16,0); 7,389 (4,9); 7,386 (4,5); 7,321 (0,8); 7,318 (0,7); 7,308 (2,2); 7,249 (0,7); 7,229 (0,7); 7,152 (0,4); 7,075 (0,4); 6,600 (0,3); 5,162 (0,6); 4,497 (0,9); 4,482 (0,8); 4,038 (0,6); 4,020 (0,7); 3,397 (0,4); 3,328 (888,9); 3,295 (0,5); 3,005 (0,7); 2,987 (0,7); 2,676 (4,7); 2,671 (6,6); 2,667 (4,9); 2,663 (2,4); 2,525 (16,1); 2,520 (24,4); 2,511 (350,0); 2,507 (724,8); 2,502 (967,1); 2,498 (711,5); 2,493 (349,4); 2,403 (0,6); 2,374 (0,7); 2,338 (2,1); 2,334 (4,6); 2,329 (6,4); 2,325 (4,8); 2,320 (2,3); 1,989 (2,6); 1,355 (0,7); 1,260 (0,9); 1,249 (0,4); 1,242 (1,8); 1,234 (1,4); 1,224 (0,9); 1,212 (0,3); 1,193 (1,0); 1,175 (1,6); 1,157 (0,7); 0,000 (2,4) |
| 6 | 2,77 | 2,78 | ¹H-NMR (400,0 MHz, d₆-DMSO): |
| | | | δ = 9,373 (8,3); 9,368 (8,3); 8,854 (12,7); 8,848 (16,0); 8,839 (6,8); 8,835 (7,3); 8,827 (7,2); 8,823 (7,4); 8,806 (15,3); 8,800 (12,6); 8,582 (3,8); 8,577 (5,2); 8,572 (4,0); 8,562 (4,2); 8,556 (5,6); 8,552 (4,1); 8,318 (0,5); 7,696 (5,5); 7,687 (8,6); 7,681 (5,0); 7,676 (10,0); 7,672 (4,5); 7,664 (11,9); 7,655 (1,3); 7,651 (0,7); 7,634 (0,7); 7,626 (5,0); 7,618 (3,6); 7,614 (6,6); 7,608 (4,5); 7,602 (8,0); 7,594 (1,3); 7,589 (0,6); 7,567 (0,5); 7,555 (0,6); 7,548 (2,3); 7,543 (1,4); 7,535 (12,8); 7,530 (7,4); 7,524 (10,0); 7,518 (6,5); 7,512 (9,2); 7,500 (1,3); 7,493 (0,5); 6,535 (1,3); 5,758 (0,8); 3,569 (0,4); 3,328 (120,3); 2,681 (0,6); 2,677 (1,2); 2,672 (1,6); 2,668 (1,2); 2,526 (4,2); 2,521 (6,4); 2,512 (87,3); 2,508 (178,1); 2,503 (238,6); 2,499 (179,7); 2,495 (92,4); 2,335 (1,2); 2,330 (1,6); 2,326 (1,2); 2,076 (0,4); 0,146 (1,3); 0,008 (9,3); 0,000 (278,6); -0,008 (12,8); -0,020 (0,7); -0,0256 (0,3); -0,0263 (0,3); -0,150 (1,3) |
| 7 | 2,98 | 3,01 | ¹H-NMR (400,0 MHz, d₆-DMSO): |
| | | | δ = 9,374 (8,4); 9,368 (8,3); 9,117 (11,3); 9,111 (15,3); 9,084 (16,0); 9,078 (11,2); 8,834 (6,6); 8,830 (6,7); 8,822 (6,8); 8,818 (6,5); 8,582 (3,8); 8,577 (5,3); 8,572 (3,6); 8,562 (4,1); 8,557 (5,5); 8,552 (3,5); 8,317 (1,9); 7,969 (6,9); 7,964 (12,0); 7,960 (6,7); 7,857 (5,8); 7,838 (6,5); 7,811 (0,4); 7,693 (5,3); 7,681 (5,2); 7,673 (5,1); 7,661 (4,9); 7,611 (4,5); 7,591 (10,7); 7,572 (7,8); 7,547 (7,4); 7,530 (2,8); 7,527 (3,2); 7,289 (0,4); 6,937 (0,5); 6,925 (0,4); 6,914 (0,4); 5,757 (0,7); 3,744 (3,2); 3,327 (467,3); 2,676 (4,1); 2,671 (5,6); 2,667 (4,1); 2,602 (0,5); 2,524 (15,1); 2,507 (606,6); 2,502 (788,2); 2,498 |
| | | | (575,0); 2,333 (3,8); 2,329 (5,2); 2,325 (3,7); 2,170 (0,5); 1,355 (0,5); 1,277 (0,4); 1,259 (0,5); 1,232 (5,8); 1,154 (0,4); 1,136 (0,6); 1,118 (0,4); 0,853 (0,7); 0,836 (0,4); 0,146 (2,7); 0,082 (0,4); 0,073 (6,2); 0,008 (20,0); 0,000 (579,1); -0,009 (21,1); -0,150 (2,8) |
| 8 | 2,98 | 3,03 | ¹H-NMR (400,0 MHz, d₆-DMSO): |
| | | | δ = 9,366 (4,0); 9,362 (3,8); 9,087 (5,4); 9,082 (6,9); 9,044 (7,1); 9,039 (5,5); 8,830 (3,1); 8,826 (3,3); 8,818 (3,2); 8,814 (3,2); 8,574 (1,8); 8,569 (2,5); 8,564 (1,8); 8,554 (1,9); 8,549 (2,6); 8,544 (1,8); 8,321 (3,9); 7,920 (1,0); 7,915 (1,3); 7,909 (7,7); 7,904 (3,1); 7,892 (3,1); 7,887 (9,1); 7,688 (2,5); 7,676 (2,4); 7,668 (2,4); 7,656 (2,3); 7,635 (8,9); 7,613 (7,6); 7,194 (0,5); 7,175 (0,6); 5,758 (0,3); 3,339 (24,8); 2,677 (0,7); 2,673 (1,0); 2,668 (0,7); 2,543 (0,8); 2,508 (100,8); 2,503 (131,2); 2,499 (99,0); 2,335 (0,6); 2,330 (0,9); 2,326 (0,6); 2,183 (0,4); 1,571 (16,0); 1,355 (3,1); 1,231 (0,4); 0,146 (0,6); 0,008 (5,1); 0,000 (130,0); -0,008 (6,4); -0,150 (0,6) |
| 9 | | 2,96 | ¹H-NMR (400,0 MHz, d₆-DMSO): |
| | | | δ = 9,364 (1,9); 9,360 (1,9); 9,094 (2,7); 9,089 (3,3); 9,046 (3,4); 9,040 (2,7); 8,827 (1,5); 8,823 (1,6); 8,815 (1,6); 8,811 (1,5); 8,570 (0,9); 8,565 (1,1); 8,560 (0,9); 8,550 (1,0); 8,545 (1,2); 8,544 (1,2); 8,540 (0,9); 7,696 (1,5); 7,692 (2,6); 7,687 (2,3); 7,674 (1,2); 7,673 (1,2); 7,666 (1,2); 7,665 (1,1); 7,654 (1,1); 7,653 (1,0); 7,626 (1,1); 7,609 (1,2); 7,606 (1,4); 7,505 (1,2); 7,485 (2,3); 7,466 (1,2); 7,364 (1,3); 7,362 (1,3); 7,344 (1,0); 7,342 (1,0); 3,331 (13,1); 2,586 (16,0); 2,573 (0,8); 2,514 (7,5); 2,510 (14,4); 2,506 (18,7); 2,501 (13,5); 2,497 (6,7) |
| 10 | | 3,36 | ¹H-NMR (400,0 MHz, d₆-DMSO): |
| | | | δ = 9,369 (4,3); 9,364 (4,5); 9,150 (0,3); 9,087 (5,0); 9,082 (6,4); 9,050 (6,4); 9,044 (5,1); 9,029 (0,4); 8,830 (3,4); 8,826 (3,3); 8,818 (3,6); 8,815 (3,2); 8,575 (1,9); 8,571 (2,7); 8,566 (1,9); 8,555 (2,1); 8,551 (2,8); 8,546 (1,9); 7,871 (0,4); 7,852 (0,5); 7,747 (5,6); 7,690 (2,6); 7,678 (2,7); 7,670 (3,2); 7,668 (3,3); 7,659 (3,8); 7,644 (3,2); 7,566 (0,4); 7,515 (2,3); 7,496 (4,8); 7,476 (2,8); 7,409 (3,3); 7,389 (2,2); 3,331 (74,9); 3,140 (2,4); 3,121 (7,6); 3,103 (7,9); 3,085 (2,7); 2,673 (0,7); 2,508 (86,2); 2,504 (107,9); 2,500 (82,1); 2,335 (0,5); 2,331 (0,7); 2,087 (0,8); 1,312 (7,8); 1,294 (16,0); 1,275 (7,6); 1,259 (1,0); 1,230 (2,9); 1,088 (0,5); 0,867 (0,3); 0,852 (0,6); 0,834 (0,4); 0,147 (0,5); 0,008 (4,7); 0,000 (101,5); - 0,149 (0,5) |
| 11 | 3,34 | 3,37 | ¹H-NMR (400,0 MHz, d₆-DMSO): |
| | | | δ = 9,362 (3,2); 9,357 (3,1); 9,090 (0,4); 9,080 (5,0); 9,075 (5,8); 9,048 (0,4); 9,017 (6,1); 9,011 (5,1); 8,826 (2,5); 8,822 (2,6); 8,814 (2,6); 8,810 (2,5); 8,569 (1,5); 8,565 (2,0); 8,559 (1,5); 8,549 (1,7); 8,544 (2,1); 8,539 (1,5); 8,317 (0,6); 7,911 (0,4); 7,889 (0,4); 7,829 (6,2); 7,812 (2,2); 7,808 (7,2); 7,688 (1,9); 7,686 (1,9); 7,674 |
| | | | (1,9); 7,666 (1,9); 7,656 (1,8); 7,654 (1,8); 7,637 (0,4); 7,616 (0,3); 7,484 (7,1); 7,463 (6,3); 3,327 (229,5); 3,099 (2,3); 3,081 (7,2); 3,063 (7,4); 3,044 (2,4); 2,676 (1,5); 2,671 (2,1); 2,667 (1,5); 2,663 (0,8); 2,525 (5,5); 2,520 (8,3); 2,511 (107,6); 2,507 (217,5); 2,502 (288,2); 2,498 (211,9); 2,493 (104,2); 2,334 (1,3); 2,329 (1,9); 2,325 (1,3); 1,308 (7,7); 1,290 (16,0); 1,272 (7,5); 1,234 (0,6); 0,146 (1,8); 0,008 (13,1); 0,000 (372,2); -0,009 (13,9); -0,150 (1,7) |
| 12 | 1,58 | 1,86 | ¹H-NMR (400,0 MHz, d₆-DMSO): |
| | | | δ = 9,375 (4,2); 9,370 (4,2); 9,145 (5,6); 9,140 (7,4); 9,107 (7,5); 9,102 (5,8); 8,835 (3,1); 8,831 (3,4); 8,823 (3,3); 8,819 (3,4); 8,583 (1,8); 8,577 (2,5); 8,573 (1,9); 8,563 (2,0); 8,557 (2,7); 8,553 (1,9); 8,084 (7,3); 8,063 (8,9); 7,824 (8,7); 7,803 (7,7); 7,693 (2,4); 7,681 (2,4); 7,673 (2,4); 7,661 (2,3); 5,759 (0,4); 4,039 (0,3); 4,021 (0,3); 3,331 (69,7); 3,154 (0,5); 3,135 (1,8); 3,117 (2,1); 3,101 (2,4); 3,083 (2,2); 3,065 (0,7); 2,887 (0,6); 2,868 (2,2); 2,850 (2,5); 2,834 (2,0); 2,816 (1,8); 2,798 (0,5); 2,677 (0,6); 2,673 (0,8); 2,668 (0,6); 2,545 (0,5); 2,540 (0,6); 2,526 (2,3); 2,508 (95,9); 2,503 (127,4); 2,499 (95,8); 2,335 (0,6); 2,330 (0,8); 2,326 (0,6); 1,230 (0,4); 1,182 (0,7); 1,166 (1,1); 1,151 (0,6); 1,096 (7,4); 1,078 (16,0); 1,059 (7,2); 0,000 (3,6) |
| 13 | 1,85 | 1,85 | ¹H-NMR (400,0 MHz, d₆-DMSO): |
| | | | δ = 9,385 (4,0); 9,379 (4,3); 9,174 (3,9); 9,168 (7,7); 9,158 (8,0); 9,152 (4,2); 9,142 (0,9); 9,111 (0,8); 9,105 (0,6); 8,853 (0,6); 8,841 (2,9); 8,837 (3,3); 8,829 (3,1); 8,825 (3,3); 8,594 (1,6); 8,589 (2,4); 8,584 (1,9); 8,574 (1,8); 8,568 (2,6); 8,564 (1,9); 8,317 (1,3); 8,167 (5,9); 8,146 (9,0); 8,085 (1,0); 8,060 (9,1); 8,039 (6,1); 7,824 (1,0); 7,804 (0,8); 7,698 (2,4); 7,686 (2,3); 7,678 (2,4); 7,666 (2,3); 5,757 (0,7); 4,037 (0,8); 4,021 (0,8); 3,413 (2,2); 3,395 (6,9); 3,377 (7,2); 3,358 (3,3); 3,329 (568,0); 3,293 (2,4); 2,675 (2,8); 2,671 (3,9); 2,667 (3,0); 2,545 (0,9); 2,540 (1,0); 2,524 (11,0); 2,506 (445,4); 2,502 (589,5); 2,498 (444,2); 2,467 (4,1); 2,430 (1,1); 2,422 (0,5); 2,371 (0,9); 2,333 (2,9); 2,329 (3,9); 2,324 (3,0); 1,232 (1,3); 1,173 (8,4); 1,154 (16,0); 1,136 (7,0); 1,095 (0,9); 1,077 (1,8); 1,059 (0,8); 0,007 (0,5); 0,000 (14,8) |
| 14 | 3,12 | 3,13 | ¹H-NMR (400,0 MHz, d₆-DMSO): |
| | | | δ = 9,364 (3,7); 9,359 (3,7); 8,834 (2,8); 8,831 (3,0); 8,823 (3,0); 8,819 (2,9); 8,742 (4,8); 8,736 (6,6); 8,713 (6,5); 8,707 (4,7); 8,572 (1,6); 8,568 (2,3); 8,563 (1,6); 8,553 (1,8); 8,548 (2,4); 8,543 (1,6); 8,317 (0,4); 8,070 (6,5); 7,695 (2,2); 7,683 (2,2); 7,675 (2,1); 7,663 (2,0); 7,540 (2,1); 7,522 (3,9); 7,520 (3,9); 7,493 (1,8); 7,488 (1,9); 7,475 (2,3); 7,471 (2,7); 7,455 (1,1); 7,451 (1,4); 7,414 (1,8); 7,410 (2,1); 7,395 (4,0); 7,391 (3,4); 7,365 (3,2); 7,362 (3,2); 7,345 (4,2); 7,328 (2,3); 7,325 (2,4); 7,315 (0,8); 7,311 (0,7); 7,297 (0,9); 7,294 (0,9); 7,278 (0,4); 7,274 (0,4); 7,177 (0,6); 7,174 (0,7); 7,159 (1,0); |
| | | | 7,156 (1,0); 7,141 (0,4); 7,138 (0,4); 4,038 (0,5); 4,020 (0,6); 3,568 (0,4); 3,328 (165,3); 2,936 (2,2); 2,918 (7,2); 2,904 (3,7); 2,900 (7,5); 2,886 (3,1); 2,882 (2,7); 2,868 (0,9); 2,676 (1,1); 2,671 (1,5); 2,667 (1,1); 2,524 (4,1); 2,507 (166,9); 2,502 (217,8); 2,498 (164,7); 2,334 (1,1); 2,329 (1,5); 2,325 (1,1); 1,989 (2,2); 1,242 (0,4); 1,234 (0,3); 1,212 (2,8); 1,194 (6,2); 1,186 (7,9); 1,175 (4,5); 1,167 (16,0); 1,157 (1,3); 1,149 (7,4); 0,000 (0,5) |
| 15 | 1,58 | 1,59 | ¹H-NMR (400,0 MHz, d₆-DMSO): |
| | | | δ = 9,374 (4,2); 9,370 (4,3); 8,866 (6,3); 8,860 (7,5); 8,843 (3,2); 8,839 (3,5); 8,831 (3,4); 8,827 (3,5); 8,805 (7,3); 8,800 (6,2); 8,585 (1,8); 8,580 (2,5); 8,575 (1,9); 8,565 (2,0); 8,559 (2,7); 8,555 (1,9); 8,318 (0,3); 8,006 (2,9); 8,003 (3,1); 7,987 (3,4); 7,984 (3,5); 7,799 (1,4); 7,795 (1,5); 7,780 (3,2); 7,777 (3,2); 7,761 (2,2); 7,757 (2,2); 7,740 (2,0); 7,736 (2,1); 7,721 (3,3); 7,718 (3,4); 7,701 (3,5); 7,699 (3,7); 7,688 (2,4); 7,681 (2,3); 7,669 (2,2); 7,667 (2,2); 7,571 (3,6); 7,569 (3,6); 7,553 (3,0); 7,550 (2,9); 5,758 (1,5); 3,332 (197,0); 2,783 (0,5); 2,764 (1,8); 2,746 (2,1); 2,731 (2,4); 2,712 (2,2); 2,694 (0,7); 2,677 (0,8); 2,672 (1,0); 2,668 (0,8); 2,542 (0,6); 2,525 (2,5); 2,508 (116,7); 2,503 (153,0); 2,499 (113,2); 2,444 (0,7); 2,426 (2,1); 2,408 (2,4); 2,392 (2,1); 2,374 (1,8); 2,355 (0,6); 2,334 (0,7); 2,330 (1,0); 2,325 (0,8); 0,905 (7,4); 0,887 (16,0); 0,869 (7,1); 0,000 (6,3) |
| 16 | 1,86 | 1,87 | ¹H-NMR (400,0 MHz, d₆-DMSO): |
| | | | δ = 9,371 (4,2); 9,365 (4,1); 8,840 (3,2); 8,836 (3,3); 8,828 (3,3); 8,824 (3,3); 8,764 (5,8); 8,759 (7,2); 8,719 (7,0); 8,713 (5,7); 8,580 (1,8); 8,575 (2,5); 8,571 (1,9); 8,561 (1,9); 8,555 (2,6); 8,551 (1,8); 8,318 (1,8); 8,132 (3,1); 8,115 (3,4); 8,112 (3,4); 7,889 (1,6); 7,873 (3,3); 7,870 (3,3); 7,854 (2,3); 7,851 (2,2); 7,815 (2,1); 7,812 (2,5); 7,795 (3,0); 7,792 (3,1); 7,776 (1,3); 7,773 (1,3); 7,699 (2,5); 7,686 (2,5); 7,679 (2,5); 7,666 (2,4); 7,627 (0,5); 7,601 (3,6); 7,598 (3,7); 7,583 (3,2); 7,580 (3,1); 5,757 (1,7); 4,040 (0,5); 4,022 (0,5); 3,376 (0,5); 3,327 (653,5); 3,012 (2,1); 2,993 (7,0); 2,975 (7,2); 2,957 (2,2); 2,675 (4,7); 2,671 (6,3); 2,667 (4,8); 2,623 (0,3); 2,524 (17,2); 2,510 (380,2); 2,506 (748,3); 2,502 (973,4); 2,497 (719,5); 2,372 (0,3); 2,333 (4,7); 2,328 (6,4); 2,324 (4,8); 2,299 (0,4); 2,206 (0,3); 1,355 (0,3); 1,234 (1,4); 1,165 (1,7); 1,026 (7,4); 1,008 (16,0); 0,989 (7,2); 0,146 (17,1); 0,096 (0,3); 0,086 (0,7); 0,070 (0,7); 0,058 (1,2); 0,047 (1,5); 0,007 (153,7); 0,000 (3211,2); -0,009 (157,6); -0,068 (0,9); -0,075 (0,9); -0,095 (0,6); - 0,121 (0,4); -0,150 (16,9) |
| 17 | 3,74 | 3,68 | ¹H-NMR (400,0 MHz, d₆-DMSO): |
| | | | δ = 9,366 (3,2); 9,361 (3,2); 9,360 (3,1); 9,125 (4,6); 9,120 (5,3); 9,042 (5,4); 9,036 (4,8); 8,827 (2,5); 8,823 (2,8); 8,815 (2,7); 8,811 (2,8); 8,570 (1,4); 8,566 (1,9); 8,561 (1,5); 8,550 (1,6); 8,545 (2,1); 8,541 (1,5); 7,975 (3,8); 7,971 (4,0); 7,693 (2,0); 7,687 (3,0); 7,673 (4,1); 7,668 (3,2); 7,654 (1,8); 7,653 (1,8); 7,443 (3,2); 7,423 |
| | | | (2,8); 4,223 (1,3); 4,197 (4,1); 4,171 (4,3); 4,145 (1,5); 3,333 (12,5); 2,528 (0,7); 2,515 (13,7); 2,510 (27,0); 2,506 (35,7); 2,502 (27,0); 2,428 (16,0); 0,008 (1,8); 0,000 (45,7); -0,008 (2,1) |
| 18 | 2,4 | 2,34 | ¹H-NMR (400,0 MHz, d₆-DMSO): |
| | | | δ = 9,378 (2,7); 9,373 (2,8); 9,196 (0,3); 9,191 (0,4); 9,168 (3,5); 9,162 (4,8); 9,153 (0,5); 9,133 (4,7); 9,127 (3,6); 8,921 (0,3); 8,834 (2,1); 8,830 (2,3); 8,822 (2,2); 8,818 (2,3); 8,753 (0,3); 8,749 (0,4); 8,584 (1,2); 8,579 (1,7); 8,574 (1,3); 8,564 (1,3); 8,558 (1,8); 8,554 (1,4); 8,317 (0,6); 8,295 (3,7); 8,290 (3,9); 8,150 (1,8); 8,024 (1,8); 8,020 (1,8); 8,004 (2,0); 8,000 (2,0); 7,693 (1,7); 7,681 (1,7); 7,674 (1,8); 7,660 (1,7); 7,562 (2,8); 7,542 (2,5); 4,256 (1,0); 4,229 (3,2); 4,202 (3,3); 4,174 (1,1); 3,328 (135,7); 2,738 (0,4); 2,676 (1,4); 2,671 (2,0); 2,667 (1,5); 2,525 (5,7); 2,507 (215,8); 2,502 (284,3); 2,498 (216,6); 2,470 (16,0); 2,429 (0,3); 2,417 (0,9); 2,333 (1,4); 2,329 (1,9); 2,325 (1,5); 2,275 (0,8); 1,754 (2,0); 1,351 (0,7); 1,230 (1,0); 0,146 (1,1); 0,008 (9,5); 0,000 (247,0); -0,008 (11,8); -0,150 (1,2) |
| 19 | 3,97 | 3,95 | ¹H-NMR (400,0 MHz, d₆-DMSO): |
| | | | δ = 9,370 (2,7); 9,366 (2,7); 9,365 (2,7); 8,834 (2,3); 8,830 (2,5); 8,822 (2,4); 8,818 (2,5); 8,759 (2,5); 8,753 (8,0); 8,749 (7,8); 8,744 (2,3); 8,578 (1,3); 8,574 (1,7); 8,568 (1,3); 8,558 (1,4); 8,553 (1,8); 8,548 (1,3); 8,318 (0,3); 7,694 (1,6); 7,693 (1,6); 7,682 (1,6); 7,681 (1,6); 7,674 (1,6); 7,673 (1,6); 7,662 (1,5); 7,661 (1,5); 7,536 (5,9); 7,297 (4,6); 4,035 (1,2); 4,009 (3,8); 3,982 (4,0); 3,956 (1,4); 3,328 (100,9); 2,676 (0,7); 2,672 (0,9); 2,667 (0,7); 2,525 (2,7); 2,520 (3,9); 2,512 (49,4); 2,507 (100,6); 2,503 (133,6); 2,498 (97,4); 2,494 (47,0); 2,418 (14,4); 2,334 (0,6); 2,329 (0,9); 2,325 (0,6); 2,275 (16,0); 1,233 (0,4); 0,146 (0,8); 0,019 (0,4); 0,008 (6,1); 0,000 (176,8); -0,009 (6,0); -0,150 (0,8) |
| 20 | 2,54 | 2,52 | ¹H-NMR (400,0 MHz, d₆-DMSO): |
| | | | δ = 9,379 (3,2); 9,374 (3,0); 8,830 (7,8); 8,825 (9,1); 8,586 (1,4); 8,581 (1,9); 8,577 (1,3); 8,566 (1,5); 8,561 (2,0); 8,557 (1,3); 8,317 (0,5); 8,145 (0,6); 7,820 (6,5); 7,697 (1,8); 7,685 (1,8); 7,676 (1,8); 7,664 (1,7); 7,400 (4,8); 5,758 (2,3); 4,197 (1,1); 4,170 (3,3); 4,142 (3,5); 4,115 (1,2); 3,366 (0,8); 3,329 (136,7); 2,891 (0,7); 2,731 (0,6); 2,695 (0,4); 2,676 (1,3); 2,671 (1,7); 2,667 (1,3); 2,539 (1,9); 2,507 (193,1); 2,502 (244,6); 2,498 (184,8); 2,431 (15,1); 2,398 (0,8); 2,378 (16,0); 2,334 (1,3); 2,329 (1,7); 2,325 (1,3); 1,234 (1,0); 1,165 (1,0); 1,150 (0,6); 0,000 (6,2) |
| 21 | 1,91 | 1,86 | ¹H-NMR (400,0 MHz, d₆-DMSO): |
| | | | δ = 8,927 (4,0); 8,861 (2,0); 8,854 (9,9); 8,848 (2,3); 8,841 (0,8); 8,830 (0,4); 8,814 (0,3); 8,690 (0,3); 8,466 (2,0); 8,448 (2,1); 8,317 (1,3); 8,148 (0,5); 8,104 (2,1); 8,084 (2,3); 7,846 (0,5); 7,818 (7,0); 7,680 (2,2); 7,663 (2,3); 7,660 (2,3); 7,643 (1,9); 7,555 (0,4); 7,401 (4,8); |
| | | | 7,383 (0,3); 5,757 (2,2); 4,194 (1,1); 4,167 (3,4); 4,140 (3,6); 4,113 (1,2); 4,037 (0,4); 4,020 (0,4); 3,428 (0,3); 3,416 (0,4); 3,402 (0,5); 3,368 (1,5); 3,330 (615,1); 3,294 (2,0); 2,891 (1,5); 2,731 (1,3); 2,695 (1,2); 2,676 (2,8); 2,671 (3,9); 2,667 (3,0); 2,545 (2,0); 2,540 (2,2); 2,524 (10,1); 2,507 (425,8); 2,502 (566,6); 2,498 (425,9); 2,430 (14,9); 2,392 (2,1); 2,372 (16,0); 2,333 (2,6); 2,329 (3,6); 2,324 (2,8); 1,234 (2,2); 1,165 (1,4); 1,149 (0,8); 0,853 (0,4); 0,008 (0,5); 0,000 (15,0); - 0,008 (0,7) |
| 22 | 3,83 | 3,78 | ¹H-NMR (400,0 MHz, d₆-DMSO): |
| | | | δ = 9,375 (3,0); 9,370 (3,0); 8,953 (9,2); 8,950 (9,1); 8,836 (2,4); 8,833 (2,6); 8,824 (2,6); 8,821 (2,7); 8,583 (1,3); 8,578 (1,8); 8,573 (1,4); 8,563 (1,5); 8,558 (2,0); 8,553 (1,4); 7,902 (3,1); 7,882 (3,1); 7,693 (1,8); 7,681 (1,8); 7,673 (1,8); 7,663 (1,7); 7,661 (1,8); 7,422 (2,7); 7,393 (2,7); 5,758 (0,6); 4,114 (1,3); 4,088 (4,0); 4,062 (4,2); 4,036 (1,5); 3,328 (42,3); 2,677 (0,5); 2,673 (0,6); 2,668 (0,5); 2,526 (1,6); 2,521 (2,5); 2,512 (35,8); 2,508 (72,9); 2,503 (97,3); 2,499 (72,8); 2,495 (36,8); 2,468 (16,0); 2,403 (0,5); 2,335 (0,5); 2,330 (0,7); 2,326 (0,5); 2,076 (1,5); 0,146 (0,5); 0,008 (3,9); 0,000 (116,1); - 0,009 (4,6); -0,150 (0,5) |
| 23 | 2,5 | 2,46 | ¹H-NMR (400,0 MHz, d₆-DMSO): |
| | | | δ = 9,383 (12,6); 9,378 (12,3); 9,036 (15,7); 9,033 (14,8); 9,022 (12,3); 9,018 (16,0); 9,013 (7,6); 8,841 (9,7); 8,837 (10,6); 8,829 (10,1); 8,825 (10,6); 8,590 (5,6); 8,586 (7,4); 8,581 (5,7); 8,570 (6,1); 8,565 (8,1); 8,560 (5,8); 8,317 (1,4); 8,159 (13,2); 8,139 (13,3); 7,697 (7,3); 7,685 (7,1); 7,679 (6,9); 7,677 (7,0); 7,667 (6,7); 7,665 (6,9); 7,644 (0,6); 7,627 (0,8); 7,624 (0,8); 7,615 (0,7); 7,597 (0,8); 7,575 (0,6); 7,567 (0,7); 7,538 (10,5); 7,509 (10,2); 5,758 (3,5); 4,329 (0,4); 4,302 (1,4); 4,293 (2,2); 4,276 (2,8); 4,266 (7,1); 4,253 (6,9); 4,239 (7,4); 4,225 (7,4); 4,212 (2,8); 4,198 (2,7); 4,189 (1,6); 4,161 (0,5); 4,056 (1,0); 4,038 (2,9); 4,020 (3,0); 4,002 (1,0); 3,827 (0,4); 3,812 (0,5); 3,640 (0,6); 3,407 (0,3); 3,392 (0,7); 3,334 (1093,5); 3,268 (0,5); 3,175 (0,4); 3,162 (0,4); 2,677 (2,9); 2,672 (4,1); 2,668 (2,9); 2,645 (0,3); 2,525 (11,2); 2,512 (231,5); 2,508 (469,6); 2,503 (621,0); 2,499 (458,4); 2,494 (229,3); 2,485 (73,3); 2,434 (0,9); 2,410 (0,6); 2,388 (0,5); 2,372 (0,6); 2,334 (3,1); 2,330 (4,2); 2,325 (3,3); 1,990 (12,4); 1,909 (1,8); 1,298 (1,4); 1,259 (2,1); 1,233 (5,3); 1,193 (3,5); 1,175 (6,8); 1,157 (3,4); 0,853 (0,8); 0,836 (0,4); 0,146 (0,7); 0,008 (6,1); 0,000 (167,9); -0,008 (6,2); -0,150 (0,8) |
| 24 | 2,25 | 2,27 | ¹H-NMR (400,0 MHz, d₆-DMSO): |
| | | | δ = 9,251 (8,5); 9,247 (8,3); 9,245 (8,3); 8,790 (13,0); 8,758 (6,7); 8,754 (7,6); 8,746 (7,2); 8,742 (7,6); 8,465 (13,4); 8,458 (14,8); 8,445 (4,0); 8,441 (5,3); 8,439 (5,2); 8,435 (4,1); 8,425 (4,3); 8,419 (5,7); 8,415 (4,2); 8,326 (14,2); 8,319 (13,4); 7,637 (4,9); 7,635 (5,3); |
| | | | 7,623 (5,2); 7,617 (4,8); 7,615 (5,1); 7,605 (4,7); 7,603 (5,0); 7,350 (6,7); 7,329 (13,8); 7,310 (11,1); 7,231 (16,0); 7,212 (10,7); 6,980 (4,4); 6,962 (7,8); 6,943 (3,7); 3,326 (508,1); 2,731 (0,3); 2,676 (4,4); 2,671 (6,2); 2,667 (4,7); 2,524 (15,3); 2,520 (23,1); 2,511 (313,8); 2,507 (651,6); 2,502 (883,9); 2,498 (671,9); 2,494 (345,0); 2,447 (0,7); 2,431 (0,5); 2,338 (2,0); 2,333 (4,3); 2,329 (6,0); 2,324 (4,5); 1,055 (0,4); 0,146 (3,4); 0,032 (0,4); 0,008 (25,2); 0,000 (779,5); -0,009 (30,2); -0,150 (3,4) |
| 25 | 2,66 | 2,69 | ¹H-NMR (400,0 MHz, d₆-DMSO): |
| | | | δ = 9,269 (6,7); 9,264 (6,8); 8,962 (11,3); 8,772 (5,4); 8,768 (5,9); 8,760 (5,8); 8,756 (5,9); 8,483 (9,8); 8,476 (13,1); 8,465 (3,5); 8,461 (4,5); 8,456 (3,4); 8,445 (3,7); 8,440 (5,6); 8,436 (16,0); 8,429 (10,0); 8,317 (1,2); 7,647 (4,3); 7,646 (4,3); 7,635 (4,1); 7,627 (4,1); 7,626 (4,1); 7,615 (4,0); 7,614 (3,9); 7,356 (0,3); 7,339 (4,5); 7,319 (9,7); 7,299 (6,0); 7,189 (4,3); 7,184 (10,1); 7,179 (7,2); 7,170 (5,9); 7,150 (4,0); 7,146 (3,3); 6,967 (4,5); 6,964 (4,4); 6,947 (3,9); 6,944 (4,1); 5,757 (0,5); 3,326 (236,5); 2,891 (1,4); 2,732 (1,2); 2,680 (1,3); 2,676 (2,6); 2,671 (3,6); 2,667 (2,7); 2,662 (1,3); 2,525 (8,9); 2,520 (13,4); 2,511 (186,4); 2,507 (384,3); 2,502 (519,2); 2,498 (389,2); 2,493 (195,6); 2,338 (1,2); 2,333 (2,5); 2,329 (3,5); 2,325 (2,6); 2,086 (4,0); 2,075 (1,1); 0,146 (2,0); 0,008 (14,5); 0,000 (450,0); -0,009 (17,3); - 0,150 (2,0) |
| 26 | | 1,62 | ¹H-NMR (400,0 MHz, d₆-DMSO): |
| | | | δ = 11,160 (0,7); 10,721 (0,7); 9,345 (14,2); 9,294 (15,7); 9,162 (14,9); 8,822 (10,6); 8,814 (10,8); 8,761 (0,5); 8,697 (15,8); 8,551 (8,4); 8,531 (8,8); 8,475 (0,5); 8,429 (0,4); 7,894 (16,0); 7,799 (0,4); 7,684 (7,1); 7,667 (8,7); 7,654 (6,8); 7,235 (0,5); 7,220 (0,4); 7,216 (0,4); 6,673 (14,6); 5,935 (0,7); 4,134 (0,5); 3,391 (0,6); 3,326 (63,2); 2,671 (2,1); 2,503 (265,8); 2,329 (2,3); 2,283 (0,7); 2,265 (0,6); 2,244 (0,5); 2,234 (0,5); 2,209 (0,4); 2,205 (0,4); 2,178 (0,4); 2,160 (0,4); 2,155 (0,4); 2,141 (0,4); 2,087 (4,1); 2,058 (0,6); 2,044 (0,4); 1,983 (0,7); 1,926 (0,4); 1,907 (0,5); 1,886 (0,4); 1,870 (0,4); 1,852 (0,4); 1,821 (0,4); 1,743 (1,2); 1,678 (0,5); 1,671 (0,5); 1,625 (1,4); 1,532 (0,7); 1,511 (0,7); 1,463 (0,6); 1,453 (0,6); 1,433 (0,6); 1,416 (0,6); 1,367 (0,9); 1,349 (1,1); 1,297 (3,4); 1,257 (4,8); 1,230 (8,0); 1,171 (2,8); 1,146 (4,1); 1,136 (4,0); 1,088 (1,2); 1,065 (1,0); 1,042 (0,9); 1,036 (0,9); 0,981 (0,6); 0,943 (0,6); 0,928 (0,7); 0,863 (3,1); 0,755 (0,5); 0,724 (0,4); 0,701 (0,4); 0,145 (0,9); 0,123 (0,4); 0,105 (0,5); 0,095 (0,6); -0,001 (131,7); - 0,150 (1,2); -0,190 (0,4); -0,201 (0,4) |
| 27 | | 2,57 | ¹H-NMR (601,6 MHz, CD3CN): |
| | | | δ = 9,373 (4,7); 9,355 (0,8); 9,156 (8,3); 9,151 (8,5); 8,877 (8,9); 8,873 (8,7); 8,789 (3,3); 8,782 (3,4); 8,734 (0,6); 8,729 (0,6); 8,511 (2,6); 8,508 (3,8); 8,505 (2,6); 8,498 (3,3); 8,495 (3,9); 8,492 (3,0); 8,488 (1,3); 8,485 |
| | | | (1,1); 8,476 (0,6); 8,385 (5,8); 8,383 (5,8); 7,585 (3,2); 7,577 (3,3); 7,572 (3,3); 7,564 (3,3); 7,545 (0,5); 7,466 (0,6); 7,452 (1,5); 7,446 (0,9); 7,441 (0,7); 7,433 (0,7); 7,238 (0,5); 7,234 (0,4); 7,224 (0,4); 7,220 (0,4); 6,953 (6,6); 6,949 (6,5); 5,448 (0,7); 2,828 (1,2); 2,748 (0,6); 2,575 (2,2); 2,563 (2,3); 2,501 (0,5); 2,172 (9,1); 2,107 (5,6); 2,087 (3,1); 2,076 (2,7); 2,059 (3,2); 2,055 (4,1); 2,051 (5,1); 2,047 (3,9); 2,043 (2,7); 1,965 (67,2); 1,957 (37,4); 1,952 (51,3); 1,949 (253,4); 1,945 (433,6); 1,940 (624,6); 1,936 (441,3); 1,932 (228,3); 1,916 (4,5); 1,883 (1,7); 1,850 (1,1); 1,842 (1,1); 1,834 (2,0); 1,830 (3,1); 1,826 (4,2); 1,822 (3,0); 1,818 (1,8); 1,567 (0,5); 1,434 (0,5); 1,340 (13,1); 1,319 (2,0); 1,308 (2,5); 1,298 (2,3); 1,285 (16,0); 1,269 (9,4); 1,248 (1,2); 1,227 (1,0); 1,217 (1,0); 1,206 (1,0); 1,184 (1,3); 1,173 (1,2); 1,153 (0,8); 1,134 (0,8); 1,112 (0,7); 1,083 (0,8); 1,071 (0,7); 1,048 (0,6); 1,038 (0,6); 1,022 (4,4); 1,010 (8,3); 0,999 (4,3); 0,914 (0,7); 0,893 (1,2); 0,882 (2,1); 0,870 (1,5); 0,844 (1,3); 0,000 (2,8) |
| 28 | 2,48 | 2,53 | ¹H-NMR (400,0 MHz, d₆-DMSO): |
| | | | δ = 9,366(16,0); 9,362(16,0); 9,356(13,8); 9,312(12,2); 9,306(15,8); 9,302(7,3); 9,288(2,1); 9,238(13,8); 9,232(14,5); 9,214(1,9); 8,836(6,5); 8,832(8,2); 8,824(8,0); 8,820(8,7); 8,569(4,3); 8,564(6,1); 8,560(6,2); 8,550(5,7); 8,544(7,2); 8,540(6,6); 8,370(14,4); 8,352(2,5); 8,238(1,2); 8,166(1,1); 7,690(4,8); 7,678(5,4); 7,673(6,7); 7,660(5,3); 7,659(5,1); 7,642(1,0); 3,320(121,7); 3,303(20,7); 2,676(1,3); 2,671(1,7); 2,667(1,6); 2,507(200,6); 2,502(274,7); 2,498(253,0); 2,494(174,9); 2,329(1,6); 2,325(1,5); 2,075(1,5); 2,071(0,7); -0,001(54,0); - 0,005(24,7); -0,019(6,3) |
| 29 | 1,00 | 1,07 | ¹H-NMR(400,0 MHz, d₆-DMSO): |
| | | | δ = 9,331(1,1); 8,822(1,1); 8,762(4,6); 8,727(6,8); 8,533(1,6); 8,513(1,6); 7,678(1,1); 7,665(1,2); 7,658(1,2); 7,646(1,1); 3,683(16,0); 3,330(24,3); 2,678(0,4); 2,509(58,9); 2,337(0,4); 2,081(0,8) |
| 30 | 1,23 | 1,42 | ¹H-NMR(400,0 MHz, d₆-DMSO): |
| | | | δ = 9,336(0,6); 9,317(2,7); 9,310(3,0); 9,307(3,9); 9,300(3,8); 9,119(3,3); 9,113(3,3); 8,808(2,1); 8,800(2,2); 8,796(2,0); 8,712(0,4); 8,543(0,4); 8,522(1,4); 8,518(1,8); 8,512(1,4); 8,502(1,4); 8,497(1,6); 8,493(1,2); 7,673(1,3); 7,661(1,6); 7,655(1,5); 7,643(1,3); 3,556(0,4); 3,322(46,2); 3,255(17,4); 3,177(1,4); 3,141(0,4); 3,127(0,6); 3,122(0,5); 3,076(0,3); 3,060(2,0); 2,755(0,3); 2,736(0,4); 2,728(0,5); 2,670(2,4); 2,666(2,1); 2,505(269,8); 2,501(349,1); 2,497(274,9); 2,356(0,9); 2,338(16,0); 2,250(1,0); 2,227(0,7); 2,119(1,8); 1,234(0,5); 0,883(0,4); 0,000(50,2) |
| 31 | 0,97 | 1,21 | ¹H-NMR(400,0 MHz, d₆-DMSO): |
| | | | δ = 9,318(0,6); 8,816(0,4); 8,806(0,4); 8,701(0,7); 8,695(1,0); 8,668(0,9); 8,662(0,8); 8,524(0,4); 8,504(0,4); 7,669(0,4); 7,657(0,4); 7,649(0,4); 3,557(4,4); 3,387(0,7); 3,319(4,7); 2,506(37,0); 2,501(51,1); 2,497(42,5); 2,406(4,4); 2,328(0,3); 2,291(0,5); 1,158(2,3); 1,069(16,0); 0,000(7,1); - 0,002(4,9) |
| 32 | 1,15 | 1,25 | ¹H-NMR(400,0 MHz, d₆-DMSO): |
| | | | δ = 9,347(1,8); 9,341(1,9); 8,830(1,2); 8,826(1,4); 8,815(4,1); 8,809(3,9); 8,757(3,5); 8,750(2,9); 8,559(0,8); 8,554(1,1); 8,549(0,9); 8,539(0,9); 8,533(1,2); 8,529(0,9); 7,690(1,0); 7,678(1,0); 7,671(1,0); 7,658(1,0); 5,747(0,8); 3,882(0,5); 3,601(0,5); 3,415(879,7); 3,409(917,4); 3,297(0,8); 3,274(0,7); 3,238(0,4); 3,221(0,3); 3,213(0,4); 3,123(16,0); 3,099(0,4); 3,038(15,3); 3,018(0,3); 3,007(0,4); 2,977(0,3); 2,969(0,5); 2,683(0,8); 2,679(1,0); 2,674(0,7); 2,532(2,0); 2,527(3,1); 2,519(55,9); 2,514(120,3); 2,510(163,2); 2,505(117,1); 2,501(55,4); 2,345(0,4); 2,341(0,7); 2,336(1,0); 2,332(0,7) |
| 33 | 2,21 | 2,21 | ¹H-NMR(400,0 MHz, d₆-DMSO): |
| | | | δ = 9,318(2,6); 9,313(2,6); 9,253(3,4); 9,247(3,7); 9,067(3,8); 9,061(3,5); 8,811(1,7); 8,807(1,9); 8,799(1,8); 8,795(1,9); 8,520(1,0); 8,515(1,4); 8,511(1,0); 8,501(1,0); 8,495(1,5); 8,491(1,0); 7,672(1,4); 7,660(1,3); 7,652(1,3); 7,640(1,3); 5,240(1,2); 5,218(3,7); 5,197(3,9); 5,175(1,3); 3,316(54,1); 3,189(16,0); 2,891(0,8); 2,732(0,7); 2,675(0,4); 2,671(0,6); 2,667(0,5); 2,506(74,0); 2,502(103,4); 2,497(79,9); 2,333(0,5); 2,328(0,6); 2,324(0,5); 0,146(0,3); 0,008(3,0); 0,000(70,0); - 0,008(3,0); -0,150(0,3) |
| 34 | 1,84 | 1,87 | ¹H-NMR(400,0 MHz, d₆-DMSO): |
| | | | δ = 9,317(1,4); 9,313(1,4); 9,306(3,1); 9,300(3,1); 9,110(3,2); 9,103(3,0); 8,807(1,0); 8,798(1,0); 8,521(0,8); 8,516(1,0); 8,511(0,8); 8,501(0,8); 8,495(1,1); 8,491(0,8); 7,670(0,9); 7,658(1,0); 7,650(0,9); 7,638(0,9); 3,319(6,7); 3,264(0,5); 3,225(13,6); 3,000(0,4); 2,689(16,0); 2,676(0,4); 2,671(0,5); 2,667(0,3); 2,525(1,0); 2,511(22,4); 2,507(47,2); 2,502(66,0); 2,498(49,3); 2,493(23,7); 2,455(0,5); 2,329(0,4); 0,008(1,0); 0,000(31,8); - 0,008(1,1) |
| 35 | 2,17 | 2,25 | ¹H-NMR(400,0 MHz, d₆-DMSO): |
| | | | δ = 9,322(2,0); 9,318(1,9); 9,301(3,2); 9,294(3,3); 9,113(3,3); 9,106(3,1); 8,822(0,4); 8,810(1,4); 8,801(1,4); 8,798(1,3); 8,526(0,9); 8,522(1,3); 8,517(0,9); 8,506(1,0); 8,501(1,3); 8,497(0,9); 7,674(1,2); 7,662(1,2); 7,654(1,2); 7,642(1,1); |
| | | | 5,756(3,9); 3,318(65,6); 3,275(1,4); 3,257(4,1); 3,239(4,4); 3,225(16,0); 2,675(0,5); 2,671(0,6); 2,666(0,5); 2,510(41,1); 2,506(81,7); 2,502(107,0); 2,497(77,1); 2,493(37,4); 2,333(0,5); 2,329(0,6); 2,324(0,5); 1,445(4,6); 1,426(9,6); 1,408(4,5); 0,008(2,9); 0,000(67,0); -0,008(2,7) |
| 36 | | | ¹H-NMR(400,0 MHz, d₆-DMSO): |
| | | | δ = 9,328(4,1); 9,324(3,8); 9,322(4,0); 9,305(6,7); 9,299(7,1); 9,146(7,1); 9,140(6,6); 8,817(3,0); 8,813(3,3); 8,805(3,2); 8,801(3,3); 8,533(1,7); 8,528(2,2); 8,523(1,7); 8,513(1,9); 8,508(2,4); 8,503(1,7); 8,314(0,3); 7,676(2,2); 7,664(2,1); 7,657(1,9); 7,656(2,1); 7,645(1,9); 7,644(2,1); 4,309(1,5); 4,283(4,7); 4,258(4,9); 4,232(1,7); 3,923(2,2); 3,318(93,5); 3,297(28,7); 2,676(0,6); 2,671(0,8); 2,667(0,6); 2,525(2,0); 2,520(3,0); 2,511(45,6); 2,507(96,4); 2,502(135,1); 2,498(100,4); 2,493(47,8); 2,334(0,6); 2,329(0,8); 2,325(0,6); 1,481(0,4); 1,159(0,4); 1,069(16,0); 0,783(0,4); 0,008(0,8); 0,000(27,0); -0,009(0,8) |
| 37 | 1,69 | 1,79 | ¹H-NMR(400,0 MHz, d₆-DMSO): |
| | | | δ = 9,325(0,9); 9,296(3,0); 9,290(3,1); 9,113(3,7); 9,107(3,5); 8,815(0,8); 8,523(1,3); 8,502(1,3); 7,677(0,9); 7,664(1,0); 7,657(1,0); 7,645(0,9); 3,840(8,3); 3,328(15,5); 3,318(69,4); 2,671(0,6); 2,506(74,1); 2,502(100,0); 2,497(77,7); 2,333(0,5); 2,329(0,6); 2,135(16,0); 2,075(0,7); 0,000(29,6) |
| 38 | 1,1 | 1,15 | ¹H-NMR(400,0 MHz, d₆-DMSO): |
| | | | δ = 9,346(2,1); 9,342(2,2); 8,828(1,5); 8,824(1,8); 8,815(2,0); 8,812(4,5); 8,806(4,1); 8,765(4,1); 8,759(3,2); 8,556(0,9); 8,551(1,1); 8,547(1,0); 8,536(1,1); 8,531(1,3); 8,527(1,0); 7,683(1,1); 7,671(1,2); 7,663(1,1); 7,652(1,1); 7,651(1,1); 5,757(1,5); 3,320(12,5); 3,122(16,0); 3,035(15,1); 2,672(0,3); 2,525(1,0); 2,511(18,4); 2,507(38,7); 2,502(54,2); 2,498(40,4); 2,494(19,4); 1,234(0,6); 0,008(0,9); 0,000(24,3); -0,008(0,9) |
| 39 | 1,29 | 1,36 | ¹H-NMR(400,0 MHz, d₆-DMSO): |
| | | | δ = 9,356(8,6); 8,902(8,8); 8,897(11,6); 8,877(11,6); 8,871(8,2); 8,834(6,1); 8,823(6,3); 8,763(1,1); 8,755(1,0); 8,731(0,9); 8,711(0,9); 8,561(5,1); 8,542(5,7); 8,313(0,5); 8,177(0,4); 7,953(1,0); 7,879(16,0); 7,692(4,3); 7,681(5,2); 7,673(4,7); 7,661(4,5); 7,647(0,9); 7,550(0,8); 7,539(0,7); 7,529(0,7); 7,517(0,6); 5,308(0,4); 3,432(0,6); 3,320(260,1); 3,256(48,0); 3,190(0,5); 3,153(0,4); 3,076(0,3); 2,995(3,2); 2,891(4,8); 2,732(4,6); 2,711(1,2); 2,671(2,4); 2,645(0,4); 2,630(0,5); 2,541(215,4); 2,502(389,5); 2,368(2,0); 2,329(3,0); 2,295(0,8); 2,277(0,7); 2,243(0,6); 2,220(0,7); 2,200(0,6); 2,189(1,2); 2,173(0,6); 2,131(0,6); 2,117(0,9); 2,105(0,7); 2,086(0,6); 2,073(0,6); |
| | | | 2,036(0,6); 2,005(0,5); 1,997(0,5); 1,990(0,5); 1,980(0,5); 1,942(0,5); 1,909(5,1); 1,840(0,9); 1,816(0,4); 1,809(0,4); 1,762(0,4); 1,754(0,4); 1,722(0,3); 1,704(0,4); 1,673(0,3); 1,621(0,3); 1,610(0,4); 1,605(0,4); 1,603(0,4); 1,585(0,4); 1,574(0,4); 1,536(0,5); 1,514(0,6); 1,507(0,6); 1,500(0,6); 1,490(0,6); 1,466(0,7); 1,458(0,6); 1,386(1,4); 1,380(1,4); 1,347(1,8); 1,335(1,8); 1,298(1,7); 1,259(1,7); 1,234(2,6); 1,201(1,3); 1,141(1,4); 1,085(0,5); 1,070(0,6); 1,054(0,5); 1,045(0,5); 1,035(0,5); 0,988(0,5); 0,968(0,4); 0,922(0,3); 0,892(0,4); 0,864(0,6); 0,852(0,9); 0,830(1,3); 0,756(0,5); 0,726(0,3); 0,000(43,1) |
| 40 | 0,83 | | ¹H-NMR(400,0 MHz, d₆-DMSO): |
| | | | δ = 9,369(2,9); 9,323(0,4); 9,317(0,4); 9,230(0,4); 9,225(0,4); 9,152(5,1); 9,147(6,5); 9,110(5,6); 9,104(4,8); 8,847(3,7); 8,837(3,5); 8,571(1,7); 8,567(2,4); 8,562(1,8); 8,551(1,8); 8,546(2,4); 8,542(1,6); 7,696(2,1); 7,684(2,1); 7,677(2,0); 7,664(1,9); 3,955(1,5); 3,903(16,0); 3,511(0,4); 3,339(354,7); 3,337(355,7); 3,175(1,4); 3,162(1,4); 2,866(13,6); 2,854(13,6); 2,678(1,1); 2,673(1,5); 2,669(1,1); 2,526(3,9); 2,513(87,9); 2,508(186,6); 2,504(262,5); 2,499(195,4); 2,495(93,0); 2,340(0,5); 2,335(1,1); 2,331(1,4); 2,326(1,1); 1,234(0,4); 0,008(0,4); 0,000(11,9); -0,008(0,4) |
| 41 | 1,19 | | ¹H-NMR(400,0 MHz, d₆-DMSO): |
| | | | δ = 9,244(6,1); 9,157(6,1); 9,151(8,2); 9,116(7,1); 9,110(6,5); 8,873(5,3); 8,866(5,6); 8,806(2,3); 8,795(2,3); 8,519(1,8); 8,513(2,7); 8,508(2,0); 8,496(1,8); 8,490(2,7); 8,485(1,9); 3,992(0,4); 3,902(10,4); 3,509(0,5); 3,345(426,7); 3,170(1,1); 2,869(15,8); 2,857(16,0); 2,802(0,3); 2,677(1,0); 2,672(1,4); 2,668(1,1); 2,512(81,1); 2,508(169,0); 2,503(238,2); 2,499(183,7); 2,335(0,9); 2,330(1,3); 2,326(1,0); 1,351(0,4); 1,258(0,4); 1,232(1,4); 0,000(7,1) |
| 42 | 1,29 | | ¹H-NMR(400,0 MHz, d₆-DMSO): |
| | | | δ = 9,245(7,9); 8,870(6,8); 8,863(7,3); 8,850(9,3); 8,845(12,2); 8,810(12,7); 8,805(10,3); 8,521(2,6); 8,514(3,5); 8,509(2,7); 8,497(2,7); 8,493(3,3); 8,491(3,5); 8,486(2,7); 8,309(0,3); 3,902(16,0); 3,509(0,8); 3,360(944,6); 3,176(0,7); 3,165(0,6); 3,062(12,0); 3,013(12,6); 2,868(0,9); 2,679(1,2); 2,674(1,7); 2,670(1,3); 2,528(4,3); 2,514(95,1); 2,510(201,4); 2,505(282,5); 2,501(211,4); 2,496(101,5); 2,337(1,1); 2,332(1,6); 2,327(1,1); 1,275(0,4); 1,259(0,8); 1,245(0,7); 1,234(0,4); 0,000(7,8) |
| 43 | 0,96 | | ¹H-NMR(400,0 MHz, d₆-DMSO): |
| | | | δ = 9,363(3,6); 9,267(0,7); 9,262(0,9); 9,212(0,8); 9,207(0,8); 8,841(2,6); 8,830(2,6); 8,823(7,2); 8,818(9,1); 8,790(9,2); 8,784(7,3); 8,576(1,9); |
| | | | 8,571(3,1); 8,566(2,4); 8,556(2,2); 8,551(3,2); 8,546(2,2); 7,699(2,4); 7,687(2,6); 7,679(2,5); 7,667(2,3); 3,902(16,0); 3,509(0,7); 3,364(596,5); 3,171(1,6); 3,060(9,1); 3,014(9,4); 2,678(1,0); 2,674(1,3); 2,669(1,0); 2,513(72,9); 2,509(152,8); 2,505(214,9); 2,500(163,4); 2,496(80,5); 2,336(0,8); 2,331(1,2); 2,327(0,9); 1,233(0,8); 0,000(5,6) |
| 44 | 1,23 | | ¹H-NMR(400,0 MHz, d₆-DMSO): |
| | | | δ = 9,361(5,8); 8,840(4,3); 8,829(4,9); 8,791(2,9); 8,746(2,1); 8,571(3,4); 8,566(4,8); 8,561(3,4); 8,551(3,7); 8,546(5,0); 8,541(3,5); 8,313(0,4); 7,696(4,0); 7,684(4,1); 7,676(4,0); 7,664(3,7); 3,902(16,0); 3,539(1,9); 3,523(2,0); 3,509(1,8); 3,346(693,4); 3,170(1,1); 3,020(6,3); 2,989(6,0); 2,677(1,8); 2,673(2,4); 2,668(1,8); 2,579(0,4); 2,526(6,4); 2,512(144,2); 2,508(304,1); 2,503(426,5); 2,499(317,6); 2,495(151,3); 2,339(0,9); 2,335(1,7); 2,330(2,4); 2,326(1,7); 1,352(0,4); 1,259(0,5); 1,233(1,7); 1,188(3,4); 1,122(3,5); 0,853(0,4); 0,000(7,2) |
| 45 | 1,55 | | ¹H-NMR(400,0 MHz, d₆-DMSO): |
| | | | δ = 9,242(6,8); 8,869(6,0); 8,862(6,5); 8,843(1,6); 8,817(1,8); 8,798(1,6); 8,766(1,5); 8,518(2,1); 8,512(3,0); 8,507(2,2); 8,495(2,2); 8,488(3,0); 8,483(2,2); 3,902(16,0); 3,525(1,8); 3,510(1,6); 3,365(833,6); 3,171(2,1); 3,023(4,5); 2,986(4,4); 2,804(0,4); 2,679(1,2); 2,674(1,6); 2,670(1,2); 2,544(1,0); 2,527(4,2); 2,514(90,1); 2,510(189,9); 2,505(266,6); 2,501(199,8); 2,496(95,7); 2,336(1,1); 2,332(1,5); 2,327(1,1); 1,351(0,3); 1,233(1,3); 1,189(2,4); 1,119(2,5); 0,000(6,6) |
| 46 | 1,54 | | ¹H-NMR(400,0 MHz, d₆-DMSO): |
| | | | δ = 9,478(2,0); 9,463(4,1); 9,447(2,0); 9,374(4,9); 9,189(6,7); 9,184(12,4); 9,172(11,8); 9,167(6,7); 8,847(3,4); 8,837(3,3); 8,583(2,7); 8,578(3,7); 8,573(2,7); 8,563(3,0); 8,558(4,0); 8,553(2,7); 8,311(0,3); 7,699(3,2); 7,687(3,2); 7,680(3,1); 7,667(2,9); 4,233(1,3); 4,216(1,5); 4,208(4,0); 4,192(4,1); 4,184(4,3); 4,168(4,1); 4,160(1,7); 4,144(1,5); 4,107(0,3); 4,094(0,4); 3,955(1,3); 3,902(16,0); 3,547(0,5); 3,508(0,7); 3,346(618,3); 3,170(5,7); 2,682(0,7); 2,678(1,3); 2,673(1,8); 2,669(1,3); 2,526(4,9); 2,513(102,4); 2,509(214,9); 2,504(300,2); 2,500(221,5); 2,495(103,8); 2,340(0,5); 2,335(1,1); 2,331(1,6); 2,326(1,1); 1,249(0,4); 1,232(0,8); 0,000(8,1) |
| 47 | 1,90 | | ¹H-NMR(400,0 MHz, d₆-DMSO): |
| | | | δ = 9,488(1,0); 9,473(1,9); 9,457(0,9); 9,257(3,9); 9,201(16,0); 8,880(3,5); 8,873(3,6); 8,536(1,2); 8,530(1,6); 8,525(1,1); 8,513(1,2); 8,506(1,5); 8,501(1,1); 4,234(0,6); 4,218(0,7); 4,210(1,8); 4,194(1,9); 4,186(2,0); 4,170(1,8); 4,162(0,7); |
| | | | 4,146(0,6); 3,902(7,6); 3,341(184,8); 3,334(195,9); 3,169(0,5); 2,677(0,6); 2,672(0,9); 2,668(0,6); 2,525(2,7); 2,512(52,7); 2,508(107,6); 2,503(148,5); 2,499(111,1); 2,494(54,0); 2,334(0,6); 2,330(0,8); 2,326(0,6); 1,234(0,4); 0,000(5,8) |
| 48 | 1,20 | | ¹H-NMR(400,0 MHz, d₆-DMSO): |
| | | | δ = 9,362(1,8); 8,997(3,0); 8,992(3,7); 8,945(3,8); 8,940(3,2); 8,844(1,2); 8,833(1,2); 8,573(0,9); 8,567(1,3); 8,563(1,0); 8,553(1,0); 8,547(1,4); 8,543(1,0); 7,697(1,1); 7,685(1,2); 7,677(1,1); 7,665(1,1); 3,902(4,2); 3,608(16,0); 3,424(0,6); 3,353(185,0); 3,347(193,9); 3,170(0,8); 2,673(0,6); 2,669(0,4); 2,526(1,4); 2,509(66,8); 2,504(94,1); 2,500(71,7); 2,335(0,4); 2,331(0,5); 2,326(0,4); 1,233(0,4); 0,000(2,7) |
| 49 | 1,54 | | ¹H-NMR(400,0 MHz, d₆-DMSO): |
| | | | δ = 9,245(3,0); 9,026(3,2); 9,021(3,9); 8,962(3,9); 8,957(3,5); 8,875(2,6); 8,868(2,8); 8,523(0,9); 8,517(1,2); 8,512(1,0); 8,499(1,0); 8,493(1,2); 8,488(0,9); 3,902(5,6); 3,607(16,0); 3,354(200,7); 3,346(249,7); 3,170(0,4); 2,678(0,5); 2,673(0,7); 2,669(0,5); 2,526(2,1); 2,513(38,5); 2,508(80,5); 2,504(112,9); 2,499(86,6); 2,495(43,4); 2,335(0,5); 2,331(0,6); 2,326(0,5); 1,234(0,8); 0,000(3,4) |
| 50 | 1,45 | | ¹H-NMR(400,0 MHz, d₆-DMSO): |
| | | | δ = 9,382(2,0); 9,209(1,1); 9,193(2,3); 9,185(4,9); 9,180(7,3); 9,159(6,3); 9,154(4,4); 8,854(1,4); 8,845(1,4); 8,582(1,3); 8,577(1,9); 8,573(1,4); 8,562(1,5); 8,557(2,0); 8,553(1,4); 7,701(1,6); 7,689(1,6); 7,682(1,6); 7,669(1,5); 3,902(16,0); 3,853(1,3); 3,837(1,3); 3,818(2,6); 3,802(2,6); 3,783(1,4); 3,767(1,3); 3,509(0,4); 3,349(364,2); 3,344(447,4); 3,341(452,3); 3,176(0,8); 3,163(0,8); 2,677(1,0); 2,673(1,4); 2,668(1,1); 2,664(0,5); 2,526(3,6); 2,521(5,4); 2,513(80,5); 2,508(172,3); 2,504(243,6); 2,499(179,6); 2,495(84,0); 2,340(0,4); 2,335(0,9); 2,330(1,3); 2,326(1,0); 1,727(4,3); 1,679(9,7); 1,632(4,8); 0,000(5,0) |
| 51 | 1,80 | | ¹H-NMR(400,0 MHz, d₆-DMSO): |
| | | | δ = 9,348(0,4); 9,343(0,4); 9,256(8,0); 9,242(0,9); 9,222(2,2); 9,206(4,4); 9,201(6,9); 9,196(12,5); 9,188(11,9); 9,183(5,9); 8,878(7,2); 8,872(7,3); 8,534(2,4); 8,528(3,3); 8,523(2,4); 8,511(2,5); 8,504(3,2); 8,499(2,3); 8,316(0,3); 4,428(0,5); 4,410(0,5); 4,110(0,4); 4,098(0,4); 3,958(0,4); 3,902(14,3); 3,854(2,2); 3,839(2,2); 3,819(4,5); 3,803(4,4); 3,784(2,4); 3,768(2,3); 3,598(0,4); 3,559(0,4); 3,528(0,5); 3,508(0,7); 3,490(0,6); 3,341(647,7); 3,176(2,2); 3,163(2,1); 2,677(1,6); 2,672(2,3); 2,668(1,6); 2,594(0,4); 2,588(0,4); 2,525(6,1); 2,512(127,9); 2,508(267,9); 2,503(375,8); 2,499(283,5); 2,494(138,3); 2,335(1,5); 2,330(2,0); |
| | | | 2,326(1,5); 1,728(7,3); 1,680(16,0); 1,633(8,0); 1,405(0,4); 1,388(0,9); 1,370(0,4); 1,244(0,3); 1,233(0,5); 0,008(0,4); 0,000(11,4) |
| 52 | 1,39 | | ¹H-NMR(400,0 MHz, d₆-DMSO): |
| | | | δ = 9,365(1,2); 9,153(2,3); 9,148(2,8); 9,101(2,7); 9,096(2,4); 8,981(0,5); 8,967(1,0); 8,953(0,5); 8,843(0,9); 8,831(0,8); 8,574(0,7); 8,568(1,0); 8,564(0,7); 8,554(0,7); 8,548(1,0); 8,544(0,8); 7,695(0,8); 7,683(0,8); 7,676(0,8); 7,664(0,8); 3,902(4,0); 3,556(0,8); 3,540(1,6); 3,522(1,6); 3,507(0,9); 3,356(130,8); 3,349(200,4); 2,724(1,7); 2,706(2,5); 2,689(1,6); 2,678(0,4); 2,673(0,5); 2,669(0,3); 2,526(1,2); 2,513(24,8); 2,509(53,4); 2,504(76,2); 2,500(58,4); 2,495(28,7); 2,331(0,4); 2,326(0,3); 2,128(16,0); 0,000(2,5) |
| 53 | 1,72 | | ¹H-NMR(400,0 MHz, d₆-DMSO): |
| | | | δ = 9,248(2,1); 9,169(2,2); 9,164(2,8); 9,130(2,5); 9,124(2,1); 8,991(0,5); 8,977(1,0); 8,962(0,5); 8,875(1,9); 8,868(2,0); 8,524(0,6); 8,518(0,9); 8,513(0,7); 8,501(0,7); 8,495(0,9); 8,490(0,6); 3,902(4,2); 3,558(0,8); 3,542(1,6); 3,524(1,6); 3,508(0,9); 3,348(170,0); 2,725(1,8); 2,706(2,6); 2,689(1,7); 2,678(0,4); 2,673(0,5); 2,669(0,4); 2,526(1,2); 2,513(27,8); 2,509(58,7); 2,504(82,4); 2,500(61,3); 2,495(29,3); 2,335(0,3); 2,331(0,5); 2,326(0,3); 2,128(16,0); 0,000(1,9) |
| 54 | 0,84 | | ¹H-NMR(400,0 MHz, d₆-DMSO): |
| | | | δ = 9,372(1,9); 9,152(3,4); 9,147(4,5); 9,125(1,7); 9,115(4,8); 9,109(4,2); 8,849(1,3); 8,838(1,3); 8,576(1,0); 8,571(1,4); 8,567(1,1); 8,556(1,1); 8,551(1,5); 8,547(1,1); 7,698(1,2); 7,686(1,2); 7,678(1,2); 7,666(1,1); 3,955(0,6); 3,902(8,0); 3,777(0,9); 3,760(2,4); 3,745(2,5); 3,729(1,2); 3,455(2,2); 3,438(4,0); 3,421(1,9); 3,344(186,2); 3,336(242,0); 3,176(0,6); 3,162(0,6); 3,075(16,0); 2,677(0,6); 2,672(0,8); 2,668(0,6); 2,543(0,6); 2,526(2,1); 2,512(47,9); 2,508(101,9); 2,503(144,1); 2,499(111,8); 2,334(0,6); 2,330(0,8); 2,325(0,6); 0,000(3,8) |
| 55 | 1,12 | | ¹H-NMR(601,6 MHz, d₆-DMSO): |
| | | | δ = 9,254(1,8); 9,252(2,8); 9,194(0,8); 9,185(1,7); 9,177(4,0); 9,173(5,2); 9,161(5,1); 9,158(3,3); 8,876(3,0); 8,872(3,0); 8,527(1,0); 8,524(1,2); 8,523(1,2); 8,520(1,0); 8,512(1,0); 8,508(1,3); 8,507(1,2); 8,504(1,0); 7,953(0,5); 3,769(1,0); 3,758(2,5); 3,748(2,5); 3,737(1,2); 3,454(2,1); 3,442(3,9); 3,431(1,9); 3,324(361,5); 3,281(0,4); 3,076(16,0); 2,986(0,5); 2,891(4,0); 2,7313(3,2); 2,7307(3,1); 2,689(0,5); 2,617(1,3); 2,614(1,8); 2,611(1,3); 2,523(3,2); 2,520(4,2); 2,517(4,3); 2,508(97,1); 2,505(201,1); 2,502(270,3); 2,499(198,0); 2,496(95,1); 2,389(1,3); 2,386(1,7); 2,383(1,3); |
| | | | 0,937(0,4); 0,000(7,3) |
| 56 | 3,14 | | ¹H-NMR(400,0 MHz, d₆-DMSO): |
| | | | δ = 10,867(7,7); 9,395(4,2); 9,284(6,7); 9,278(9,8); 9,257(9,3); 9,252(6,8); 8,855(3,0); 8,846(3,0); 8,602(2,2); 8,597(3,1); 8,593(2,3); 8,582(2,4); 8,577(3,4); 8,573(2,3); 8,313(0,4); 8,277(6,2); 8,032(2,9); 8,030(2,8); 8,009(3,2); 7,708(2,8); 7,696(2,8); 7,688(2,7); 7,676(2,6); 7,603(3,1); 7,583(6,6); 7,563(4,4); 7,507(4,3); 7,487(2,7); 4,108(0,8); 4,095(0,8); 4,083(0,3); 3,902(16,0); 3,508(0,5); 3,495(0,4); 3,346(476,4); 3,340(619,0); 3,176(6,2); 3,163(6,1); 2,677(1,3); 2,672(1,7); 2,668(1,3); 2,664(0,7); 2,526(4,7); 2,512(103,2); 2,508(217,0); 2,503(304,9); 2,499(228,7); 2,494(110,1); 2,335(1,2); 2,330(1,7); 2,326(1,2); 1,249(0,3); 0,008(0,4); 0,000(12,2); -0,009(0,4) |
| 57 | 3,53 | | ¹H-NMR(400,0 MHz, d₆-DMSO): |
| | | | δ = 10,872(8,7); 9,298(6,5); 9,293(10,8); 9,277(16,0); 8,887(6,9); 8,880(7,1); 8,555(2,2); 8,549(3,3); 8,544(2,4); 8,532(2,3); 8,525(3,3); 8,521(2,3); 8,308(0,3); 8,273(7,1); 8,025(3,4); 8,004(3,7); 7,605(3,2); 7,585(7,0); 7,565(4,6); 7,509(5,1); 7,490(3,2); 3,902(15,3); 3,705(0,4); 3,657(0,4); 3,603(0,6); 3,508(1,4); 3,361(1440,1); 3,174(0,6); 2,678(1,6); 2,674(2,2); 2,670(1,6); 2,582(0,4); 2,544(1,6); 2,509(266,9); 2,505(367,0); 2,500(281,7); 2,336(1,5); 2,331(2,0); 1,233(0,6); 0,000(7,6) |
| 58 | 2,54 | | ¹H-NMR(601,6 MHz, d₆-DMSO): |
| | | | δ = 11,037(1,3); 9,317(7,8); 9,313(14,4); 9,306(16,0); 9,302(8,6); 9,278(8,6); 8,887(8,8); 8,883(10,4); 8,833(0,9); 8,829(0,8); 8,825(0,9); 8,554(2,8); 8,551(3,7); 8,547(2,8); 8,539(3,0); 8,536(3,8); 8,532(2,6); 8,452(8,0); 8,270(1,6); 8,189(0,8); 8,176(0,9); 8,160(4,1); 8,147(4,3); 8,145(4,3); 8,022(0,8); 8,007(0,8); 7,760(4,0); 7,746(8,2); 7,733(4,8); 7,646(5,2); 7,634(4,1); 7,596(0,7); 7,583(1,5); 7,569(0,9); 7,505(1,0); 7,493(0,8); 3,322(411,9); 2,614(2,3); 2,611(1,7); 2,541(1,0); 2,523(4,5); 2,520(5,8); 2,517(6,8); 2,505(256,7); 2,502(334,0); 2,499(250,5); 2,386(2,0); 1,231(0,6); 0,000(7,3) |
| 59 | 1,65 | | ¹H-NMR(400,0 MHz, d₆-DMSO): |
| | | | δ = 9,231(6,6); 8,861(5,8); 8,854(6,1); 8,761(8,3); 8,508(1,9); 8,503(2,6); 8,485(1,9); 8,480(2,6); 8,475(1,8); 3,902(16,0); 3,508(0,4); 3,342(396,2); 3,338(410,6); 3,176(0,7); 3,164(0,7); 2,677(1,1); 2,672(1,5); 2,668(1,2); 2,548(0,6); 2,525(4,3); 2,512(90,4); 2,508(185,6); 2,503(257,8); 2,499(197,1); 2,335(1,2); 2,330(1,5); 2,325(1,2); 2,199(0,5); 2,143(0,6); 2,130(0,6); 2,112(0,6); 2,074(0,4); 1,249(0,3); 1,235(0,3); 0,969(4,6); 0,008(0,4); |
| | | | 0,000(10,5) |
| I-a-1 | | 1,95 | ¹H-NMR (400,0 MHz, d₆-DMSO): |
| | | | δ = 9,339 (8,2); 9,337 (8,7); 9,333 (8,6); 9,332 (8,2); 9,046 (15,3); 9,040 (16,0); 8,842 (15,8); 8,836 (15,1); 8,830 (7,6); 8,827 (7,8); 8,818 (7,7); 8,814 (7,5); 8,548 (4,4); 8,544 (5,4); 8,543 (5,3); 8,538 (4,3); 8,528 (4,7); 8,524 (5,4); 8,523 (5,8); 8,518 (4,4); 8,318 (0,4); 7,679 (5,3); 7,677 (5,4); 7,667 (5,2); 7,665 (5,3); 7,659 (5,2); 7,657 (5,1); 7,647 (5,0); 7,645 (5,0); 3,757 (1,1); 3,329 (112,8); 2,678 (0,5); 2,674 (0,7); 2,669 (0,5); 2,527 (1,9); 2,514 (39,9); 2,509 (80,5); 2,505 (105,7); 2,500 (75,9); 2,496 (35,9); 2,336 (0,5); 2,331 (0,7); 2,327 (0,5); 0,146 (0,7); 0,008 (5,9); 0,000 (157,7); -0,009 (5,5); -0,150 (0,7) |
| I-j-1 | | | LC-MS: *m*/*z* 272 [M+H⁺] |
| I-j-2 | | | LC-MS: *m*/*z* 290 [M+H⁺] |
| I-s-1 | | | LC-MS: *m*/*z* 229 [M+H⁺] |
| I-s-2 | | | LC-MS: *m*/*z* 247 [M+H⁺] |

### Biologische Beispiele

### Myzus persicae - Sprühtest

| | |
|---|---|
| Lösungsmittel: | 78 Gewichtsteile Aceton |
| | 1,5 Gewichtsteile Dimethylformamid |
| | |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 500g/ha: 2, 4, 6, 7, 9, 19, 22, 30, 41, 42, 43, 44

Bei diesem Test zeigen z. B. die folgenden Verbindungen der Herstellungsbeispiele Wirkung von 90% bei einer Aufwandmenge von 500g/ha: 13, 16, 17, 18, 20, 23, 25, 26, 31, 32, 39, 45, 48, 49

### Tetranychus urticae - Sprühtest, OP-resistent

| | |
|---|---|
| Lösungsmittel: | 78,0 GewichtsteileAceton |
| | 1,5 Gewichtsteile Dimethylformamid |
| | |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90% bei einer Aufwandmenge von 500g/ha: 22

### Phaedon cochleariae - Sprühtest

| | |
|---|---|
| Lösungsmittel: | 78,0 Gewichtsteile Aceton |
| | 1,5 Gewichtsteile Dimethylformamid |
| | |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 83% bei einer Aufwandmenge von 500g/ha: 39

### Myzus persicae - Sprühtest

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (*Capsicum annuum*), die stark von der Grünen Pfirsichblattlaus (*Myzus persicae*) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigen z. B. die folgenden Verbindungen der Herstellungsbeispiele Wirkung von 100% bei einer Aufwandmenge von 4ppm: 21

## Patentansprüche

1. Verbindungen der Formel (I) 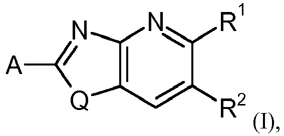 in welcher
A für den Rest (A-a) 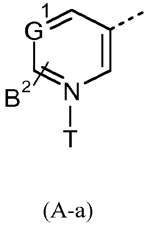 steht, worin die gestrichelte Linie die Bindung zum Kohlenstoffatom des Bicyclus der Formel (I) bedeutet,
G¹ für C-B¹ steht,
B¹ für Wasserstoff oder Fluor steht,
B² für Wasserstoff steht,
T für ein Elektronenpaar oder für Sauerstoff steht,
Q für Schwefel steht,
R¹ für Wasserstoff steht,
R² a) für einen der folgenden B-Reste 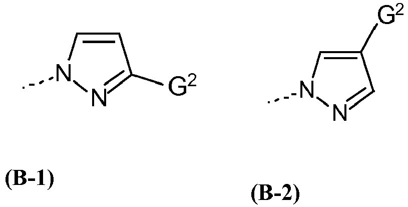 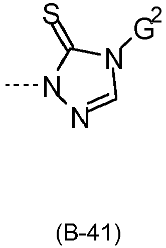 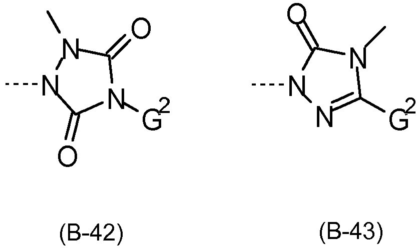 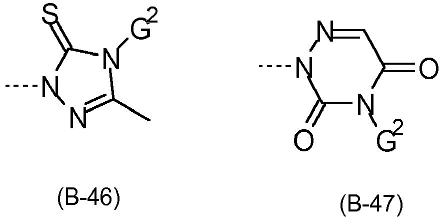
steht, worin die gestrichelte Linie die Bindung zum Kohlenstoffatom des Bicyclus der Formel (I) bedeutet, oder
R² c) für einen Rest der Formel 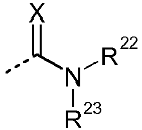
steht, worin X für Sauerstoff oder Schwefel steht und gestrichelte Linie die Bindung zum Kohlenstoffatom des Bicyclus der Formel (I) bedeutet, oder
R² f) für einen Rest der Formel 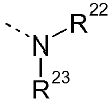 steht, worin die gestrichelte Linie die Bindung zum Kohlenstoffatom des Bicyclus der Formel (I) bedeutet, worin
G² für einen Rest aus der Reihe Wasserstoff, C₁-C₄-Alkyl, Halogen-C₁-C₄-alkyl C₁-C₄-Alkoxy, Halogen-C₁-C₄-alkoxy, C₁-C₄-Alkylthio und C₁-C₄-Halogenalkylthio steht, und, im Fall, dass R² für c) steht
R²² für den D-Rest 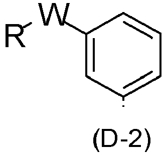 steht, worin
R für gegebenenfalls einfach, zweifach, dreifach, vierfach oder fünffach durch Fluor, Chlor substituiertes C₁-C₄-Alkyl steht,
W für einen Rest aus der Reihe S, SO und SO₂ steht, und, im Fall, dass R² für f) steht,
R²² für gegebenenfalls durch Fluor, Chlor oder Brom substituiertes Phenyl steht und
R²³ für Wasserstoff oder C₁-C₆-Alkyl steht.

2. Mittel, **gekennzeichnet durch** einen Gehalt von mindestens einer Verbindung der Formel (I) gemäß Anspruch 1 und üblichen Streckmitteln und/oder oberflächenaktiven Substanzen.

3. Nicht-therapeutische Verwendung von Verbindungen der Formel (I) gemäß Anspruch 1 oder von Mitteln gemäß Anspruch 2 zur Bekämpfung von Schädlingen.

4. Verbindung der Formel

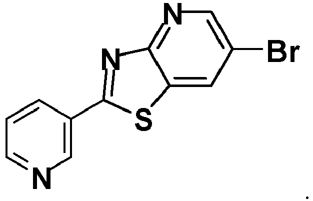

## Claims

1. Compounds of the formula (I) 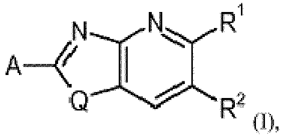 in which
A is the radical (A-a) 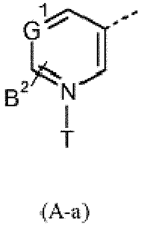 wherein the dashed line denotes the bond to the carbon atom of the bicycle of the formula (I),
G¹ is C-B¹
B¹ is hydrogen or fluorine,
B² is hydrogen,
T is an electron pair or is oxygen
Q is sulfur
R¹ is hydrogen
R² a) is one of the following B radicals 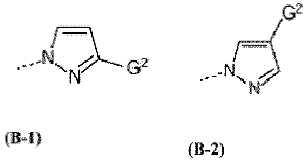 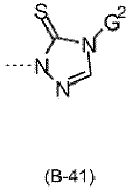 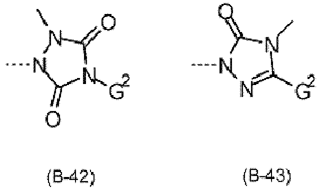 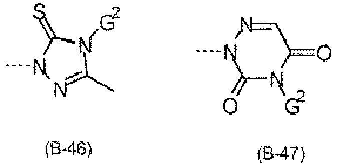
wherein the dashed line denotes the bond to the carbon atom of the bicycle of the formula (I), or
R² c) is a radical of the formula 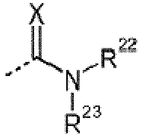
wherein X is oxygen or sulfur and the dashed line denotes the bond to the carbon atom of the bicycle of the formula (I), or
R² f) is a radical of the formula 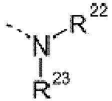
wherein the dashed line denotes the bond to the carbon atom of the bicycle of the formula (I), in which
G² is a radical from the series comprising hydrogen, C₁-C₄-alkyl, halo-C₁-C₄-alkyl, C₁-C₄-alkoxy, halo-C₁-C₄-alkoxy, C₁-C₄-alkylthio and C₁-C₄-haloalkylthio,
and, in the case that R² is c).
R²² is the D radical 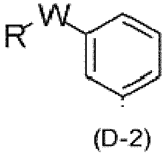
in which
R is C₁-C₄-alkyl optionally mono-, di-, tri-, tetra- or penta-substituted by fluorine or chlorine,
W is a radical from the series comprising S, SO and SO₂, and, in the case that R² is f),
R²² is phenyl optionally substituted by fluorine, chlorine or bromine,
R²³ is hydrogen or C₁-C₆-alkyl.

2. Composition **characterized by** a content of at least one compound of the formula (I) according to Claim 1 and customary extenders and/or surface-active substances.

3. Non-therapeutic use of compounds of the formula (I) according to Claim 1 or of compositions according to Claim 2 for controlling pests.

4. Compound of the formula

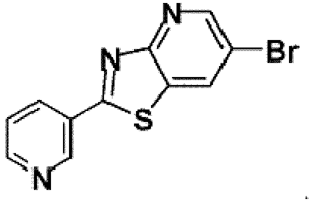

## Revendications

1. Composés de formule (I) 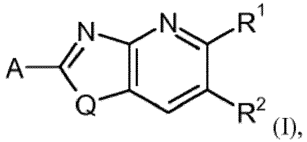 dans laquelle
A représente le radical (A-a) 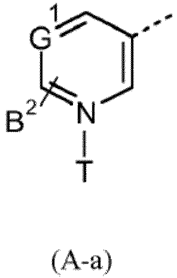
dans lequel la ligne en pointillés signifie la liaison à l'atome de carbone du bicycle de formule (I),
G¹ représente C-B¹,
B¹ représente hydrogène ou fluor,
B² représente hydrogène,
T représente une paire d'électrons ou l'oxygène,
Q représente soufre,
R¹ représente hydrogène,
R² représente a) un des radicaux B suivants : 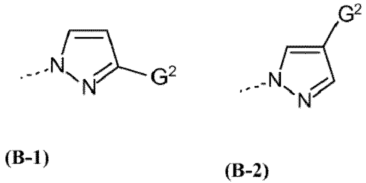 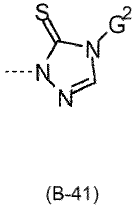 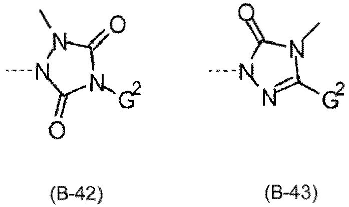 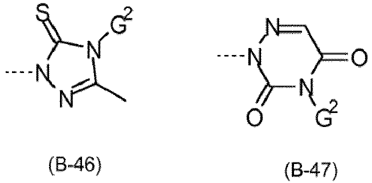
dans lesquels la ligne en pointillés signifie la liaison à l'atome de carbone du bicycle de formule (I), ou
R² représente c) un radical de formule 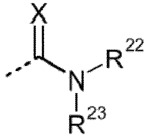 dans lequel X représente oxygène ou soufre, et
la ligne en pointillés signifie la liaison à l'atome de carbone du bicycle de formule (I), ou
R² représente f) un radical de formule 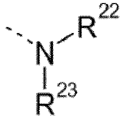
dans lequel la ligne en pointillés signifie la liaison à l'atome de carbone du bicycle de formule (I),
G² représente un radical de la série constituée par hydrogène, alkyle en C₁-C₄, halogéno-alkyle en C₁-C₄, alcoxy en C₁-C₄, halogéno-alcoxy en C₁-C₄, alkylthio en C₁-C₄ et halogéno-alkylthio en C₁-C₄, et, lorsque R² représente c),
R²² représente le radical D 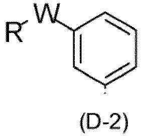
dans lequel
R représente alkyle en C₁-C₄ éventuellement substitué une fois, deux fois, trois fois, quatre fois ou cinq fois par fluor, chlore,
W représente un radical de la série constituée par S, SO et SO₂, et, lorsque R² représente f),
R²² représente phényle éventuellement substitué par fluor, chlore ou brome, et
R²³ représente hydrogène ou alkyle en C₁-C₆.

2. Agent, **caractérisé par** une teneur en au moins un composé de formule (I) selon la revendication 1 et des extendeurs et/ou des substances tensioactives usuels.

3. Utilisation non thérapeutique de composés de formule (I) selon la revendication 1 ou d'agents selon la revendication 2 pour lutter contre des nuisibles.

4. Composé de formule
